# EUROPEAN PATENT APPLICATION

(11) **EP 4 424 821 A1**
(43) Date of publication of application: **04.09.2024**
(21) Application number: 23159422.7
(22) Date of filing: 01.03.2023
(51) Int. Cl.: C12N 9/02, A61P 31/04, C07C 227/16

(54) **ENZYMATIC SYNTHESIS OF ODILORHABDIN AND RELATED PEPTIDES/PROTEINS COMPRISING NON-STANDARD AMINO ACID RESIDUES**

(71) Applicant: Max-Planck-Gesellschaft zur Förderung der Wissenschaften e.V., 80539 München (DE)
(72) Inventor: The designation of the inventor has not yet been filed
(74) Representative: Vossius & Partner Patentanwälte Rechtsanwälte mbB

(57) **Abstract**

The present invention relates to a novel method of preparing a non-standard amino acid or a peptide/protein comprising a non-standard amino acid residue, as appropriate, wherein said amino acid residue is selected from 2,4-diamino-3-hydroxybutyrate, L-5-hydroxylysine, L-3-hydroxyarginine, 2,3-didehydroarginine by using enzymatic transformations. Accordingly, the present invention further relates to a method of preparing a peptide/protein comprising one or more non-standard amino acids selected from 2,4-diamino-3-hydroxybutyrate, L-5-hydroxylysine, L-3-hydroxyarginine and 2,3-didehydroarginine. The methods of the present invention are particularly useful in enzyme-assisted preparation of odilorhabdins and their derivatives. The present invention further relates to a polypeptide having 2,4-diaminobutyrate-3-hydroxylase activity and its use for catalyzing a hydroxylation at position 3 of 2,4-diaminobutyrate, to a polypeptide having having L-arginine 3-hydroxylase activity and its use for catalyzing a hydroxylation at position 5 of L-lysine, to a polypeptide having L-arginine 3-hydroxylase activity and its use for for catalyzing a hydroxylation at position 3 of L-arginine, and to a polypeptide having a deacylase activity, which is preferably capable of removing fatty acyl from the N-terminus of an odilorhabdin, and its use for catalyzing a deacylation, preferably for removing a fatty acyl from the N-terminus of an odilorhabdin.

## Description

The present invention relates to a novel method of preparing a non-standard amino acid or a peptide/protein comprising a non-standard amino acid residue, as appropriate, wherein said amino acid residue is selected from 2,4-diamino-3-hydroxybutyrate, L-5-hydroxylysine, L-3-hydroxyarginine, 2,3-didehydroarginine by using enzymatic transformations. Accordingly, the present invention further relates to a method of preparing a peptide/protein comprising one or more non-standard amino acids selected from 2,4-diamino-3-hydroxybutyrate, L-5-hydroxylysine, L-3-hydroxyarginine and 2,3-didehydroarginine. The methods of the present invention are particularly useful in enzyme-assisted preparation of odilorhabdins and their derivatives. The present invention further relates to a polypeptide having 2,4-diaminobutyrate-3-hydroxylase activity and its use for catalyzing a hydroxylation at position 3 of 2,4-diaminobutyrate, to a polypeptide having having L-arginine 3-hydroxylase activity and its use for catalyzing a hydroxylation at position 5 of L-lysine, to a polypeptide having L-arginine 3-hydroxylase activity and its use for for catalyzing a hydroxylation at position 3 of L-arginine, and to a polypeptide having a deacylase activity, which is preferably capable of removing fatty acyl from the N-terminus of an odilorhabdin, and its use for catalyzing a deacylation, preferably for removing a fatty acyl from the N-terminus of an odilorhabdin.

Intense research efforts have also been devoted to the characterization of unknown biosynthetic gene clusters (BGCs) for natural product discovery, as well as functional assignments of genes encompassed in said gene clusters. An example of such recently discovered products are odilorhabdins, which constitute a new family of 10-mer linear cationic peptide antibiotics inhibiting bacterial translation by binding to the 30S subunit of the ribosome. These bioactive secondary metabolites are produced by entomopathogenic bacterial symbiont *Xenorhabdus* (Morganellaceae), vectored by the soil-dwelling nematodes. More specifically, odilorhabdins are produced by the enzymes of the non-ribosomal peptide synthetase gene cluster of the nematode-symbiotic bacterium *Xenorhabdus nematophila.*

Odilorhabdins show activity against Gram-positive and Gram-negative pathogens, including carbapenem-resistant Enterobacteriaceae, and can eradicate infections in animal models. Odilorhabdin (NOSO-95) is a promising new antibiotic described first in 2018. It shows binding to a new target site at the ribosome and therefore might be an interesting lead compound and potential clinical drug. It is a linear peptide made of 10 amino acids, six of them being non-proteinogenic.

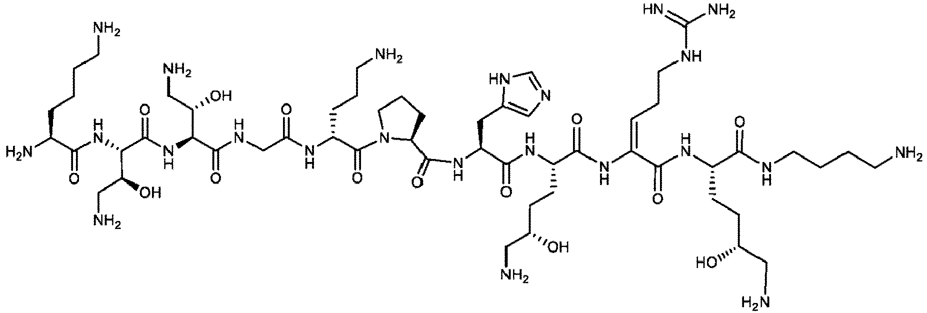

A shortened and simplified version of it (NOSO-95179) is made synthetically but still contains two non-proteinogenic amino acids that are difficult and expensive to synthesize. NOSO-95179 (and other odilorhabins) are currently produced synthetically requiring these modified amino acids (as Fmoc or Boc-protected amino acids) that are expensive to synthesize. This hampers access to large amounts of the compound and its subsequent clinical application.

Accordingly, the invention provides means and methods for preparing a non-standard amino acid or a peptide/protein comprising a non-standard amino acid residue, selected from 2,4-diamino-3-hydroxybutyrate, L-5-hydroxylysine, L-3-hydroxyarginine and 2,3-didehydroarginine. The invention is based, at least in part, on a surprising discovery of the function of polypeptides belonging to odilorhabdin biosynthetic gene cluster. The methods of the present invention are particularly useful in the synthesis of odilorhabdins, but can be applied to any peptide/protein comprising a non-standard amino acid residue, selected from 2,4-diamino-3-hydroxybutyrate, L-5-hydroxylysine, L-3-hydroxyarginine and 2,3-didehydroarginine.

In some aspects the present invention is based, at least in part, on a surprising discovery of the present inventors that the protein product encoded by odlB gene in Xhenorabdus nematophila and previous described as conserved hypothetical protein, putative Cupin_8 domain or Clavaminate synthase-like domain is actually an enzyme with 2,4-diaminobutyrate 3-hydroxylase. The present invention is further based on a surprising discovery of the present inventors that the protein product encoded by odlE gene in Xhenorhabdus nematophila and annotated as conserved hypothetical protein is in fact lysine-6-hydroxylase. Finally, the present invention is further based, at least in part, on a surprising discovery that the protein encoded by odlF gene as aspartyl/asparaginyl b-hydrolase domain and isopenicillin N-synthase like is in fact an enzyme with arginine-3-hydroxylase activity.

The invention will be summarized in the following embodiments.

In a first embodiment, the present invention relates to a method of preparing a non-standard amino acid or a peptide/protein comprising a non-standard amino acid residue, wherein the non-standard amino acid is 2,4-diamino-3-hydroxybutyrate, the method comprising the step of contacting 2,4-diaminobutyrate with an enzyme having 2,4-diaminobutyrate 3-hydroxylase activity.

In a second embodiment, the present invention relates to a method of preparing a non-standard amino acid or a peptide/protein comprising a non-standard amino acid residue, wherein the non-standard amino acid is L-5-hydroxylysine, the method comprising the step of contacting L-lysine or a peptide/protein comprising an L-lysine residue with an enzyme having L-lysine 5-hydroxylase activity.

In a third embodiment, the present invention relates to a method of preparing L-3-hydroxyarginine or a peptide/protein comprising a non-standard amino acid residue, wherein the non-standard amino acid is L-3-hydroxyarginine, the method comprising the step of contacting L-arginine with an enzyme having L-arginine 3-hydroxylase activity.

In a fourth embodiment, the present invention relates to a method of preparing a peptide/protein comprising a non-standard amino acid residue, wherein the non-standard amino acid is 2,3-didehydroarginine, the method comprising the step of contacting a peptide/protein comprising an L-3-hydroxyarginine residue with an enzyme having L-3-hydroxyarginine dehydratase activity, wherein a peptide/protein comprising a 2,3-didehydroarginine residue is formed.

In a fifth embodiment, the present invention relates to a method of preparing a peptide/protein comprising one or more non-standard amino acids selected from 2,4-diamino-3-hydroxybutyrate, L-5-hydroxylysine, L-3-hydroxyarginine and 2,3-didehydroarginine, the method comprising the step(s) as defined in any one of preceding embodiments.

In a sixth embodiment, the present invention relates to use of a polypeptide comprising an amino acid sequence with at least 80% sequence identity to SEQ ID NO: 1 for catalyzing a hydroxylation at position 3 of 2,4-diaminobutyrate.

In a seventh embodiment, the present invention relates to use of a polypeptide comprising an amino acid sequence with at least 80% sequence identity to SEQ ID NO: 2 for catalyzing a hydroxylation at position 5 of L-lysine.

In an eight embodiment, the present invention relates to use of a polypeptide comprising an amino acid sequence with at least 80% sequence identity to SEQ ID NO: 3 for catalyzing a hydroxylation at position 3 of L-arginine.

In a ninth embodiment, the present invention relates to use of a polypeptide comprising an amino acid sequence with at least 80% sequence identity to SEQ ID NO: 4 for catalyzing a deacylation, preferably for removing a fatty acyl from the N-terminus of an odilorhabdin.

In a tenth embodiment, the present invention relates to use of a polypeptide comprising an amino acid sequence with at least 80% sequence identity to SEQ ID NO: 5 for catalyzing a dehydratation reaction of L-3-hydroxyarginine. In an elevent embodiment, the present invention relates to a polypeptide comprising an amino acid sequence with at least 80% sequence identity to SEQ ID NO: 1 and having 2,4-diaminobutyrate-3-hydroxylase activity, wherein said polypeptide is not a polypeptide consisting of the amino acid sequence according to SEQ ID NO: 1.

In a twelfth embodiment, the present invention relates to a polypeptide comprising an amino acid sequence with at least 80% sequence identity to SEQ ID NO: 2 and having L-lysine 5-hydroxylase activity, wherein said polypeptide is not a polypeptide consisting of the amino acid sequence according to SEQ ID NO: 2.

In a thirteenth embodiment, the present invention relates to a polypeptide comprising an amino acid sequence with at least 80% sequence identity to SEQ ID NO: 3 and having L-arginine 3-hydroxylase activity, wherein said polypeptide is not a polypeptide consisting of the amino acid sequence according to SEQ ID NO: 3.

In a fourteenth embodiment, the present invention relatesto a polypeptide comprising an amino acid sequence with at least 80% sequence identity to SEQ ID NO: 4 and having a deacylase activity, which is preferably capable of removing fatty acyl from the N-terminus of an odilorhabdin, wherein said polypeptide is not a polypeptide consisting of the amino acid sequence according to SEQ ID NO: 4.

In a fifteenth embodiment, the present invention relatesto a polypeptide comprising an amino acid sequence with at least 80% sequence identity to SEQ ID NO: 5 and having a L-3-hydroxyarginine dehydratase activity, wherein said polypeptide is not a polypeptide consisting of the amino acid sequence according to SEQ ID NO: 5.

The invention is further illustrated by the appended figures.

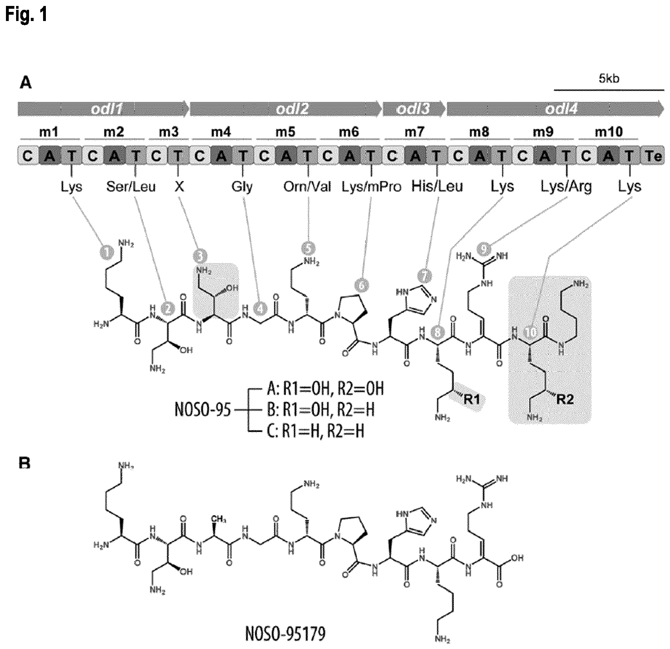

- **Figure 1**: illustrates **(A)** the responsibility of different modules of non-ribosomal peptide synthetase encoded by the genes odl1, odl2, odl3 and odl4 for extension of a particular amino acid residue in the synthesis of odilorhabdin; and **(B)** a simplified version of odilorhabdin, NOSO-95179.

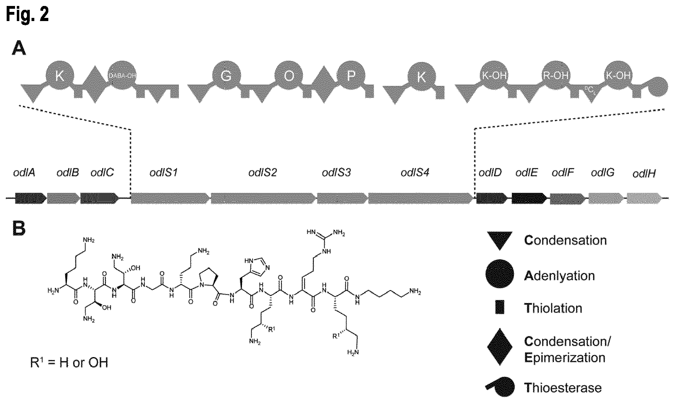

- **Figure 2**: shows: (A) Cluster overview of the odilorhabdin BGC and legend of NRPS domains. (B) Structure of odilorhabdin.

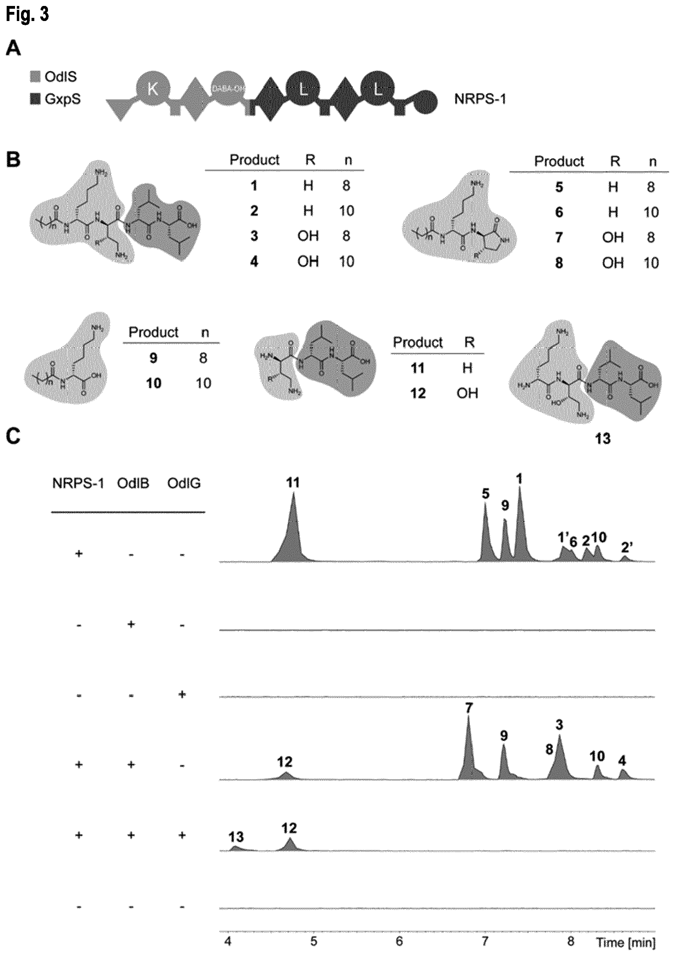

- **Figure 3**: presents: (A) Schematic representation of NRPS-1. (B) Structures of products **1** to **13** detected for NRPS-1 alone and either co-expressed with *odIB* or *odIB* and *odIG*. The origin of the peptides amino acid building blocks is highlighted in the color of the corresponding BGCs. (C) HPLC/MS data of compounds **1** to **13** produced in E. *coli* DH10B::*mtaA* expressing *NRPS-1* with *odIB* and *odIG*. Minus (-) indicates corresponding empty vector control while plus (+) refers to expression of the gene. Extracted Ion Chromatograms (EIC) of **1** (*m*/*z* [M+2H]²⁺ = 314.24), **2** (*m*/*z* [M+2H]²⁺ = 328.25), **3** (*m*/*z* [M+2H]²⁺ = 322.24), **4** (*m*/*z* [M+2H]²⁺ = 336.25), **5** (*m*/*z* [M+H]⁺ = 383.30), **6** (*m*/*z* [M+H]⁺ = 411.33), **7** (*m*/*z* [M+H]⁺ = 399.29), **8** (*m*/*z* [M+H]⁺ = 427.32), **9** (*m*/*z* [M+H]⁺ = **301.24), 10** (*m*/*z* [M+H]⁺ = 329.27), **11** (*m*/*z* [M+H]⁺ = **345.24), 12** (*m*/*z* [M+H]⁺ = 361.24) and **13** (*m*/*z* [M+H]⁺ = 245.17).

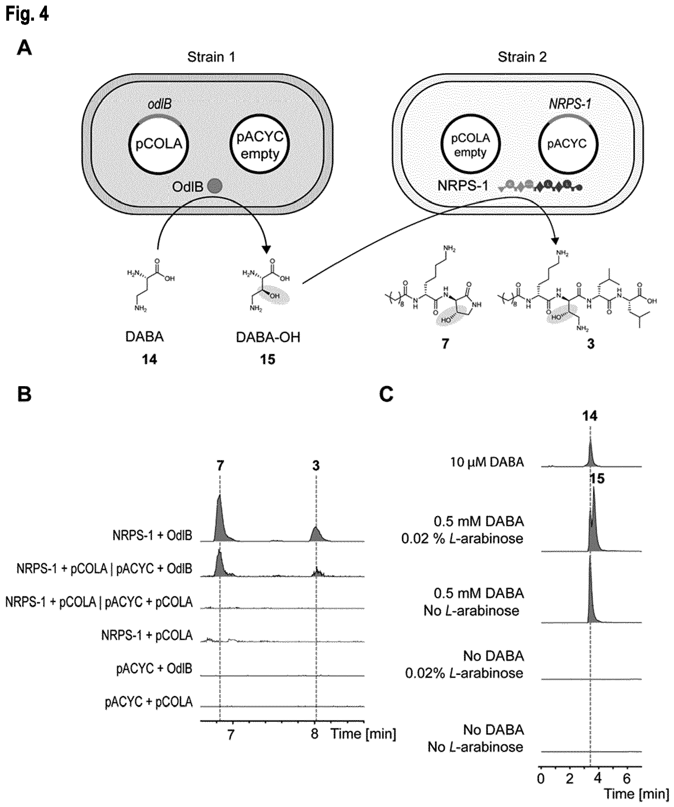

- **Figure 4**: shows: (A) Schematic representation of the cross-feeding experiment with NRPS-1 and OdIB. (B) HPLC/MS data of compounds **3** and **7** produced in *E. coli* DH10B::*mtaA* expressing *NRPS-1* with *odIB* in a cross-feeding experiment. Straight line (|) indicates two different strains with different set of plasmids co-cultivated in the same culture medium. Depicted are EICs of compound **3** (*m*/*z* [M+2H]²⁺ = 322.24) and **7** *(m*/*z* [M+H]⁺ = 399.29). EIC signal intensity corresponding to NRPS-1 and OdIB is 15 times decreased compared to all other signals. (C) HPLC/MS data of compounds small metabolite analysis of *odIB* expressing *E*. *coli* DH10B::*mtaA* under the depicted conditions. EIC of compound **14** (*m*/*z* [M+H]⁺ = 119.08) and **15** (*m*/*z* [M+H]⁺ = 135.07).

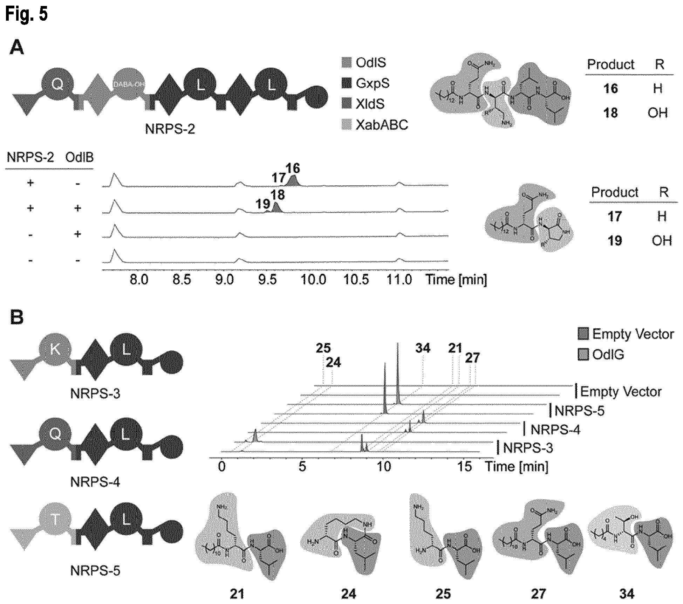

- **Figure 5**: presents: (A) Schematic representation of NRPS-2. HPLC/MS base peak chromatograms (BPC) of compounds **16** to **19** produced in *E*. *coli* DH10B::*mtaA* expressing *NRPS-2* with or without co-expression of *odIB.* Chemical structures with respective peptide origin highlighted in the color of the corresponding BGC. (B) Schematic representation of NRPS-3, -4 and -5. HPLC/MS data of compounds **21, 24, 25, 27** and **34** produced in *E. coli* DH10B::*mta*A expressing *NRPS-3, -4* or *-5* with or without co-expression of *odIG* with 24 hours delay. Depicted are EIC's of compound **21** (*m*/*z* [M+H]⁺ =422.36), **24** (*m*/*z* [M+H]⁺ = 242.18)**, 25** (m/z[M+H]⁺ =260.19), **27** (*m*/*z* [M+H]⁺ = 442.32) and **34** (*m*/*z* [M+H]⁺ = 331.22). The origin of the peptides amino acid building blocks are highlighted in the color of the corresponding BGCs.

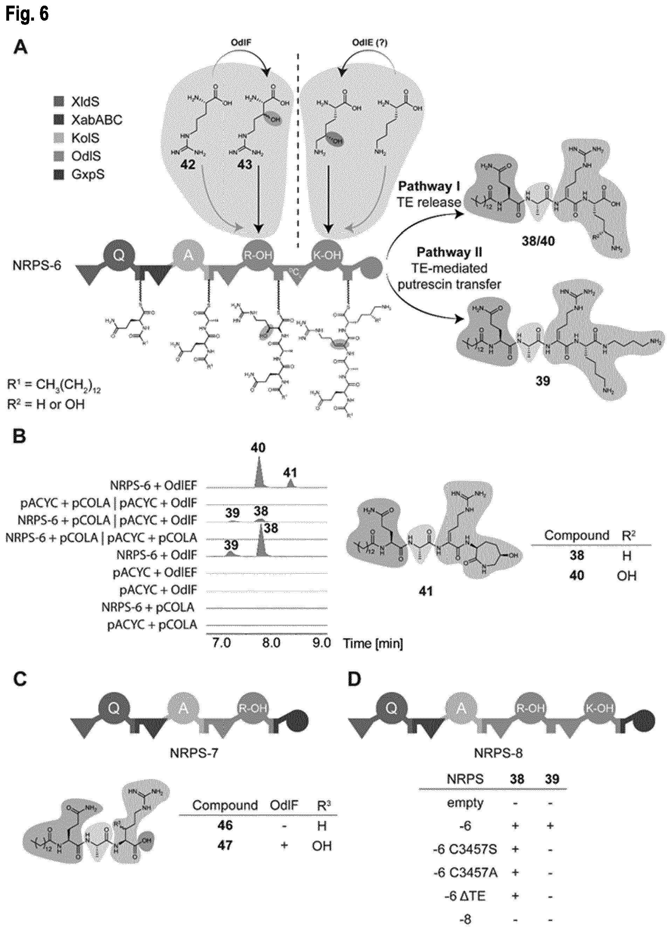

- **Figure 6**: presents: (A) Schematic representation of NRPS-6 and the biosynthesis mechanism involving OdIF and OdIE. Product structures with respective peptide origin are highlighted in the color of the corresponding BGC. (B) HPLC/MS data of compounds **38, 39, 40** and **41** produced in *E*. *coli* DH10B::*mtaA* expressing *NRPS-6* with *odIF* or *odIEF.* EIC's of compound **38** (*m*/*z* [M+2H]²⁺ =355.74), **39** (*m*/*z* [M+2H]²⁺ = 390.79) , **40** (*m*/*z* [M+2H]²⁺ = 363.74) and **41** (*m*/*z* [M+H]⁺ = 708.47) are shown. Furthermore, a cross-feeding experiment is included where a straight line (|) indicates two different strains with different sets of plasmids co-cultivated in the same medium. (C) Schematic representation of NRPS-7 and its product formation of either **46** or **47** with and without co-expression of *odIF.* (D) Schematic representation of NRPS-8. Table of NRPS-6 variants and NRPS-8 with their ability to produce compound **38** and **39** when co-expressed with *odIF.*

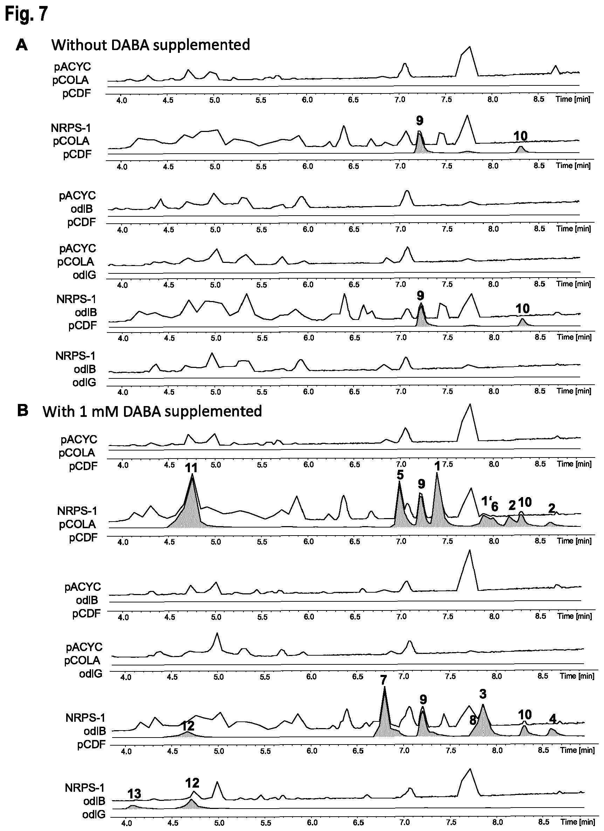

- **Figure 7**: presents: HPLC/MS data referred to Figure 2 of compounds **1** to **13** produced in *E*. *coli* DH10B::*mta*A expressing *NRPS-1* with *odIB* and *odIG*. *NRPS-1* was expressed from pACYC, *odIB* from pCOLA and *odIG* from pCDF plasmid. pACYC, pCOLA and pCDF served as empty plasmid controls. Base Peak Chromatogram (BPC, top) and Extracted Ion Chromatograms (EIC, below) of **1** (*m*/*z* [M+2H]²⁺ = 314.24), **2** (*m*/*z* [M+2H]²⁺ = 328.25), **3** (*m*/*z* [M+2H]²⁺ = 322.24), **4** (*m*/*z* [M+2H]²⁺ = 336.25), **5** (*m*/*z* [M+H]⁺ = 383.30), **6** (*m*/*z* [M+H]⁺ = 411.33), **7** (*m*/*z* [M+H]⁺ = 399.29), **8** (*m*/*z* [M+H]⁺ = 427.32), **9** (*m*/*z* [M+H]⁺ = **301.24), 10** (*m*/*z* [M+H]⁺ = 329.27), **11** (*m*/*z* [M+H]⁺ = 345.24), **12** (*m*/*z* [M+H]⁺ = 361.24) and **13** (*m*/*z* [M+H]⁺ = 245.17). No DABA was supplemented for cultures analyzed in (A) while 1 mM DABA was added to cultures analyzed in (B).

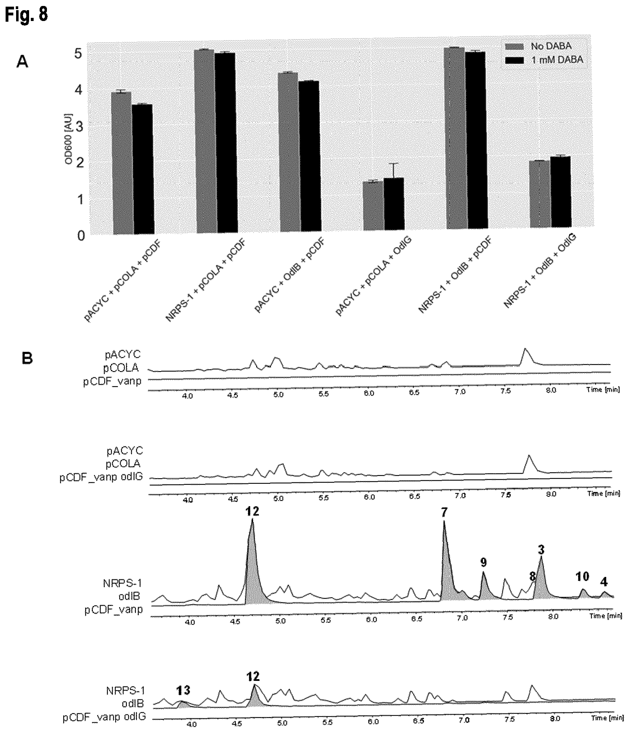

- **Figure 8**: shows (A) OD₆₀₀ of *E*. *coli* DH10B::*mtaA* expressing pACYC, pCOLA or pCDF containing *NRPS-1, odIB* or *odIG* after 72 hours growth at 22 °C in XPPM supplemented with 0.02% *L*-arabinose and with and without 1 mM DABA. (B) HPLC/MS data of compounds **3, 4, 7, 8, 9, 10, 12** and **13** produced in *E*. *coli* DH10B::*mtaA* expressing *NRPS-1* with *odlB* and *odlG* in XPP medium supplemented with 1 mM DABA. *NRPS-1* was expressed from pACYC, *odlB*from pCOLA and *odlG* from pCDF_vanp plasmid. pACYC, pCOLA and pCDF_vanp served as empty plasmid controls. All cultures were induced with 0.02% *L-*arabinose. After 24 hours 100 µM vanillic acid was added to induce *odIG*. Base Peak Chromatogram (BPC, top) and Extracted Ion Chromatograms (EIC, below) of **3** (*m*/*z* [M+2H]²⁺ = 322.24), **4** (*m*/*z* [M+2H]²⁺ = 336.25), **7** (*m*/*z* [M+H]⁺ = 399.29), **8** (*m*/*z* [M+H]⁺ = 427.32), **9** (*m*/*z* [M+H]⁺ = 301.24), **10** (*m*/*z* [M+H]⁺ = 329.27), **12** (*m*/*z* [M+H]⁺ = 361.24) and **13** (*m*/*z* [M+H]⁺ = 245.17).

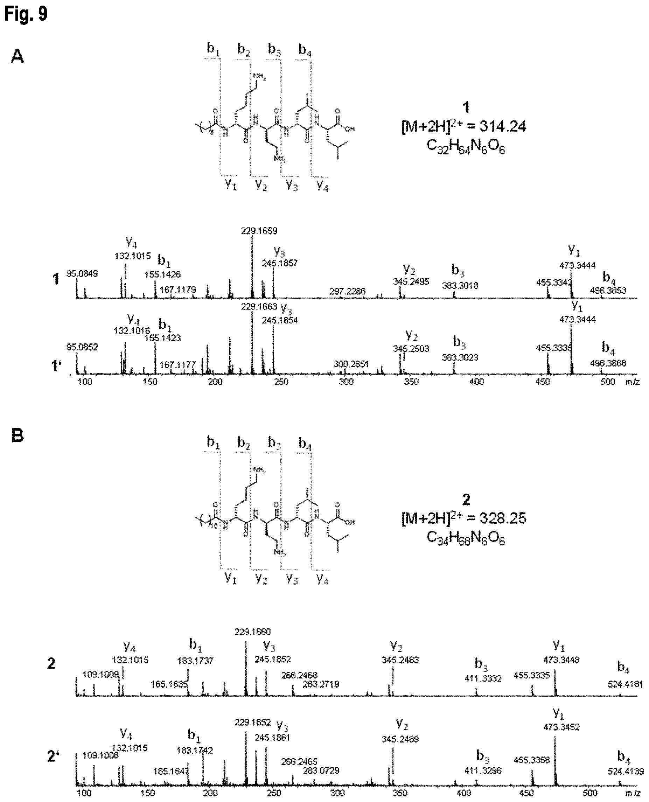

- **Figure 9**: presents: (A) Fragmentation HPLC/MS data refers to Figure 2 and Figure 7B of compound **1** (A) and **2** (B) produced in *E*. *coli* DH10B::*mtaA* expressing *NRPS-1* in XPP medium supplemented with 1 mM DABA

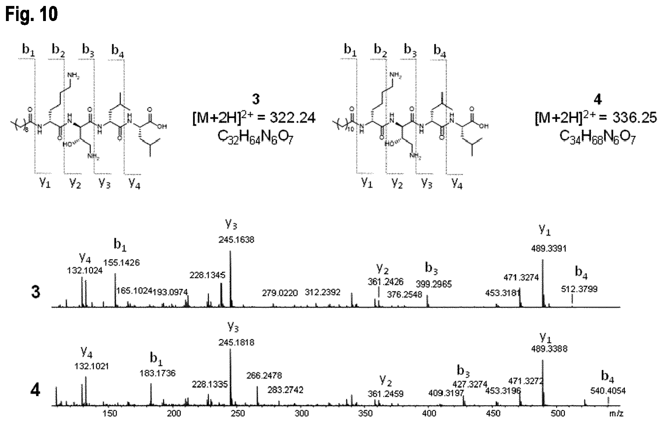

- **Figure 10**: shows: (A) Fragmentation HPLC/MS data refers to Figure 2 and Figure 7B of compound **3** (A) and **4** (B) produced in *E*. *coli* DH10B::*mtaA* expressing *NRPS-1* and *odIB* in XPP medium supplemented with 1 mM DABA.

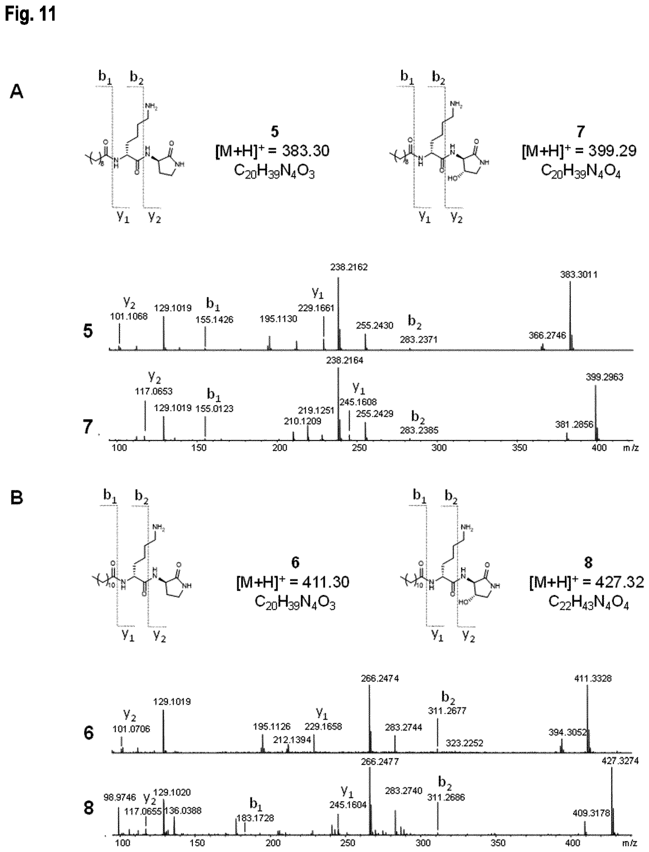

- **Figure 11**: shows: (A) Fragmentation HPLC/MS data refers to Figure 2 and Figure 7B (NRPS-1) of compound **5** (A) and **6** (B) produced in *E*. *coli* DH10B::*mtaA* expressing *NRPS-1* and compound **7** (A) and **8** (B) produced in *E*. *coli* DH10B::*mtaA* expressing *NRPS-1* and *odIB* in XPP medium supplemented with 1 mM DABA.

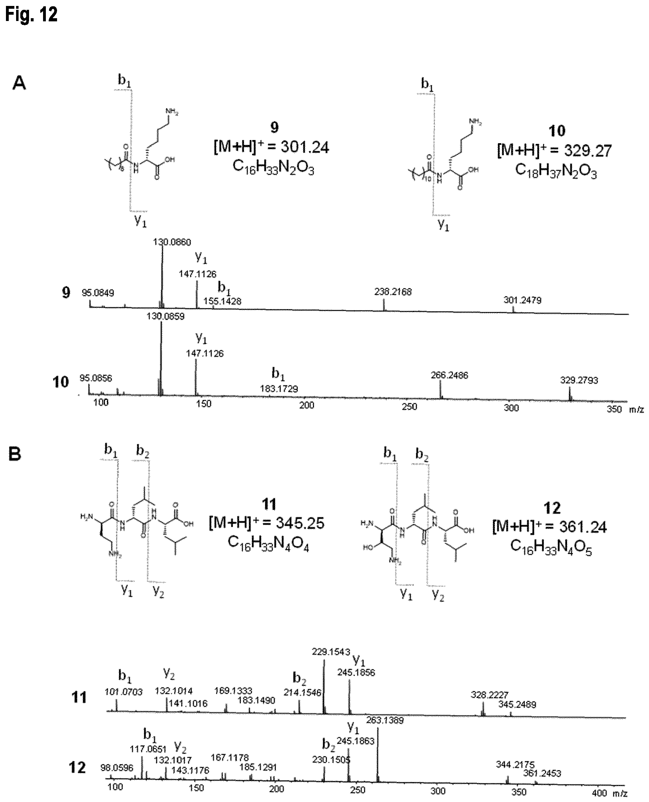

- **Figure 12**: presents: (A) Fragmentation HPLC/MS data refers to Figure 2 and Figure 7B (NRPS-1) of compound **9, 10** (A) and **11** (B) produced in *E*. *coli* DH10B::*mtaA* expressing *NRPS-1* and compound **12** (B) produced in *E*. *coli* DH10B::*mtaA* expressing *NRPS-1* and *odIB* in XPP medium supplemented with 1 mM DABA.

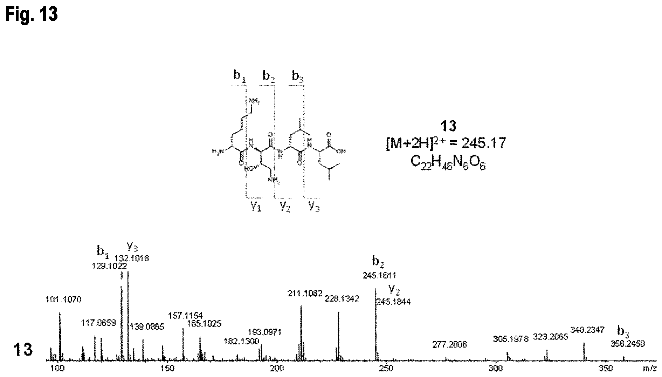

- **Figure 13**: presents: (A) Fragmentation HPLC/MS data refers to Figure 2 and Figure 7B (NRPS-1) of compound **13** produced in *E*. *coli* DH10B:*:mtaA* expressing *NRPS-1, odIB and odIG* in XPP medium supplemented with 1 mM DABA.

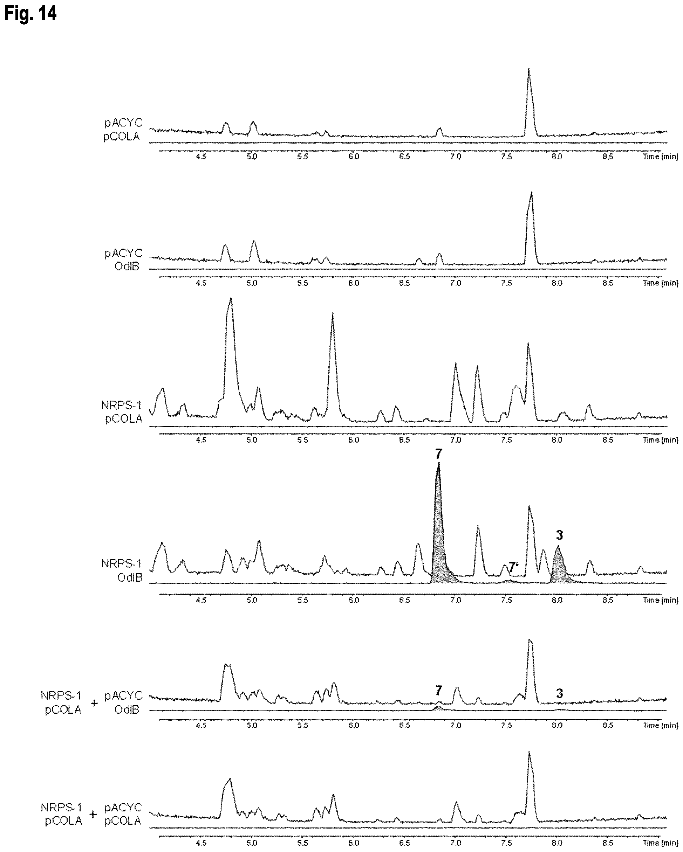

- **Figure 14**: presents: HPLC/MS data referred to Figure 3A and 3B of compounds **3** and **7** produced in *E*. *coli* DH10B::*mtaA* expressing *NRPS-1* and *odIB. NRPS-1* was expressed from pACYC while *odIB* was expressed from pCOLA plasmid. pACYC and pCOLA served as empty plasmid controls. Base Peak Chromatogram (BPC, top) and Extracted Ion Chromatograms (EIC, below) of **3** (*m*/*z* [M+2H]²⁺ = 322.24) and **7** *(m*/*z* [M+H]⁺ = 399.29). XPP medium was supplemented with 1 mM DABA. The last two spectra were obtained from cross-feeding experiments co-cultivating two *E*. *coli* DH10B::*mtaA* strains containing the specified plasmid pairs.

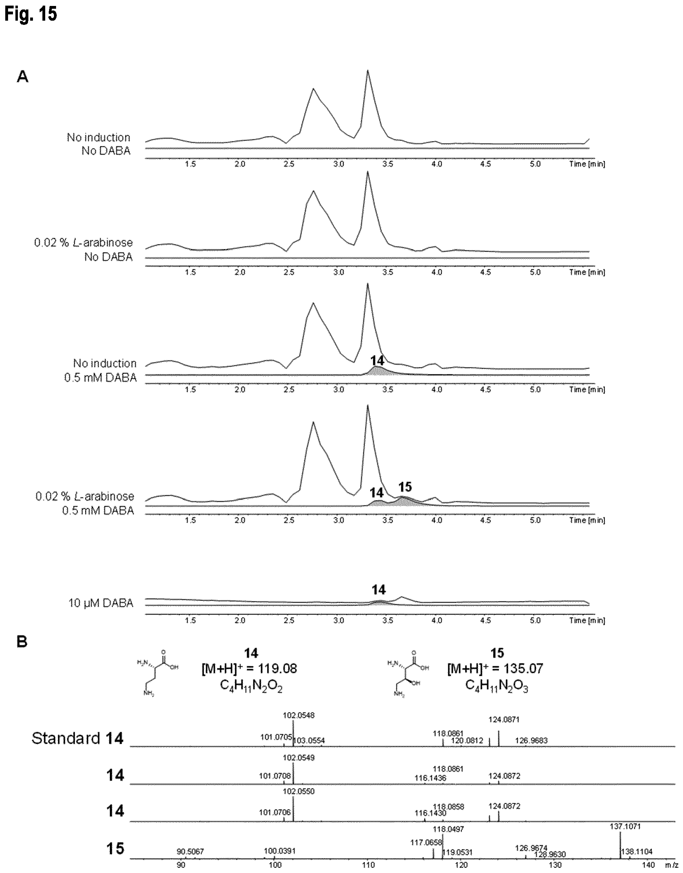

- **Figure 15**: shows: (A) HPLC/MS data of small metabolite analysis referred to figure 3C of compounds **14** and **15** produced in *E*. *coli* DH10B::*mta*A expressing *odIB* in XPP medium with the noted supplements. The strain carried an empty pACYC plasmid and pCOLA containing *odIB.* Base Peak Chromatogram (BPC, top) and Extracted Ion Chromatograms (EIC, below) of **14** *(m*/*z* [M+H]⁺ = 119.08) and **15** *(m*/*z* [M+H]⁺ = 135.07). The spectra were compared to a 10 µM DABA standard. (B) Fragmentation HPLC/MS data of compound **14** and **15** produced in *E*. *coli* DH10B::*mtaA* expressing *odIB.*

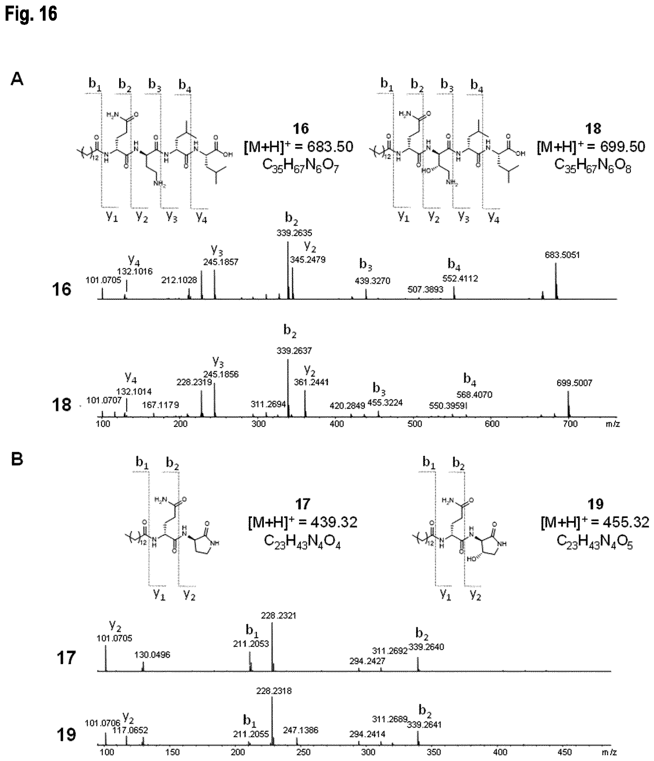

- **Figure 16**: shows: HPLC/MS data referred to Figure 5A of compounds **16** to **19** produced in E. coli DH10B::mtaA expressing NRPS-2 and odIB in XPP medium with 1 mM DABA supplemented. NRPS-2 was cloned to pACYC plasmid while pCOLA containing odIB. Fragmentation HPLC/MS data of compound **16** and **18** (A) and **17** and **19** produced in E. coli DH10B::mtaA expressing NRPS-1 with or without odIB.

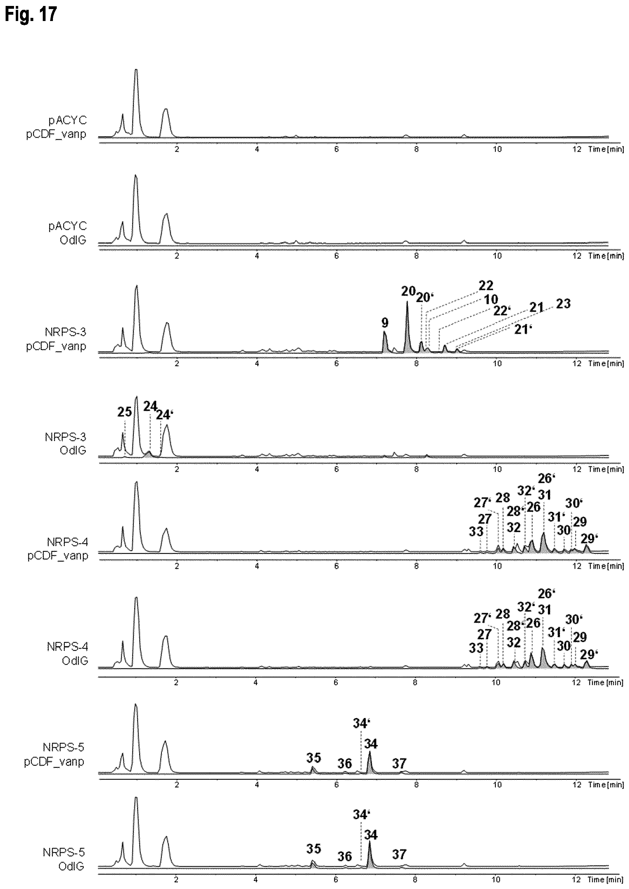

- **Figure 17**: shows: HPLC/MS data referred to Figure 5B (NRPS-3, -4 and -5) of compounds **9, 10** and **20** to **37** produced in *E*. *coli* DH10B::*mtaA*. *NRPS-3, -4 or -5* was cloned on a pACYC plasmid while *odIG* was cloned to a pCDF_vanp plasmid. All cultures were induced with 0.02% *L*-arabinose. After 24 hours 100 µM vanillic acid was added to induce *odIG*. pCDF_vanp and pACYC served as an empty vector control. Base Peak Chromatogram (BPC, top) and Extracted Ion Chromatograms (EIC, below) of **9** (*m*/*z* [M+H]⁺ = 301.24), **10** (*m*/*z* [M+H]⁺ = 329.27), **20** (*m*/*z* [M+H]⁺ = 414.33), **21** (*m*/*z* [M+H]⁺ = 442.36), **22** (*m*/*z* [M+H]⁺ = 440.34), **23** (*m*/*z* [M+H]⁺ = 396.32), **24** (*m*/*z* [M+H]⁺ = 242.18), **25** (*m*/*z* [M+H]⁺ = 260.19), **26** (*m*/*z* [M+H]⁺ = 470.35), **27** (*m*/*z* [M+H]⁺ = 442.32), **28** (*m*/*z* [M+H]⁺ = 468.34), **29** (*m*/*z* [M+H]⁺ = 498.38), **30** (*mlz*[M+H]⁺=484.37), **31** (*mlz*[M+H]⁺=496.37), **32** (*mlz*[M+H]⁺=488.31), **33** (*mlz*[M+H]⁺=460.28), **34** (*m*/*z* [M+H]⁺ = 331.22), **35** (*m*/*z* [M+H]⁺ = 303.19), **36** (*m*/*z* [M+H]⁺ = 349.17) and **37** (*m*/*z* [M+H]⁺ = 345.23).

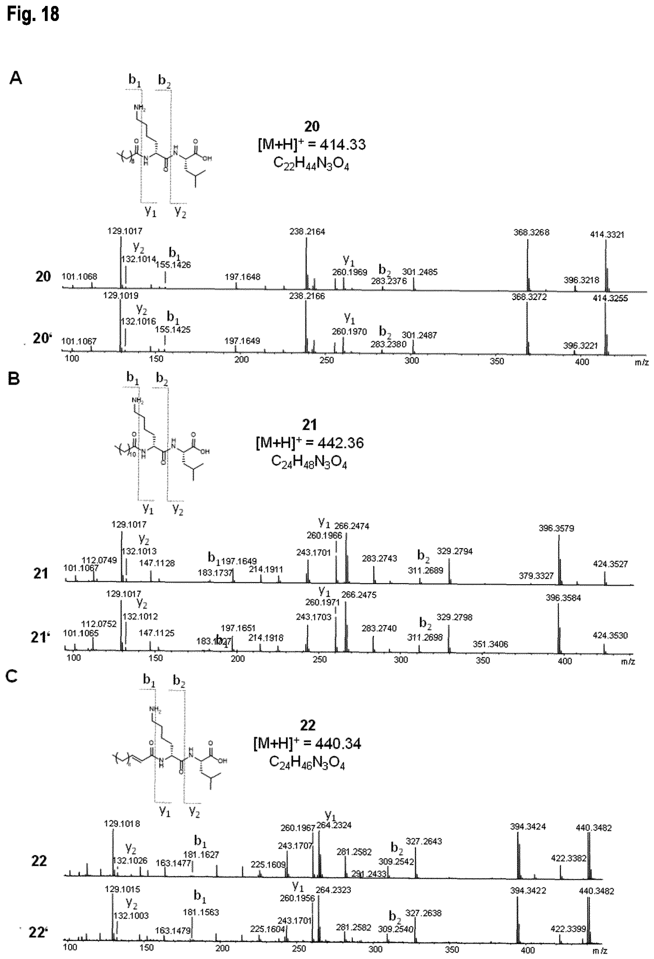

- **Figure 18**: shows fragmentation HPLC/MS data referred to Figure 5B and Figure 17 of compound **20** (A), **21 (**B) and **22** (C) produced in *E*. *coli* DH10B::*mtaA* expressing *NRPS-3.*

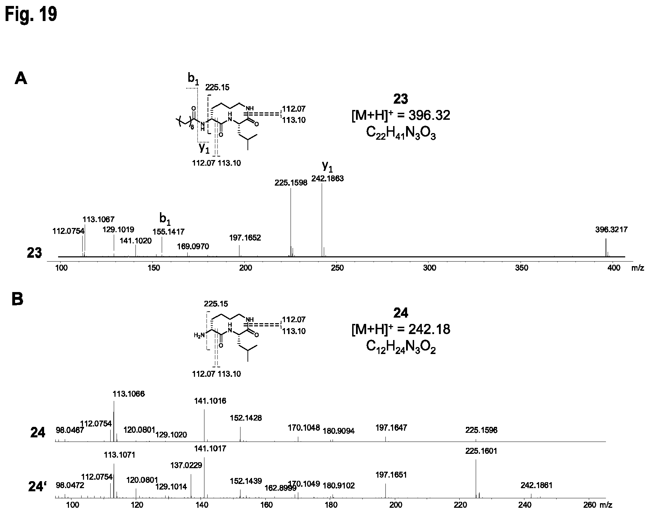

- **Figure 19**: shows: Fragmentation HPLC/MS data referred to Figure 5B and Figure 17 of compound **23** (A) and **24** (B) produced in E. *coli* DH10B::mtaA expressing NRPS-3 with **(24)** or without **(23)** *odIG*.

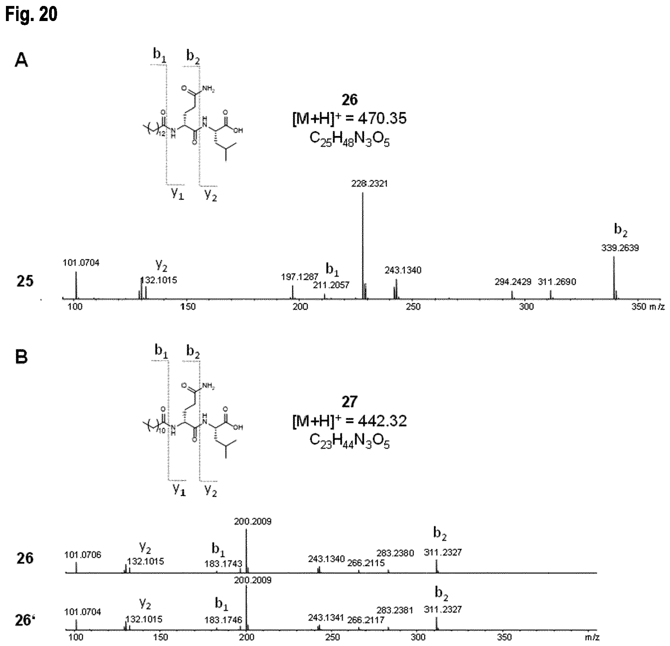

- **Figure 20**: shows: Fragmentation HPLC/MS data referred to Figure 5B and Figure 17 of compound **26** (A) and **27** (B) produced in *E*. *coli* DH10B::*mtaA* expressing *NRPS-4* with or without *odIG*.

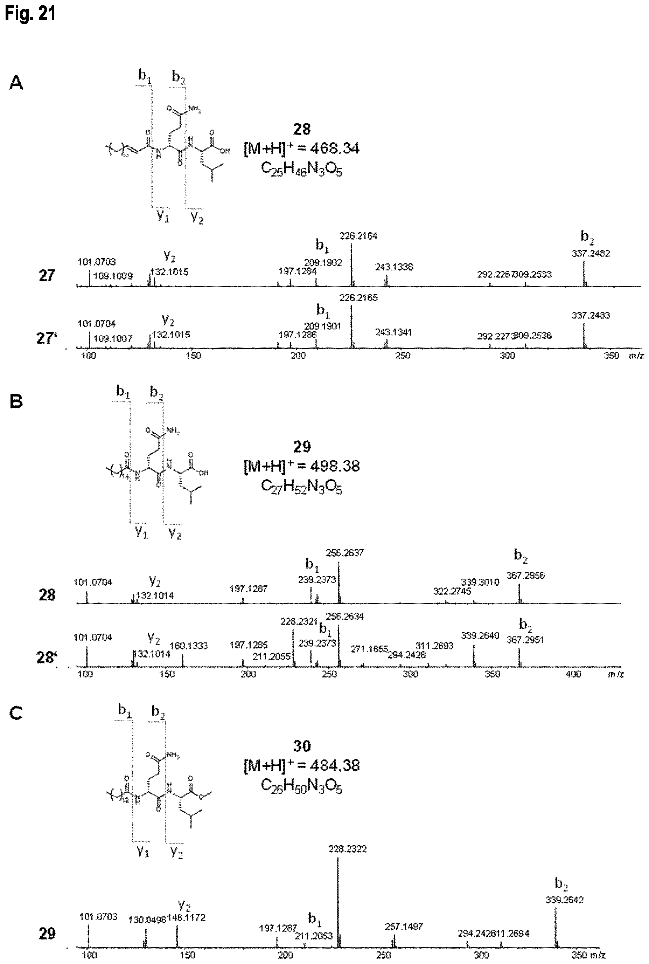

- **Figure 21**: shows: Fragmentation HPLC/MS data referred to Figure 5B and Figure 17 of compound **28** (A), **29** (B) and **30** (C) produced in *E*. *coli* DH10B::*mtaA* expressing *NRPS-4* with or without *odIG*.

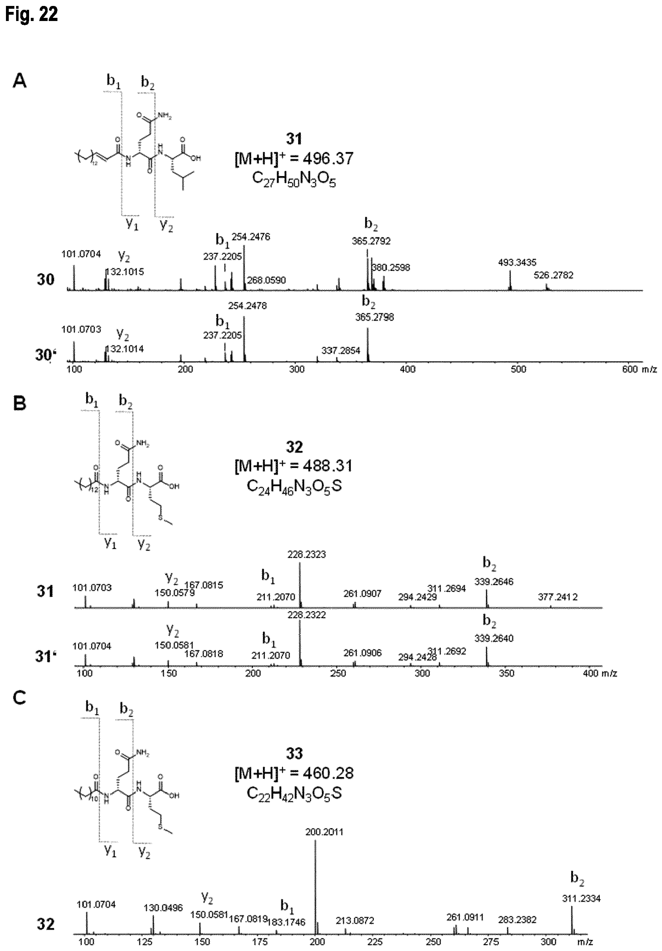

- **Figure 22**: shows: Fragmentation HPLC/MS data referred to Figure 5B and Figure 17 of compound **31** (A), **32** (B) and **33** (C) produced in *E*. *coli* DH10B::*mtaA* expressing *NRPS-4* with or without *odIG*.

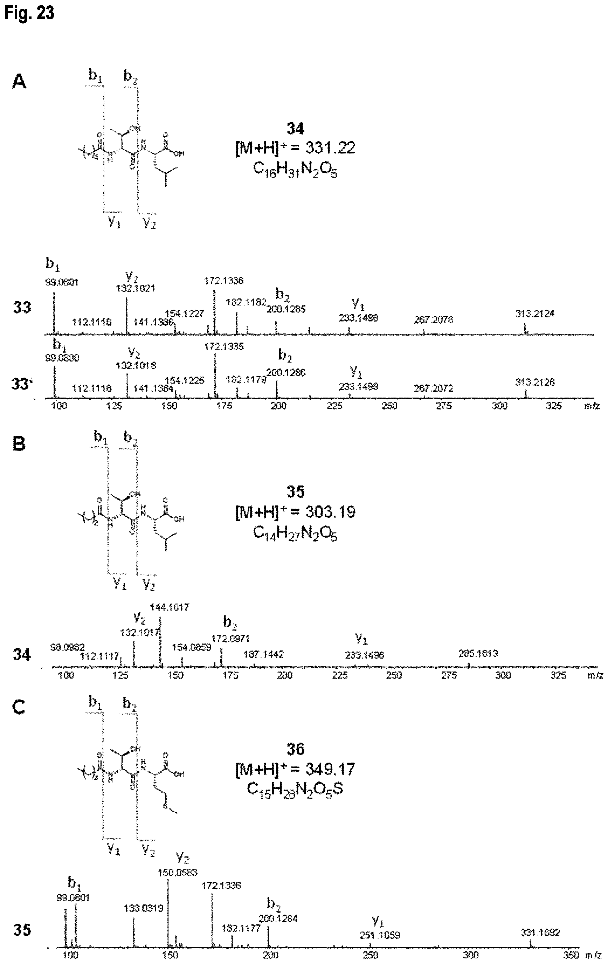

- **Figure 23**: shows: Fragmentation HPLC/MS data referred to Figure 5B and Figure 17 of compound **34** (A), **35** (B) and **36** (C) produced in *E*. *coli* DH10B::*mtaA* expressing *NRPS-5* with or without *odIG*.

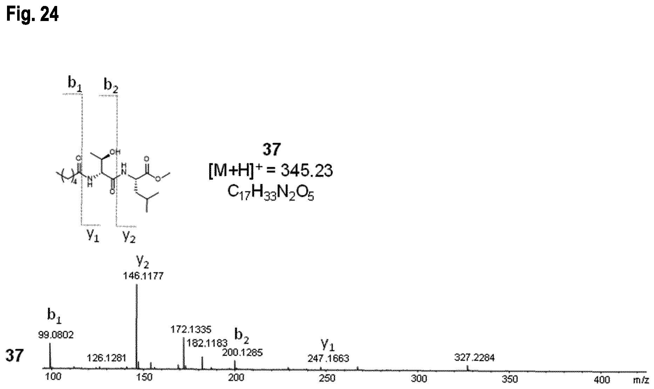

- **Figure 24**: shows: Fragmentation HPLC/MS data referred to Figure 5B and Figure 17 of compound **37** produced in *E. coli* DH10B::*mtaA* expressing NRPS-5 with or without *odIG*.

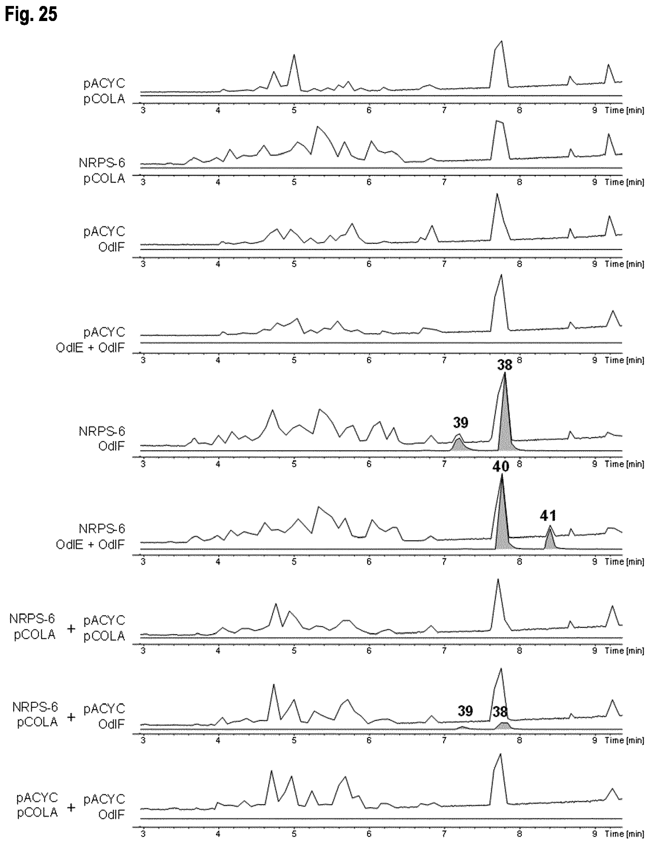

- **Figure 25**: shows: HPLC/MS data referred to Figure 6A and B (NRPS-6) of compounds **38** to **41** produced in *E*. *coli* DH10B::*mtaA. NRPS-6* was cloned on a pACYC plasmid while *odIF* was cloned to a pCOLA plasmid. All cultures were induced with 0.02% *L*-arabinose*.* pACYC and pCOLA served as an empty vector control. Base Peak Chromatogram (BPC, top) and Extracted Ion Chromatograms (EIC, below) of **38** (*m*/*z* [M+2H]²⁺ = 355.74), **39** (*m*/*z* [M+2H]²⁺ = 390.79), **40** (*m*/*z* [M+2H]²⁺ = 363.74) and **41** (*m*/*z* [M+H]⁺ = 708.47). The last three spectra were obtained from cross-feeding experiments co-cultivating two *E*. *coli* DH10B::*mtaA* strains containing the specified plasmid pairs.

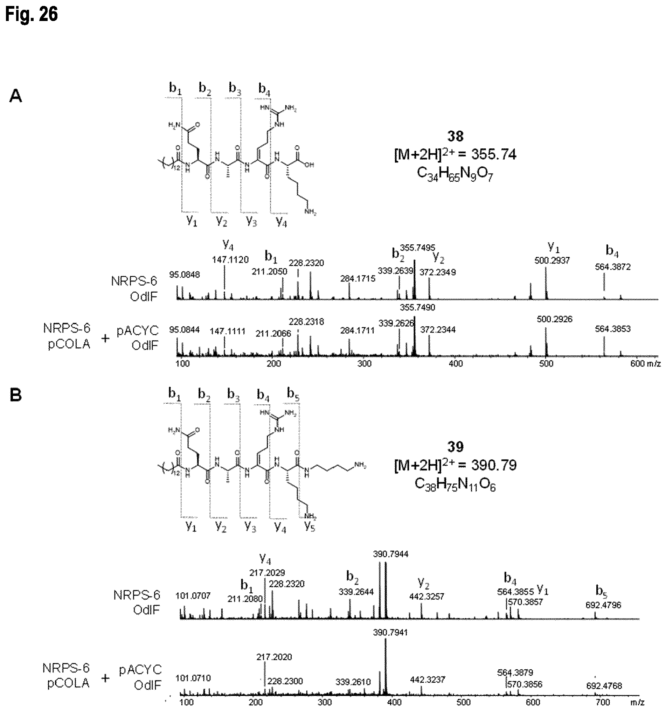

- **Figure 26**: shows: Fragmentation HPLC/MS data referred to Figure 6B and Figure 25 of compound **38** (A) and **39** (B) produced in *E*. *coli* DH10B::*mtaA* expressing *NRPS-6* with *odIF.* The fragmentation pattern between cells expressing both genes in the same cell and a cross-feeding experiment, expressing the genes in separate cells, were compared.

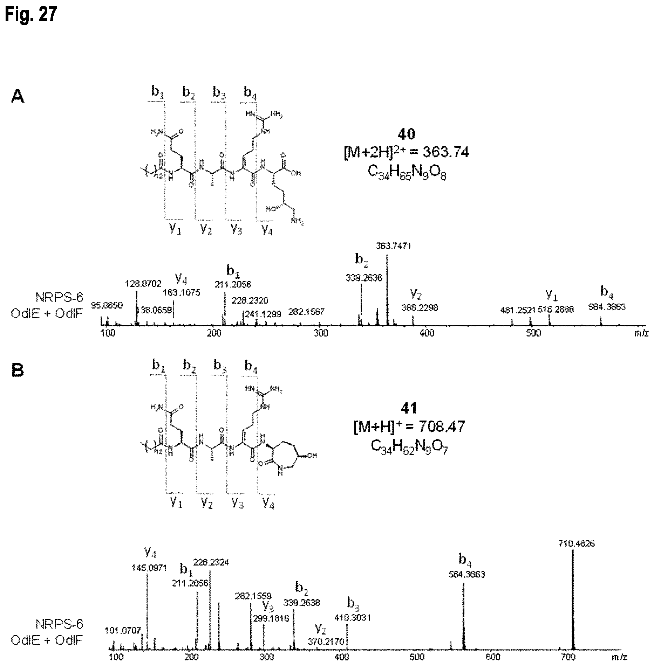

- **Figure 27**: shows: Fragmentation HPLC/MS data referred to Figure 6B and Figure 25 of compound **40** (A) and **41** (B) produced in *E*. *coli* DH10B::*mta*A expressing *NRPS-6* with *odIE* and *odIF.*

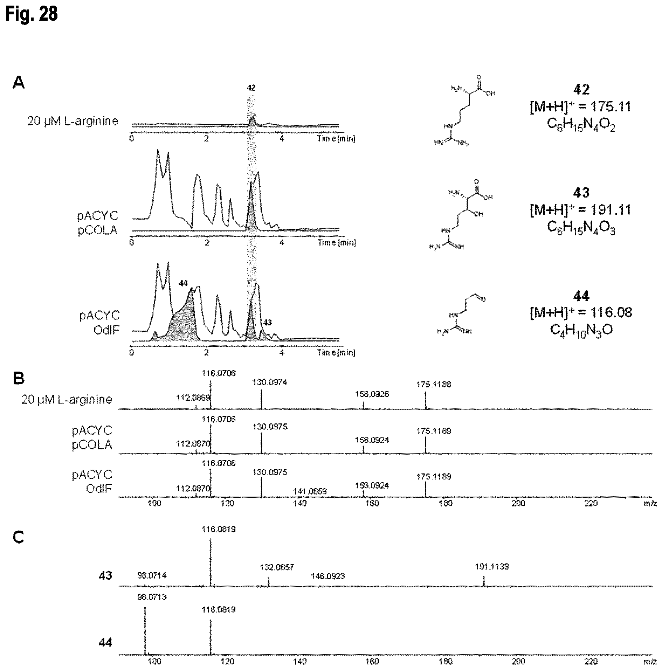

- **Figure 28**: shows (A) Small metabolite analysis of OdlF. HPLC/MS data of compounds 42 to 44 produced in *E. coli* DH10B::*mtaA* expressing *odIF* in LB medium. All cultures were induced with 0.02% *L*-arabinose. pACYC and pCOLA served as empty vector controls. *odIF* was cloned to a pCOLA plasmid. Base Peak Chromatogram (BPC, top) and Extracted Ion Chromatograms (EIC, below) of 42 (*m*/*z* [M+H]⁺ = 175.11), 43 *(m*/*z* [M+H]⁺ = 191.11) and 44 *(m*/*z* [M+H]⁺ = 116.08). Abundancy of *L*-arginine was determined by 20 µM standard. (B) Comparison of fragmentation HPLC/MS data of compound 42 detected in *E. coli* DH10B::*mtaA* cultures expressing *odIF,* empty vector controls or an commercial *L*-arginine standard. (C) Comparison of fragmentation HPLC/MS data of compound 43 and 44 detected in *E. coli* DH10B::*mtaA* expressing *odIF.*

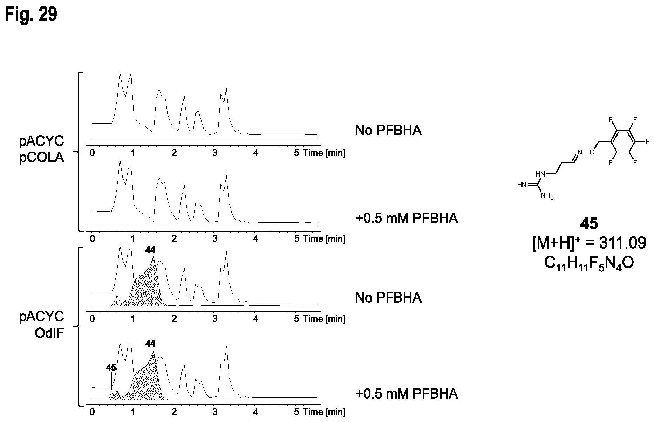

- **Figure 29**: presents small metabolite analysis of OdIF. HPLC/MS data of compounds **42** to **44** produced in *E*. *coli* DH10B::*mtaA* expressing *odIF* in LB medium. All cultures were induced with 0.02% *L*-arabinose. pACYC and pCOLA served as empty vector controls. *odIF* was cloned to a pCOLA plasmid. Base Peak Chromatogram (BPC, top) and Extracted Ion Chromatograms (EIC, below) of **44** (*m*/*z* [M+H]⁺ = 116.08) and **45** *(m*/*z* [M+H]⁺ = 311.09). If indicated, the culture was treated after 72 hours of production for 4 hours with 0.5 mM PFBHA at 30 °C.

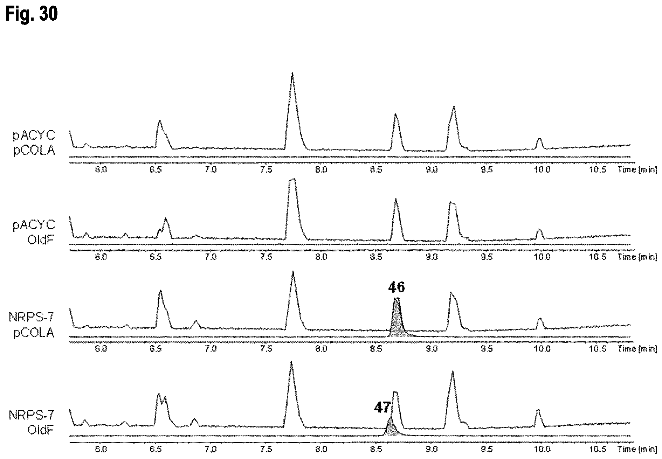

- **Figure 30**: presents HPLC/MS data referred to Figure 6C (NRPS-7) of compounds **46** and **47** produced in *E. coli* DH10B::*mtaA*. *NRPS-7* was cloned on a pACYC plasmid while *odIF* was cloned to a pCOLA plasmid. All cultures were induced with 0.02% *L*-arabinose. pACYC and pCOLA served as an empty vector control. Base Peak Chromatogram (BPC, top) and Extracted Ion Chromatograms (EIC, below) of **46** *(m*/*z* [M+H]⁺ = 584.41) and **47** *(m*/*z* [M+H]⁺ = 600.40).

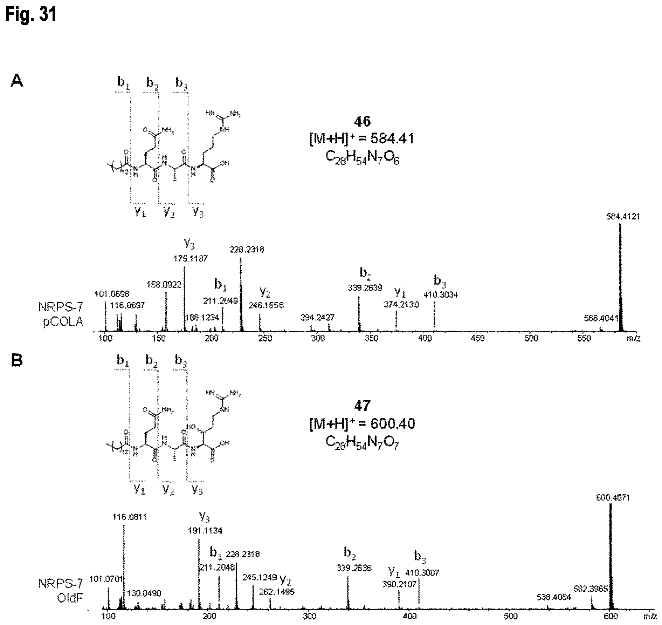

- **Figure 31**: shows: Fragmentation HPLC/MS data referred to Figure 6C and Figure 30 of compound **46** (A) and **47** (B) produced in *E. coli* DH10B::*mtaA* expressing *NRPS-7* with *odIF.*

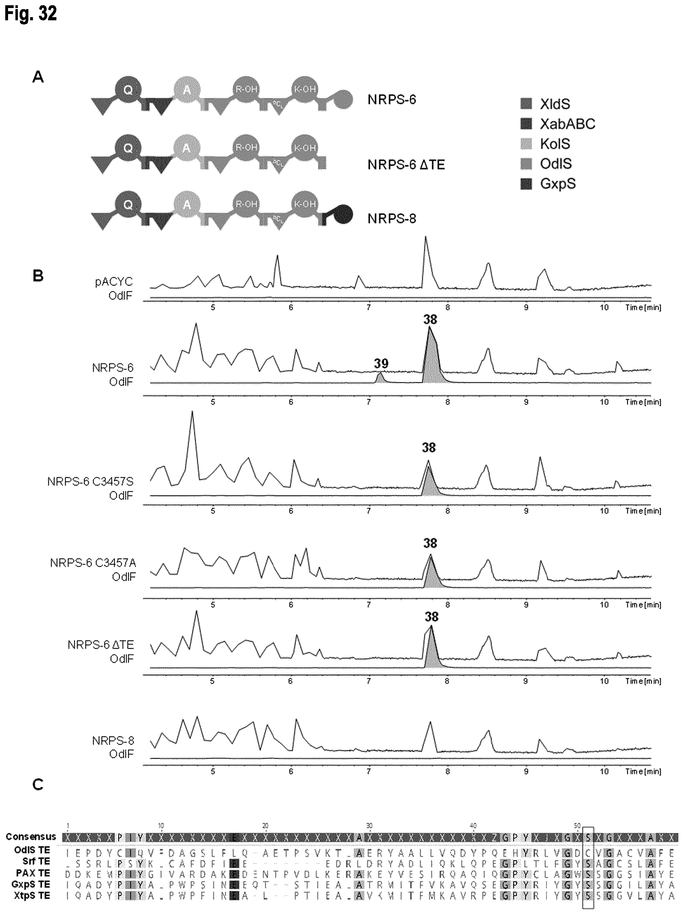

- **Figure 32**: presents: (A) Schematic representation of NRPS-6, NRPS-6 ΔTE and NRPS-8. (B) HPLC/MS data referred to Figure 6D of compounds 38 and 39 produced in *E. coli* DH10B::*mtaA*. *NRPS* were cloned on a pACYC plasmid while *odIF* was cloned to a pCOLA plasmid. All cultures were induced with 0.02% *L-*arabinose. pACYC served as an empty vector control. Base Peak Chromatogram (BPC, top) and Extracted Ion Chromatograms (EIC, below) of 38 *(m*/*z* [M+2H]²⁺ = 355.74) and 39 *(m*/*z* [M+2H]²⁺ = 390.79). (C) Sequence alignment with ClustalOmega algorithm of thioesterase (TE) domains derived from OdlS, Surfactin (Srf), PAX, GxpS and XtpS. A red box highlights the TE conserved serine residue, which was target to mutagenesis studies.

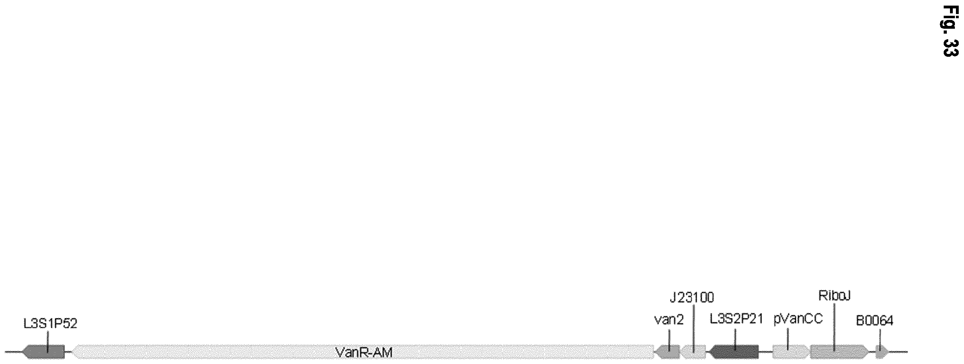

- **Figure 33**: shows schematic overview of Vanp depicted in Table 4.

As explained above, in a first embodiment the present invention relates to a method of preparing a non-standard amino acid or a peptide/protein comprising a non-standard amino acid residue, wherein the non-standard amino acid is 2,4-diamino-3-hydroxybutyrate, the method comprising the step of contacting 2,4-diaminobutyrate with an enzyme having 2,4-diaminobutyrate 3-hydroxylase activity.

The step of transforming 2,4-diaminobutyrate (or 2,4-diaminobutyric acid) to 2,4-diamino-3-hydroxybutyrate (or 2,4-diamino-3-hydroxybutyric acid) is shown in the following scheme:

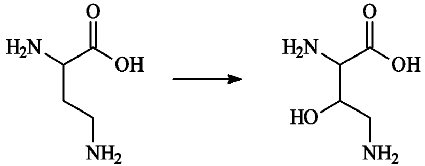

Preferred configuration of the substrate is that corresponding to that of natural-L amino acids. Accordingly, the substrate 2,4-diaminobutyrate exhibits an (S) configuration of its chiral centre. As it is further prefererred, the enzyme having 2,4-diaminobutyrate 3-hydroxylase activity preferably introduces hydroxy group at the position 3 in such a way that the configuration of newly created stereogenic centre is (S). Accordingly, both chiral centers in the product molecule preferably have (S) configuration, the so obtained product is (2S,3S)-2,4-diamino-3-hydroxybutyrate, as shown in the following scheme:

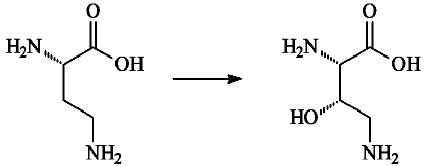

Preferably, the enzyme having 2,4-diaminobutyrate 3-hydroxylase activity is a polypeptide comprising an amino acid sequence with at least 80% sequence identity to SEQ ID NO: 1. More preferably, the enzyme having 2,4-diaminobutyrate 3-hydroxylase activity is a polypeptide comprising an amino acid sequence with at least 85% sequence identity to SEQ ID NO: 1. Even more preferably, the enzyme having 2,4-diaminobutyrate 3-hydroxylase activity is a polypeptide comprising an amino acid sequence with at least 90% sequence identity to SEQ ID NO: 1. Even more preferably, the enzyme having 2,4-diaminobutyrate 3-hydroxylase activity is a polypeptide comprising an amino acid sequence with at least 95% sequence identity to SEQ ID NO: 1. Even more preferably, the enzyme having 2,4-diaminobutyrate 3-hydroxylase activity is a polypeptide comprising an amino acid sequence with at least 99% sequence identity to SEQ ID NO: 1. Most preferably, the enzyme having 2,4-diaminobutyrate 3-hydroxylase activity is a polypeptide comprising an amino acid sequence according to SEQ ID NO: 1. Particularly preferred enzyme as disclosed herein is an enzyme encoded by *odIB* gene from *Xenorhabdus nematophila,* as determined by the present inventors.

It is to be understood that said enzyme catalyzes the step of transforming 2,4-diaminobutyrate (or 2,4-diaminobutyric acid) to 2,4-diamino-3-hydroxybutyrate (or 2,4-diamino-3-hydroxybutyric acid) for a free amino acid or its salt. Preferably, the reaction is not to be performed on an 2,4-diaminobutyrate once incorporated into a peptide, in other words preferably the reaction is not to be performed on an 2,4-diaminobutyrate (or its salt) wherein at least one of its COOH group or its 2-amino group is engaged in forming a peptide bond with another amino acid residue.

The step of contacting 2,4-diaminobutyrate with an enzyme having 2,4-diaminobutyrate 3-hydroxylase activity is standard to the skilled person and can involve preparing an aqueous composition comprising 2,4-diaminobutyrate or its salt, as described herein, and an enzyme enzyme having 2,4-diaminobutyrate 3-hydroxylase activity, as described herein, and inclubating said composition for a certain period of time at a certain temperature. As recognizable to the skilled person, the composition may include further components like salts, reducing agent, enzyme cofactors. Particularly preferred comditions for performing the reaction are given in the Examples section.

The step of contacting 2,4-diaminobutyrate with an enzyme having 2,4-diaminobutyrate 3-hydroxylase activity may alternatively involve contacting 2,4-diaminobutyrate with a microorganism comprising or secreting an enzyme having 2,4-diaminobutyrate 3-hydroxylase activity. Such process would be referred to as enzymatic conversion (biotransformation) of 2,4-diaminobutyrate comprising its 3-hydroxylation. The skilled person is capable of preparing or selecting such a microorganism, for example by using a microorganism that comprises an enzyme having 2,4-diaminobutyrate 3-hydroxylase activity for example by indogenously expressing said enzyme, which may optionally involve secreting said enzyme, or by modifying a microorganism to comprise an enzyme having 2,4-diaminobutyrate 3-hydroxylase activity. This can be achieved by standard methods known to the skilled person, for example by introducing to the microorganism a polynucleotide configured to express an enzyme having 2,4-diaminobutyrate 3-hydroxylase activity (for example a plasmid suitable for a particular microorganism) by the means of transformation under selection pressure, or by genetically modifying said organism using the methods commonly known in the field, for example methods based on CRISPR/Cas. It is to be understood that the product obtained in the course of such transformation, the non-standard amino acid, may need to be purified before it is used in the next steps, for example in the incorporation in the peptide/protein comprising said non-standard amino acid. The methods of purification are known to the skilled person and include chromatography methods (e.g. ion exchange, size exclusion, thin layer or affinity chromatography), precipitation methods, crystallization methods, extraction methods, distillation methods and other methods known to the skilled person.

If in said method of preparing a non-standard amino acid or a peptide/protein comprising a non-standard amino acid residue, wherein the non-standard amino acid is 2,4-diamino-3-hydroxybutyrate, said peptide/protein comprising a non-standard amino acid residue is prepared, the method includes an additional step of incorporating the non-standard amino acid residue into the peptide protein. This can be achieved using the methods known to the skilled person, including the methods of chemical synthesis or enzymatic peptide/protein synthesis methods. If the methods of chemical synthesis are to be used, the non-standard amino acid can be protected by using suitable protecting groups and coupled with appropriately activated/protected amino acid, peptide or protein. Alternatively, and preferably, the means of solid-state peptide synthesis can be used, for example involving Fmoc protection groups strategy. The peptide/protein comprising non-standard amino acid residue can alternatively be prepared by the means of enzymatic synthesis, for example by using a suitable non-ribosomal peptide synthetase or another enzyme capable of creating a peptide bond.

In a second embodiment, the present invention relates to a method of preparing a non-standard amino acid or a peptide/protein comprising a non-standard amino acid residue, wherein the non-standard amino acid is L-5-hydroxylysine, the method comprising the step of contacting L-lysine or a peptide/protein comprising an L-lysine residue with an enzyme having L-lysine 5-hydroxylase activity.

Preferably, in this second embodiment, the present invention relates to a method of preparing a peptide/protein comprising a non-standard amino acid residue, wherein the non-standard amino acid residue is L-5-hydroxylysine, the method comprising the step of contacting a peptide/protein comprising an L-lysine residue with an enzyme having L-lysine 5-hydroxylase activity. Accordingly, in the method of the present invention it is preferred that L-5-hydroxylysine residue as a substrate of an enzyme having L-lysine 5-hydroxylase activity is incorporated into a peptide.

L-5-hydroxylysine is a compound wherein the configuration of the stereocenter of the mainchain carbon is the same as in L-lysine. It may also be referred to as 5-hydroxy-L-lysine. The compound is known to the skilled person to exist in two diastereoisomeric forms, which may also be referred to as (2S, 5S)-5-hydroxylysine and (2S, 5R)-5-hydroxylysine.

The step of transforming an L-lysine residue in a peptide protein to L-5-hydroxylysine residue incorporated in a peptide is shown in the following scheme:

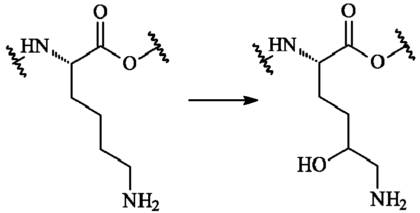

It is to be understood that, preferably, L-lysine is transformed into 5-hydroxylysine, wherein the configuration of the stereogenic centre at position 5 is preferably (S). Accordingly, it is preferred that the step of transforming L-Lysine or an L-lysine residue in a peptide protein to L-5-hydroxylysine or L-5-hydroxylysine residue in a peptide is as follows:

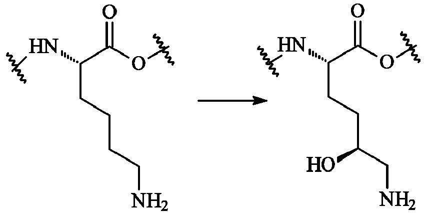

However, also encompassed is an embodiment wherein the method of the second embodiment is a method of preparing a non-standard amino acid, wherein the non-standard amino acid is L-5-hydroxylysine, the method comprising the step of contacting L-lysine with an enzyme having L-lysine 5-hydroxylase activity.

Accordingly, such step of transforming L-lysine to L-5-hydroxylysine is shown in the scheme below:

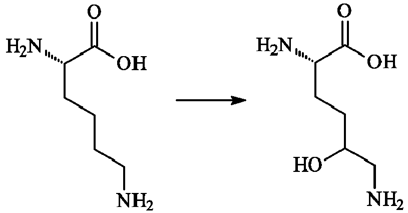

It is to be understood that, preferably, L-lysine is transformed into L-5-hydroxy-lysine, wherein the configuration of the stereogenic centre at position 5 is preferably (S). Accordingly, it is preferred that the step of transforming L-lysine to L-5-hydroxylysine is as shown in the scheme below:

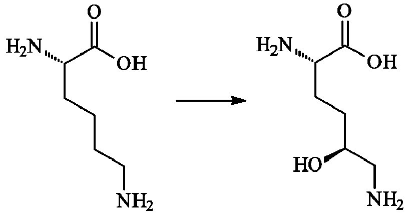

Preferably, the enzyme having L-lysine 5-hydroxylase activity is a polypeptide comprising an amino acid sequence with at least 80% sequence identity to SEQ ID NO: 2. More preferably, the enzyme having L-lysine 5-hydroxylase activity is a polypeptide comprising an amino acid sequence with at least 85% sequence identity to SEQ ID NO: 2. Even more preferably, the enzyme having L-lysine 5-hydroxylase activity is a polypeptide comprising an amino acid sequence with at least 95% sequence identity to SEQ ID NO: 2. Even more preferably, the enzyme having L-lysine 5-hydroxylase activity is a polypeptide comprising an amino acid sequence with at least 99% sequence identity to SEQ ID NO: 2. Even more preferably, the enzyme having L-lysine 5-hydroxylase activity is a polypeptide comprising an amino acid sequence according to SEQ ID NO: 2. Particularly preferred enzyme is a polypeptide encoded by odlE gene from *Xenorhabdus nematophila.*

The step of contacting the peptide/protein comprising L-lysine residue with an enzyme having L-lysine-5-hydroxylase activity is standard to the skilled person and can involve preparing an aqueous composition comprising the peptide/protein comprising L-lysine residue or its salt, as described herein, and an enzyme having L-lysine-5-hydroxylase activity, as described herein, and inclubating said composition for certain time at a certain temperature. As recognizable to the skilled person, the composition may include further components like salts, reducing agent, enzyme cofactors. In the case of L-lysine-5-hydroxylase enzyme, typical cofactors are iron ions and ascorbic acid. Particularly preferred comditions for performing the reaction can be derived from the specific examples as given in the Examples section.

The step of contacting the peptide/protein comprising L-lysine residue with an enzyme having L-lysine-5-hydroxylase activity may alternatively involve contacting the peptide/protein comprising L-lysine residue with a microorganism comprising or secreting an enzyme having L-lysine-5-hydroxylase activity. Such process would be referred to as enzymatic conversion (or biotransformation) of L-lysine residue to L-5-hydroxylysine. The skilled person is capable of preparing or selecting such a microorganism, for example by using a microorganism that comprises an enzyme having L-lysine-5-hydroxylase activity for example by indogenously expressing said enzyme, which may involve optionally secreting said enzyme, or by modifying a microorganism to comprise an enzyme having L-lysine-5-hydroxylase activity. This can be achieved by standard methods known to the skilled person, for example by introducing to the microorganism a polynucleotide configured to express an enzyme having L-lysine-5-hydroxylase activity (for example a plasmid suitable for a particular microorganism, such plasmid can be also referred to as an expression vector) by the means of transformation under selection pressure, or by genetically modifying said organism using the methods commonly known in the field, for example methods based on CRISPR/Cas. It is to be understood that the product obtained in the course of such transformation, the non-standard amino acid, may need to be purified before it is used in the next steps, for example in the incorporation in the peptide/protein comprising said non-standard amino acid. The methods of purification are known to the skilled person and include chromatography methods (e.g. ion exchange, size exclusion or affinity chromatography), precipitation methods, crystallization methods, extraction methods, distillation methods and other methods known to the skilled person.

As mentioned before, it is preferred that in the method of this second embodiment, the peptide/protein comprising an L-lysine residue is used to produce a peptide/protein comprising an L-5-hydroxylysine residue. However, in an embodiment of the invention L-lysine is used as a substrate to produce an L-5-hydroxylysine or a peptide/protein comprising an L-5-hydroxylisine residue. If said method of preparing a non-standard amino acid or a peptide/protein comprising a non-standard amino acid residue, wherein the non-standard amino acid is L-5-hydroxylysine, said peptide/protein comprising a non-standard amino acid residue is prepared, the method includes an additional step of incorporating the non-standard amino acid residue into the peptide protein. This can be achieved using the methods known to the skilled person, including the methods of chemical synthesis or enzymatic peptide/protein synthesis methods. If the methods of chemical synthesis are to be used, the non-standard amino acid can be protected by using suitable protecting groups and coupled with appropriately activated/protected amino acid, peptide or protein. Alternatively, and preferably, the means of solid-state peptide synthesis can be used, for example involving Fmoc protection groups strategy. The peptide/protein comprising non-standard amino acid residue can alternatively be prepared by the means of enzymatic synthesis, for example by using a suitable non-ribosomal peptide synthetase or another enzyme capable of creating a peptide bond.

In a third embodiment, the present invention relates to a method of preparing L-3-hydroxyarginine or a peptide/protein comprising a non-standard amino acid residue, wherein the non-standard amino acid is L-3-hydroxyarginine, the method comprising the step of contacting L-arginine with an enzyme having L-arginine 3-hydroxylase activity.

Accordingly, the present invention is based, at least in part, on a surprising discovery of the present inventors that the enzyme encoded by the gene odIF, which is capable of transforming L-arginine into L-3-hydroxyarginine.

The step of transforming L-arginine into L-3-hydroxyarginine is shown in the following scheme:

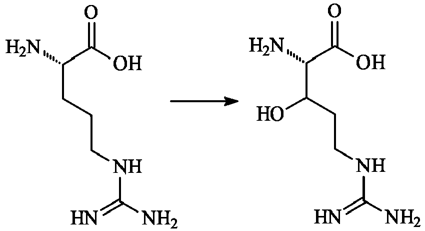

Accordingly and preferably, the enzyme having L-arginine 3-hydroxylase activity acts on free L-arginine.

Preferably, the enzyme having L-arginine 3-hydroxylase activity is a polypeptide comprising an amino acid sequence with at least 80% sequence identity to SEQ ID NO: 3. More preferably, the enzyme having L-arginine 3-hydroxylase activity is a polypeptide comprising an amino acid sequence with at least 85% sequence identity to SEQ ID NO: 3. Even more preferably, the enzyme having L-arginine 3-hydroxylase activity is a polypeptide comprising an amino acid sequence with at least 90% sequence identity to SEQ ID NO: 3. Even more preferably, the enzyme having L-arginine 3-hydroxylase activity is a polypeptide comprising an amino acid sequence with at least 95% sequence identity to SEQ ID NO: 3. Even more preferably, the enzyme having L-arginine 3-hydroxylase activity is a polypeptide comprising an amino acid sequence with at least 99% sequence identity to SEQ ID NO: 3. Particularly preferred enzyme as disclosed herein is an enzyme encoded by odIF gene from *Xenorhabdus nematophila,* as determined by the present inventors.

The step of contacting L-arginine with an enzyme having L-arginine-3-hydroxylase activity is standard to the skilled person and can involve preparing an aqueous composition comprising L-arginine or its salt, as described herein, and an enzyme having L-arginine-3-hydroxylase activity, as described herein, and inclubating said composition for certain time at a certain temperature. As recognizable to the skilled person, the composition may include further components like salts, reducing agent, enzyme cofactors. In the case of L-arginine-3-hydroxylase enzyme, typical cofactors are iron ions. Particularly preferred comditions for performing the reaction can be derived from the specific examples as given in the Examples section.

The step of contacting L-arginine with an enzyme having L-arginine-3-hydroxylase activity may alternatively involve contacting L-arginine with a microorganism comprising or secreting an enzyme having L-arginine-3-hydroxylase activity. Such process would be referred to as enzymatic conversion (or biotransformation) of L-arginine comprising its 3-hydroxylation. The skilled person is capable of preparing or selecting such a microorganism, for example by using a microorganism that comprises an enzyme having L-arginine-3-hydroxylase activity for example by indogenously expressing said enzyme, which may involve optionally secreting said enzyme, or by modifying a microorganism to comprise an enzyme having L-arginine-3-hydroxylase activity. This can be achieved by standard methods known to the skilled person, for example by introducing to the microorganism a polynucleotide configured to express an enzyme having L-arginine-3-hydroxylase activity (for example a plasmid suitable for a particular microorganism, such plasmid can be also referred to as an expression vector) by the means of transformation under selection pressure, or by genetically modifying said organism using the methods commonly known in the field, for example methods based on CRISPR/Cas. It is to be understood that the product obtained in the course of such transformation, the non-standard amino acid, may need to be purified before it is used in the next steps, for example in the incorporation in the peptide/protein comprising said non-standard amino acid. The methods of purification are known to the skilled person and include chromatography methods (e.g. ion exchange, size exclusion or affinity chromatography), precipitation methods, crystallization methods, extraction methods, distillation methods and other methods known to the skilled person.

If in said method of preparing a non-standard amino acid or a peptide/protein comprising a non-standard amino acid residue, wherein the non-standard amino acid is L-3-hydroxyarginine, said peptide/protein comprising a non-standard amino acid residue is prepared, the method includes an additional step of incorporating the non-standard amino acid residue into the peptide protein. This can be achieved using the methods known to the skilled person, including the methods of chemical synthesis or enzymatic peptide/protein synthesis methods. If the methods of chemical synthesis are to be used, the non-standard amino acid can be protected by using suitable protecting groups and coupled with appropriately activated/protected amino acid, peptide or protein. Alternatively, and preferably, the means of solid-state peptide synthesis can be used, for example involving Fmoc protection groups strategy. The peptide/protein comprising non-standard amino acid residue can alternatively be prepared by the means of enzymatic synthesis, for example by using a suitable non-ribosomal peptide synthetase or another enzyme capable of creating a peptide bond. Herein, for peptide/protein comprising L-3-hydroxy-arginine non standard amino acid residue, the enzymatic way of incorporation involving a particular non-ribosomal peptide synthetase is preferred.

In a fourth embodiment, the present invention relates to a method of preparing a peptide/protein comprising a non-standard amino acid residue, wherein the non-standard amino acid is 2,3-didehydroarginine, the method comprising the step of contacting a peptide/protein comprising an L-3-hydroxyarginine residue with an enzyme having L-3-hydroxyarginine dehydratase activity, wherein a peptide/protein comprising a 2,3-didehydroarginine residue is formed.

Accordingly, the transformation of L-3-hydroxyarginine residue to 2,3-didehydroarginine residue (which may also be referred to as dehydroarginine residue for brevity), wherein a peptide/protein comprising a 2,3-didehydroarginine residue is formed is as follows in the scheme below:

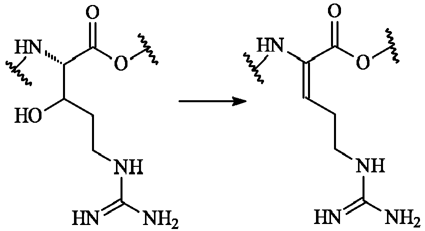

Accordingly and preferably, the enzyme having L-3-hydroxyarginine dehydratase activity acts on L-3-hydroxyarginine when incorporated into a peptide or protein.

As it is to be understood herein, the enzyme having L-3-hydroxyarginine dehydratase activity is a condensation domain comprised within a non-ribosomal peptide synthetase. It is to be understood that upon transformation of the free L-arginine into L-3-hydroxyarginine, said 3-hydroxyarginine is activated by its adenylation by the adenylation domain and transferred to the thioylation/carrier domain. Subsequently, the condensation domain catalyzes the amide bond formation and the attachment of the L-3-hydroarginine residue to the nascent chain of the peptide synthetized by the non-ribosomal peptide synthetase. This mechanism is consistent with the general principle of modular peptide synthesis by non-ribosomal peptide synthetases. The present invention is, at least in part, based on the surprising discovery that the condensation domain responsible for attachment of L-3-hydroxyarginine into the nascent peptide chain in the *Xenorhabdus nematophila* NRPS responsible for biosynthesis of oligorhabdin catalyzes the transformation of L-3-hydroxyarginine to dehydroarginine.

Preferably said condensation domain is a polypeptide comprising an amino acid sequence with at least 80% sequence identity to SEQ ID NO.: 5. More preferably, said condensation domain is a polypeptide comprising an amino acid sequence with at least 85% sequence identity to SEQ ID NO.: 5. Even more preferably, said condensation domain is a polypeptide comprising an amino acid sequence with at least 90% sequence identity to SEQ ID NO.: 5. Even more preferably, said condensation domain is a polypeptide comprising an amino acid sequence with at least 95% sequence identity to SEQ ID NO.: 5. Even more preferably, said condensation domain is a polypeptide comprising an amino acid sequence with at least 99% sequence identity to SEQ ID NO.: 5. Even more preferably, said condensation domain is a polypeptide comprising an amino acid sequence according to SEQ ID NO.: 5.

In a fifth embodiment, the present invention provides a method for preparing a peptide/protein comprising one or more non-standard amino acids selected from 2,4-diamino-3-hydroxybutyrate, L-5-hydroxylysine, L-3-hydroxyarginine and 2,3-didehydroarginine. It is to be understood that the method comprises one or more steps as defined in any one of first, second, third and/or fourth embodiments of the present invention, as defined hereinabove.

Accordingly, and as explained previously, the method for preparing a peptide/protein comprising one or more non-standard amino acids selected from 2,4-diamino-3-hydroxybutyrate, L-5-hydroxylysine, L-3-hydroxyarginine and 2,3-didehydroarginine comprises one or more steps selected from:
contacting 2,4-diaminobutyrate with an enzyme having 2,4-diaminobutyrate 3-hydroxylase activity, followed by incorporating the so obtained 2,4-diamino-3-hydroxybutyrate into said peptide/protein;
contacting L-lysine with an enzyme having L-lysine 5-hydroxylase activity, followed by incorporating the so obtained L-5-hydroxylysine into said peptide/protein;
contacting a peptide/protein comprising an L-lysine residue with an enzyme having L-lysine 5-hydroxylase activity;
contacting L-arginine with an enzyme having L-arginine 3-hydroxylase activity followed by incorporating the so obtained L-5-hydroxylysine into said peptide/protein, and
contacting a peptide/protein comprising an L-3-hydroxyarginine residue with an enzyme having L-3-hydroxyarginine dehydratase activity, wherein a peptide/protein comprising a 2,3-didehydroarginine residue is formed.

The skilled person is capable of planning the synthesis of a peptide/protein comprising one or more non-standard amino acids selected from 2,4-diamino-3-hydroxybutyrate, L-5-hydroxylysine, L-3-hydroxyarginine and 2,3-didehydroarginine, by selecting suitable steps and their order, depending on the structure of the planned peptide/protein.

Some of the enzymes used in the methods of the present invention allow modification of a free amino acid, followed by its incorporation to the peptide/protein. This is the case for the transformation of 2,4-diamino-butyrate into 2,4-diamino-3-hydroxybutyrate, transformation of L-lysine into L-5-hydroxylysine, and transformation of L-arginine into L-3-hydroxyarginine. In such a case, the appropriate enzymes can be used to produce the required amino acid at a required scale, which then will be used in the synthesis, for example using solid-state peptide synthesis approaches, e.g. Fmoc chemistry, as explained hereinabove, or can be fed as a substrate to a microbial process, for example to a culture of a suitable producer strain configured to express appropriate non-ribosomal peptide synthetase. It is noted that the microbial process can also be combined with chemical synthesis. It is recognizable to the skilled person that a peptide obtain in a microbiological process can be further modified by the means of chemical synthesis.

Some of the enzymes used in the methods of the present invention allow modification of an amino acid residue present/incorporated into a peptide/protein. This is the case for the transformation of an L-lysine residue in a protein/peptide into an L-5-hydroxylysine and for the transformation of an L-3-hydroxyarginine residue in a protein/prptide a 2,3-didehydroarginine residue. These enzymatic biotransformation steps can be combined with the means of standard (e.g. solid state) chemical synthesis.

Preferably, the peptide/protein produced in the method for preparing a peptide/protein comprising one or more non-standard amino acids selected from 2,4-diamino-3-hydroxybutyrate, L-5-hydroxylysine, L-3-hydroxyarginine and 2,3-didehydroarginine of the present invention is an odilorhabdin. Preferably, said odilorhabdin is selected from NOSO-95179, NOSO-95A, NOSO-95B, NOSO-95C, and NOSO-502.

Preferably, NOSO-95179 is a compound according to formula: 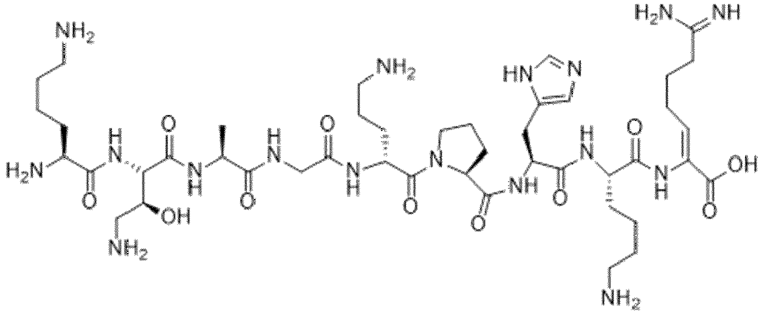 or a pharmaceutically acceptable salt thereof.

As it is to be understood herein, preferably NOSO-95A, NOSO-95B and NOSO95C are each a compound according to formula: 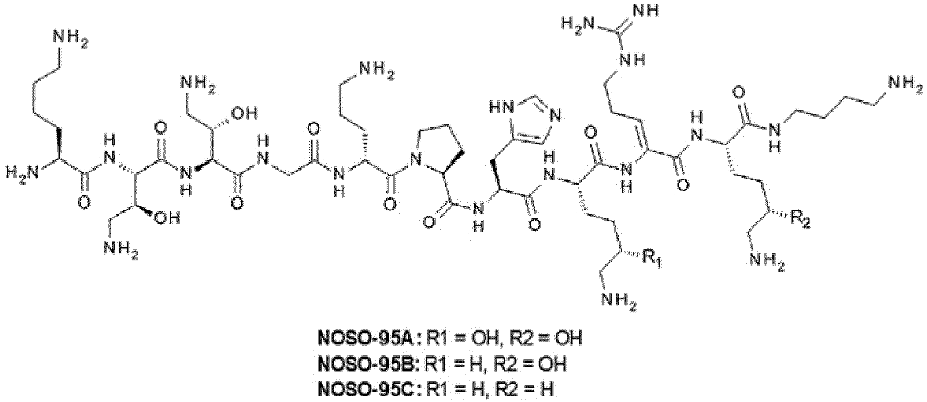 respectively, or a pharmaceutically acceptable salt thereof.

As it is to be understood herein, NOSO-502 is a compound according to formula: 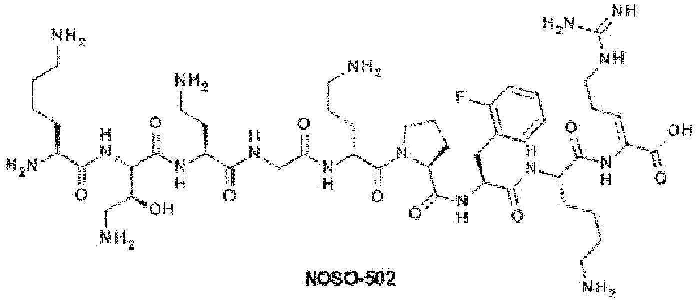 or a pharmaceutically acceptable salt thereof. It is to be noted that in addition to non-standard amino acids selected from 2,4-diamino-3-hydroxybutyrate, L-5-hydroxylysine, L-3-hydroxyarginine and 2,3-didehydroarginine, NOSO-502 includes 2-fluorophenylalanine, which preferably is to be introduced into the peptide by the means of solid-state chemical synthesis. These operations are known to the skilled person.

The method for preparing a peptide/protein comprising one or more non-standard amino acids selected from 2,4-diamino-3-hydroxybutyrate, L-5-hydroxylysine, L-3-hydroxyarginine and 2,3-didehydroarginine can be performed using a cell-based or a cell-free system comprising a biosynthetic gene cluster.

The applications relying on a cell-based system, which was also referred to as an appropriate producer strain, have been discussed hereinabove. While modification of intracellular metabolic pathways by metabolic engineering has generated many engineered strains with relatively high yields of various target products in the past few decades, the unpredictable accumulation of toxic products, the cell membrane barrier, and competition between the carbon flux of cell growth and product synthesis have severely retarded progress toward the industrial-scale application of cell-based systems in many cases. An alternative to cell-based systems is the use of cell-free systems, wherein the issues related to competition for carbon flux with other metabolic pathways vital to cell survival and culture growth and issues related to the presence of the cell membrane may be overcome. One example of commonly applied cell-free system is cell-free protein production in solution using biomolecular translation machinery extracted from cells. Because protein synthesis occurs in cell lysates rather than within cultured cells, the method is also called cell-free protein expression. In the method of the present invention, cell lysates comprising suitable non-ribosomal peptide synthetase and other genes required for post- or co-synthesis modification of peptide/protein/amino acid, are prepared from suitable microbial strains and used in the process. Optionally, said cell lysate can be further processed, e.g. subjected to a purification or enrichment step, and/or supplemented with additional co-factors, substrates and/or enzymes.

In the method of the present invention, preferably the biosynthetic gene cluster comprises a gene encoding a polypeptide having 2,4-diaminobutyrate 3-hydroxylase activity, a gene encoding a polypeptide having L-lysine 5-hydroxylase activity, a gene encoding a polypeptide having L-arginine 3-hydroxylase activity, and/or a gene encoding a polypeptide having L-3-hydroxyarginine dehydratase activity. Preferably, for the synthesis of odilorhabdin, the biosynthetic gene cluster comprises a gene encoding a polypeptide having 2,4-diaminobutyrate 3-hydroxylase activity, a gene encoding a polypeptide having L-lysine 5-hydroxylase activity, a gene encoding a polypeptide having L-arginine 3-hydroxylase activity, and a gene encoding a polypeptide having L-3-hydroxyarginine dehydratase activity. It is preferred that the genes in the biosynthetic gene cluster are operably linked. As it is apparent to the skilled person, the nucleic acid molecules can further comprise expression control sequences operably linked to the polynucleotide comprised in the nucleic acid molecule. The term "operatively linked" or "operably linked", as used throughout the present description, refers to a linkage between one or more expression control sequences and the coding region in the polynucleotide to be expressed in such a way that expression is achieved under conditions compatible with the expression control sequence.

Preferably, the biosynthetic gene cluster further comprises a gene encoding a polypeptide with deacylase activity which is capable of enzymatic removal of fatty acyl from the N-terminus of the peptide/protein.The presence of a fatty acyl at the N-terminus of the peptide/protein, in the case of odilorhabdins, is due to the actions of the self-resistance locus in the cells, and is species-specific. Accordingly, the presence of N-terminal fatty acyl group prevents odilorhabdin from becoming toxic to his host. As known to the skilled person, the fatty acid group can be removed by an action of an enzyme having deacylase activity. In the case of odilorhabdins, deacylation is performed by an enzyme encoded by the gene odIG, which acts in the periplasm of the host cell. Accordingly, the protein encoded by the odIG gene is expressed in perisplasm.

Preferably, the polypeptide with deacylase activity is a polypeptide comprising an amino acid sequence with at least 80% sequence identity to SEQ ID NO: 4. More preferably, the polypeptide with deacylase activity is a polypeptide comprising an amino acid sequence with at least 85% sequence identity to SEQ ID NO: 4. Even more preferably, the polypeptide with deacylase activity is a polypeptide comprising an amino acid sequence with at least 90% sequence identity to SEQ ID NO: 4. Even more preferably, the polypeptide with deacylase activity is a polypeptide comprising an amino acid sequence with at least 95% sequence identity to SEQ ID NO: 4. Even more preferably, the polypeptide with deacylase activity is a polypeptide comprising an amino acid sequence with at least 99% sequence identity to SEQ ID NO: 4. Even more preferably, the polypeptide with deacylase activity is a polypeptide comprising an amino acid sequence according to to SEQ ID NO: 4. Particularly preferred polypeptide with deacylase activity is a polypeptide expression product of odIG gene from *Xhenorhabdus nematophila.* As it will be understood to the skilled person, said gene may also be modified to configure it for cytoplasmic expression of the polypeptide.

Preferably, the polypeptide with deacylase activity as described hereinabove exhibits deacylase activity for substrates wherein a lysine residue at the N-terminus of said substrate carries a fatty acid moiety attached through an amide bond to its N-terminal amino group.

The present invention further relates to specific use of polypeptides as described herein. Said specific uses will be described in the following.

In a sixth embodiment of the present invention, the present invention relates to use of a polypeptide comprising an amino acid sequence with at least 80% sequence identity to SEQ ID NO: 1 for catalyzing a hydroxylation at position 3 of 2,4-diaminobutyrate. Said hydroxylation at position 3 of 2,4 diaminobutyrate is a reaction according to the following scheme:

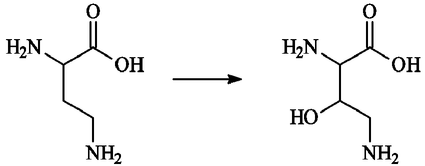

Preferably, the hydroxylation at position 3 of 2,4 diaminobutyrate is a hydroxylation at position 3 of (2S)-2,4-diaminobutyrate, yielding (2S,3S)-2,4-diamino-3-hydroxybutyrate, as shown in the following scheme:

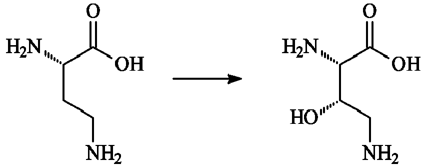

Preferably, the polypeptide comprising an amino acid sequence with at least 80% sequence identity to SEQ ID NO: 1 is a polypeptide comprising an amino acid sequence with at least 85% sequence identity to SEQ ID NO: 1, more preferably a polypeptide comprising an amino acid sequence with at least 90% sequence identity to SEQ ID NO: 1, even more preferably a polypeptide comprising an amino acid sequence with at least 95% sequence identity to SEQ ID NO: 1, even more preferably a polypeptide comprising an amino acid sequence with at least 99% sequence identity to SEQ ID NO: 1, even more preferably a polypeptide comprising an amino acid sequence according to SEQ ID NO: 1.

In a seventh embodiment of the present invention, the present invention relates to use of a polypeptide comprising an amino acid sequence with at least 80% sequence identity to SEQ ID NO: 2 for catalyzing a hydroxylation at position 5 of L-lysine. Said hydroxylation at position 5 of L-lysine, where L-lysine is incorporated into a peptide/protein, is shown in the following scheme:

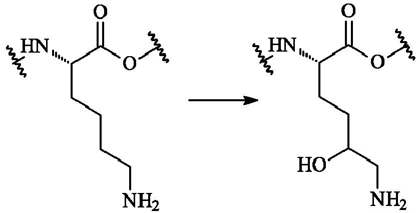

It is preferred that the configuration of a newly formed stereocenter is (S), and accordingly said hydroxylation at position 5 of L-lysine, where L-lysin is incorporated into a peptide/protein, is shown in the following scheme:

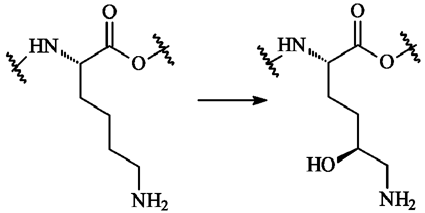

However, also encompassed by the present invention is aspecific embodiment wherein said hydroxylation at position 5 of L-lysine is performed on a free L-lysine, according to the scheme below:

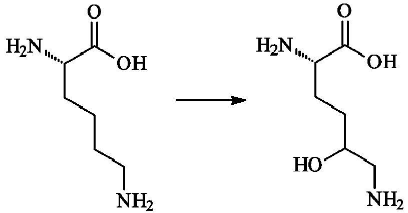

It is to be understood that, preferably, L-lysine is transformed into L-5-hydroxy-lysine, wherein the configuration of the stereogenic centre at position 5 is preferably (S). Accordingly, it is preferred that the step of transforming L-lysine to L-5-hydroxylysine is as shown in the scheme below:

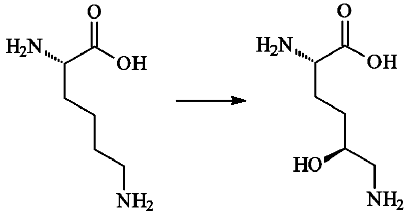

Preferably, the polypeptide comprising an amino acid sequence with at least 80% sequence identity to SEQ ID NO: 2 is a polypeptide comprising an amino acid sequence with at least 85% sequence identity to SEQ ID NO: 2, more preferably a polypeptide comprising an amino acid sequence with at least 90% sequence identity to SEQ ID NO: 2, even more preferably a polypeptide comprising an amino acid sequence with at least 95% sequence identity to SEQ ID NO: 2, even more preferably a polypeptide comprising an amino acid sequence with at least 99% sequence identity to SEQ ID NO: 2, even more preferably a polypeptide comprising an amino acid sequence according to SEQ ID NO: 2.

In an eighth embodiment, the present invention relates to use of a polypeptide comprising an amino acid sequence with at least 80% sequence identity to SEQ ID NO: 3 for catalyzing a hydroxylation at position 3 of L-arginine. The step of the hydroxylation at position 3 of L-arginine is shown in the following scheme:

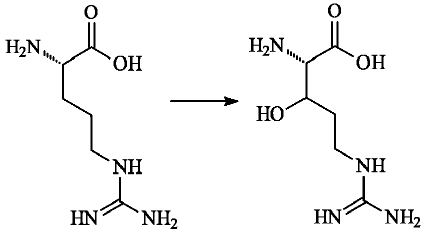

Accordingly and preferably, the enzyme having L-arginine 3-hydroxylase activity acts on free L-arginine.

Preferably, the polypeptide comprising an amino acid sequence with at least 80% sequence identity to SEQ ID NO: 3 is a polypeptide comprising an amino acid sequence with at least 85% sequence identity to SEQ ID NO: 3, more preferably a polypeptide comprising an amino acid sequence with at least 90% sequence identity to SEQ ID NO: 3, even more preferably a polypeptide comprising an amino acid sequence with at least 95% sequence identity to SEQ ID NO: 3, even more preferably a polypeptide comprising an amino acid sequence with at least 99% sequence identity to SEQ ID NO: 3, even more preferably a polypeptide comprising an amino acid sequence according to to SEQ ID NO: 3.

In a ninth embodiment, the present invention relates to use of a polypeptide comprising an amino acid sequence with at least 80% sequence identity to SEQ ID NO: 4 for catalyzing a deacylation, preferably for removing a fatty acyl from the N-terminus of an odilorhabdin.

Preferably, the polypeptide comprising an amino acid sequence with at least 80% sequence identity to SEQ ID NO: 4 is a polypeptide comprising an amino acid sequence with at least 85% sequence identity to SEQ ID NO: 4, more preferably a polypeptide comprising an amino acid sequence with at least 90% sequence identity to SEQ ID NO: 4, even more preferably a polypeptide comprising an amino acid sequence with at least 95% sequence identity to SEQ ID NO: 4, even more preferably a polypeptide comprising an amino acid sequence with at least 99% sequence identity to SEQ ID NO: 4, even more preferably a polypeptide comprising an amino acid sequence according to SEQ ID NO: 4.

In a tenth embodiment, the present invention relates to use of a polypeptide comprising an amino acid sequence with at least 80% sequence identity to SEQ ID NO: 5 for catalyzing a dehydratation reaction of L-3-hydroxyarginine. The dehydratation reaction of L-3-hydroxyarginine is as shown in the scheme below:

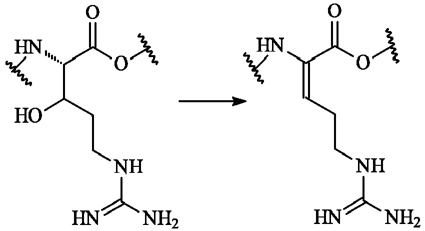

Accordingly and preferably, the enzyme having L-3-hydroxyarginine dehydratase activity acts on L-3-hydroxyarginine when incorporated into a peptide or protein.

Preferably, the polypeptide comprising an amino acid sequence with at least 80% sequence identity to SEQ ID NO: 5 is a polypeptide comprising an amino acid sequence with at least 85% sequence identity to SEQ ID NO: 5, more preferably a polypeptide comprising an amino acid sequence with at least 90% sequence identity to SEQ ID NO: 5, even more preferably a polypeptide comprising an amino acid sequence with at least 95% sequence identity to SEQ ID NO: 5, even more preferably a polypeptide comprising an amino acid sequence with at least 99% sequence identity to SEQ ID NO: 5, even more preferably a polypeptide comprising an amino acid sequence according to SEQ ID NO: 5.

In further embodiments, the present invention also relates to polypeptides with activities identified by the present inventors that are used in the methods of the present invention. These will be provided in the following.

Accordingly, in an eleventh embodiment, the present invention relates to a polypeptide comprising an amino acid sequence with at least 80% sequence identity to SEQ ID NO: 1 and having 2,4-diaminobutyrate-3-hydroxylase activity.

Preferably, the polypeptide comprising an amino acid sequence with at least 80% sequence identity to SEQ ID NO: 1 is a polypeptide comprising an amino acid sequence with at least 85% sequence identity to SEQ ID NO: 1, more preferably a polypeptide comprising an amino acid sequence with at least 90% sequence identity to SEQ ID NO: 1, even more preferably a polypeptide comprising an amino acid sequence with at least 95% sequence identity to SEQ ID NO: 1, even more preferably a polypeptide comprising an amino acid sequence with at least 99% sequence identity to SEQ ID NO: 1, even more preferably a polypeptide comprising an amino acid sequence according to SEQ ID NO: 1.

However, preferably said polypeptide is not a polypeptide consisting of the amino acid sequence according to SEQ ID NO: 1.

In a twelfth embodiment, the present invention relates to a polypeptide comprising an amino acid sequence with at least 80% sequence identity to SEQ ID NO: 2 and having L-lysine 5-hydroxylase activity.

Preferably, the polypeptide comprising an amino acid sequence with at least 80% sequence identity to SEQ ID NO: 2 is a polypeptide comprising an amino acid sequence with at least 85% sequence identity to SEQ ID NO: 2, more preferably a polypeptide comprising an amino acid sequence with at least 90% sequence identity to SEQ ID NO: 2, even more preferably a polypeptide comprising an amino acid sequence with at least 95% sequence identity to SEQ ID NO: 2, even more preferably a polypeptide comprising an amino acid sequence with at least 99% sequence identity to SEQ ID NO: 2, even more preferably a polypeptide comprising an amino acid sequence according to SEQ ID NO: 2

However, preferably said polypeptide is not a polypeptide consisting of the amino acid sequence according to SEQ ID NO: 2.

In thirteenth embodiment, the present invention relates to a polypeptide comprising an amino acid sequence with at least 80% sequence identity to SEQ ID NO: 3 and having L-arginine 3-hydroxylase activity.

Preferably, the polypeptide comprising an amino acid sequence with at least 80% sequence identity to SEQ ID NO: 3 is a polypeptide comprising an amino acid sequence with at least 85% sequence identity to SEQ ID NO: 3, more preferably a polypeptide comprising an amino acid sequence with at least 90% sequence identity to SEQ ID NO: 3, even more preferably a polypeptide comprising an amino acid sequence with at least 95% sequence identity to SEQ ID NO: 3, even more preferably a polypeptide comprising an amino acid sequence with at least 99% sequence identity to SEQ ID NO: 3, even more preferably a polypeptide comprising an amino acid sequence according to to SEQ ID NO: 3.

Preferably, said polypeptide is not a polypeptide consisting of the amino acid sequence according to SEQ ID NO: 3.

In a fourteenth embodiment, the present invention relates to a polypeptide comprising an amino acid sequence with at least 80% sequence identity to SEQ ID NO: 4 and having a deacylase activity.

Preferably, said polypeptide is capable of removing fatty acyl from the N-terminus of an odilorhabdin.

Preferably, the polypeptide comprising an amino acid sequence with at least 80% sequence identity to SEQ ID NO: 4 is a polypeptide comprising an amino acid sequence with at least 85% sequence identity to SEQ ID NO: 4, more preferably a polypeptide comprising an amino acid sequence with at least 90% sequence identity to SEQ ID NO: 4, even more preferably a polypeptide comprising an amino acid sequence with at least 95% sequence identity to SEQ ID NO: 4, even more preferably a polypeptide comprising an amino acid sequence with at least 99% sequence identity to SEQ ID NO: 4, even more preferably a polypeptide comprising an amino acid sequence according to SEQ ID NO: 4.

Preferably, said polypeptide is not a polypeptide consisting of the amino acid sequence according to SEQ ID NO: 4.

In a fifteenth embodiment, the present invention relates to a polypeptide comprising an amino acid sequence with at least 80% sequence identity to SEQ ID NO: 5 and having an L-3-hydroxyarginine dehydratase activity.

Preferably, the polypeptide comprising an amino acid sequence with at least 80% sequence identity to SEQ ID NO: 5 is a polypeptide comprising an amino acid sequence with at least 85% sequence identity to SEQ ID NO: 5, more preferably a polypeptide comprising an amino acid sequence with at least 90% sequence identity to SEQ ID NO: 5, even more preferably a polypeptide comprising an amino acid sequence with at least 95% sequence identity to SEQ ID NO: 5, even more preferably a polypeptide comprising an amino acid sequence with at least 99% sequence identity to SEQ ID NO: 5, even more preferably a polypeptide comprising an amino acid sequence according to SEQ ID NO: 5.

Preferably, said polypeptide is not a polypeptide consisting of the amino acid sequence according to SEQ ID NO: 5.

The enzymes used according to the invention and disclosed herein can be naturally occurring enzymes or enzymes which are derived from naturally occurring enzymes, e.g., by the introduction of mutations or other alterations which, e.g., alter or improve the enzymatic activity, the stability, etc. Methods for modifying and/or improving the desired enzymatic activities of proteins are well-known to the person skilled in the art and include, e.g., random mutagenesis or site-directed mutagenesis and subsequent selection of enzymes/transporters having the desired properties or approaches of the so-called "directed evolution". Enzymes characterized by specific sequences disclosed herein as described in the specific embodiments and examples of the invention are suitable starting points for such modification. For example, said modifications can be introduced using the means of genetic modification. For genetic modification in prokaryotic cells, a nucleic acid molecule encoding a corresponding enzyme can be introduced into plasmids which permit mutagenesis or sequence modification by recombination of DNA sequences. Standard methods (see Sambrook and Russell (2001), Molecular Cloning: A Laboratory Manual, CSH Press, Cold Spring Harbor, NY, USA) allow base exchanges to be performed or natural or synthetic sequences to be added. DNA fragments can be ligated by using adapters and linkers complementary to the fragments. Moreover, engineering measures which provide suitable restriction sites or remove surplus DNA or restriction sites can be used. In those cases, in which insertions, deletions or substitutions are possible, in vitro mutagenesis, "primer repair", restriction or ligation can be used. In general, a sequence analysis, restriction analysis and other methods of biochemistry and molecular biology are carried out as analysis methods. The resulting enzyme variants are then tested for the desired activity, e.g., enzymatic activity, with an assay as described above and in particular for their increased enzyme activity and transporter activity/capacity, respectively.

The following definitions apply through the present description and the appended claims.

The term "peptide" refers to a polymer of two or more amino acid residues linked via amide bonds that are formed between an amino group of one amino acid residue and a carboxylic acid group of another amino acid residue. The amino acid residues comprised in the peptide or protein, which are also referred to as amino acids, may be selected from the 20 standard proteinogenic α amino acids (i.e., Ala, Arg, Asn, Asp, Cys, Glu, Gln, Gly, His, Ile, Leu, Lys, Met, Phe, Pro, Ser, Thr, Trp, Tyr, and Val) but also from non-proteinogenic and/or non-standard α amino acids (such as, e.g., ornithine, citrulline, homolysine, pyrrolysine, 4 hydroxyproline, α methylalanine (i.e., 2 aminoisobutyric acid), norvaline, norleucine, terleucine (tert-leucine), labionin, 2,4-diamino-3-hydroxybutyric acid (2,4-diamino-3-hydroxybutyrate), 2,4-diaminobutyrate, 5-hydroxylysine, 3-hydroxyarginine, 2,3-didehydroarginine or an alanine or glycine that is substituted at the side chain with a cyclic group such as, e.g., cyclopentylalanine, cyclohexylalanine, phenylalanine, naphthylalanine, pyridylalanine, thienylalanine, cyclohexylglycine, or phenylglycine) as well as β amino acids (e.g., β alanine), γ-amino acids (e.g., γ-aminobutyric acid, isoglutamine, or statine) and δ-amino acids. Preferably, the amino acid residues comprised in the peptide or protein are selected from α amino acids, more preferably from the 20 standard proteinogenic α amino acids (which can be present as the L isomer or the D-isomer, and are preferably all present as the L isomer). The peptide may be unmodified or may be modified, e.g., at its N terminus, at its C terminus and/or at a functional group in the side chain of any of its amino acid residues (particularly at the side chain functional group of one or more Lys, His, Ser, Thr, Tyr, Cys, Asp, Glu, and/or Arg residues). Such modifications may include, e.g., the attachment of any of the protecting groups described for the corresponding functional groups in: Wuts PG & Greene TW, Greene's protective groups in organic synthesis, John Wiley & Sons, 2006. Such modifications may also include the covalent attachment of one or more polyethylene glycol (PEG) chains (forming a PEGylated peptide), the glycosylation and/or the acylation with one or more fatty acids (e.g., one or more C8-30 alkanoic or alkenoic acids; forming a fatty acid acylated peptide or protein). Moreover, such modified peptides or proteins may also include peptidomimetics, provided that they contain at least two amino acids that are linked via an amide bond (formed between an amino group of one amino acid and a carboxyl group of another amino acid). The amino acid residues comprised in the peptide or protein may, e.g., be present as a linear molecular chain (forming a linear peptide) or may form one or more rings (corresponding to a cyclic peptide). Unless indicated to the contrary, the peptides as referred to herein are linear peptides of less than 40 residues, comprising amino acid residues selected from the 20 standard proteinogenic α amino acids, each present as L isomer. Preferably, side chains of amino acid residues are not modified. Preferably, N-terminus of the peptide is either not modified or is acetylated, and/or C-terminus of the peptide is either not modified or is amidated. Acetylation is preferably understood herein as a modification of the amino group, preferably a main chain amino group NH₂-, into CH₃CONH- moiety. Amidation is preferably understood herein as a modification of the carboxylic group, preferably a main chain carboxylic group -COOH, into a -CONH₂ moiety.

The term non-standard amino acid refers to a compound comprising an amino group -NH-, preferably -NH₂, or an ionized form thereof, and a carboxylic group -COOH, or an ionized form thereof, excluding the 20 standard proteinogenic α amino acids (i.e., Ala, Arg, Asn, Asp, Cys, Glu, Gin, Gly, His, Ile, Leu, Lys, Met, Phe, Pro, Ser, Thr, Trp, Tyr, and Val). It is preferred in the present invention that the non-standard amino acid residues are selected fmo 2,4-diamino-3-hydroxybutyric acid (2,4-diamino-3-hydroxybutyrate), 2,4-diaminobutyrate, 5-hydroxylysine, 3-hydroxyarginine, and 2,3-didehydroarginine.

Preferably, the terms peptide and protein are used interchangeably. The peptide or the protein may also be referred to as polypeptide. Preferebly, when reference is made to a protein, protein is meant to comprise a linear peptide of at least 41 amino acid residues.

As referred to herein, the term "enzyme" comprises a protein, as defined herein, and is capable of catalyzing a chemical reaction between substrate or a chemical transformation of a substrate, upon being contacted with said substrate(s). A reaction as described herein may be referred to as an enzymatic reaction. In addition to a polypeptide chain, which is characterized through its amino acid sequence, or a sequence of a gene encoding said polypeptide, the enzyme may comprise cofactor(s) and/or metal ions. Cofactor is herein understood as a compound which plays a role in catalyzing the enzymatic reaction. Cofactor may be covalently or non-covaletly bound to the enzyme. Cofactor may be directly participating in the reaction and undergoing transformation, which would necessitate a reverse transformation before the cofactor can be used again in the same reaction, or exchange of the used up, transformed cofactor molecule with another molecule. Alternatively, cofactor may play a role in catalyzing the enzymatic reaction without being transformed in said reaction. The enzyme may further comprise more than one protein, wherein said more than one protein interacts with each other, thus forming a multiprotein enzyme complex. The enzyme may also constitute a part of larger protein, for example a domain of a larger protein bearing a particular enzymatic activity. For example, each domain of non-ribosomal peptide synthetase that is capable of catalyzing an enzymatic reaction, as referred to herein, may be considered an enzyme. Enzymes may also comprise non-protein molecules, for example nucleic acid molecules.

The term "expression vector" as used herein relates to a polynucleotide configured to express a particular protein in a microorganism upon its introduction to said microorganism, e.g. by the means of transformation. Expression vectors have been widely described in the literature. As a rule, they contain not only a selection marker gene and a replication-origin ensuring replication in the host selected, but also a bacterial or viral promoter, and in most cases a termination signal for transcription. Between the promoter and the termination signal there is in general at least one restriction site or a polylinker which enables the insertion of a coding DNA sequence. The DNA sequence naturally controlling the transcription of the corresponding gene can be used as the promoter sequence, if it is active in the selected host organism. However, this sequence can also be exchanged for other promoter sequences. It is possible to use promoters ensuring constitutive expression of the gene and inducible promoters which permit a deliberate control of the expression of the gene. Bacterial and viral promoter sequences possessing these properties are described in detail in the literature. Regulatory sequences for the expression in microorganisms (for instance *E*. *coli, S*. *cerevisiae)* are sufficiently described in the literature. Promoters permitting a particularly high expression of a downstream sequence are for instance the T7 promoter (Studier et al., Methods in Enzymology 185 (1990), 60-89), lacUV5, trp, trp-lacUV5 (DeBoer et al., in Rodriguez and Chamberlin (Eds), Promoters, Structure and Function; Praeger, New York, (1982), 462-481; DeBoer et al., Proc. Natl. Acad. Sci. USA (1983), 21-25), Ip1, rac (Boros et al., Gene 42 (1986), 97-100). Inducible promoters are preferably used for the synthesis of polypeptides. These promoters often lead to higher polypeptide yields than do constitutive promoters. In order to obtain an optimum amount of polypeptide, a two-stage process is often used. First, the host cells are cultured under optimum conditions up to a relatively high cell density. In the second step, transcription is induced depending on the type of promoter used. In this regard, a tac promoter is particularly suitable which can be induced by lactose or IPTG (=isopropyl-β-D-thiogalactopyranoside) (deBoer et al., Proc. Natl. Acad. Sci. USA 80 (1983), 21-25). Termination signals for transcription are also described in the literature.

The term "transformation" is used herein in its standard meaning, known to the skilled person. The transformation of the host cell with a polynucleotide or vector as described above can be carried out by standard methods, as for instance described in Sambrook and Russell (2001), Molecular Cloning: A Laboratory Manual, CSH Press, Cold Spring Harbor, NY, USA; Methods in Yeast Genetics, A Laboratory Course Manual, Cold Spring Harbor Laboratory Press, 1990. The host cell is cultured in nutrient media meeting the requirements of the particular host cell used, in particular in respect of the pH value, temperature, salt concentration, aeration, antibiotics, vitamins, trace elements etc.

The term "microorganism" in the context of the present invention refers to bacteria, as well as to fungi, such as yeasts, and also to algae and archaea. In one preferred embodiment, the microorganism is a bacterium. In principle any bacterium can be used.

The term "secretion" as referred to herein means directing the newly synthesized protein in a microorganism configured to synthesize said protein through a cellular membrane, for example through the inner membrane or through both the inner and outer membranes of a prokaryotic organism. Accordingly, the produced polypeptide chain is directed either to the periplasm or to the culture medium. The secretion of newly produced proteins occurs upon presence of a particular sequence which is to be comprised in the sequence of said newly produced protein. Such particular sequence can be referred to as "Secretion signal sequence". The signal peptide encoded by the secretion signal sequence may be endogenous to the host cells, or they may be exogenous, including signal peptides native to the polypeptide to be expressed. Secretion signal sequences are typically present at the amino terminus of a polypeptide to be expressed, and are typically removed enzymatically between biosynthesis and secretion of the polypeptide from the cytoplasm. Thus, the signal peptide is usually not present in a mature protein product.

As used herein, the term "sequence identity" describes to which extent two sequences, for example two nucleic acid sequences or two polypeptide sequences, in the context of the present invention preferably two polypeptide sequences, are identical. When sequences to be compared are of the same length, the sequence identity refers to the percentage of amino acid residues in a first sequence that are identical to amino acid residues in second the second sequence and includes reference to the residues in the two sequences that are the same when aligned for maximum correspondence over a specified comparison window. When the sequences which are compared do not have the same length, the degree of identity either refers to the percentage of amino acid residues in the shorter sequence which are identical to amino acid residues in the longer sequence or to the percentage of amino acid residues in the longer sequence which are identical to amino acid residues in the shorter sequence. Preferably, it refers to the percentage of amino acid residues in the shorter sequence which are identical to amino acid residues in the longer sequence. The degree of sequence identity can be determined according to methods well known in the art using preferably suitable computer algorithms such as CLUSTAL. When using the Clustal analysis method to determine whether a particular sequence is, for instance, at least 60% identical to a reference sequence default settings may be used or the settings are preferably as follows: Matrix: blosum 30; Open gap penalty: 10.0; Extend gap penalty: 0.05; Delay divergent: 40; Gap separation distance: 8 for comparisons of amino acid sequences. For nucleotide sequence comparisons, the Extend gap penalty is preferably set to 5.0. In a preferred embodiment ClustalW2 is used for the comparison of amino acid sequences. In the case of pairwise comparisons/alignments, the following settings are preferably chosen: Protein weight matrix: BLOSUM 62; gap open: 10; gap extension: 0.1. In the case of multiple comparisons/alignments, the following settings are preferably chosen: Protein weight matrix: BLOSUM 62; gap open: 10; gap extension: 0.2; gap distance: 5; no end gap. Preferably, the degree of identity is calculated over the complete length of the sequence.

Sequences referred to in this application are recited according to FASTA format hereinbelow, and are also given in a sequence listing (sequence protocol) prepared in .xml format according to WIPO St. 26 appended to this application. Each sequence is identified by its specific sequence identifier, also referred to as SEQ ID. In the case of conflict between sequences recited in the present description and corresponding sequences recited in the appended sequence listing, the present invention relates to both sequences, preferably the present invention relates to a sequence as in the appended sequence listing.

If a reference is made to an amino acid residue incorporated in a peptide it is meant that said amino acid residue is connected to a further amino acid residue or a peptide moiety through a peptide bond, in other words that either its -COOH group or its amino group (i.e. -NH₂ for the natural aminoacid with the exception of proline, which includes a secondary amine) is transformed into a peptide bond.

Odilorhabdin as referred to herein are are a class of natural antibacterial agents produced by the bacterium Xenorhabdus nematophila. Odilorhabdins act against both Gram-positive and Gram-negative pathogens, and were shown to eliminate infections in mouse models. One exemplary odilorhabdin is characterized by the followingsequence:
(Lys)₁-(Dab(β OH))₂-(Dab(β OH))₃-(Gly)₄-(Orn)₅-(Pro)₆-(His)₇-(Dhl)₈-(Dha)₉-(Dhl)₁₀-(Dbt)₁₁
wherein Dab(βOH) is 2,4-diamino-3-hydroxy-butyrate, preferably (2S,3S)-2,4-diamino-3-hydroxybutyrate, Orn is L-ornithine, Dhl is L-5-hydroxylysine (also referred to as δ-hydroxy lysine), Dha is 2,3-didehydroarginine (also referred to as α,β-dehydroarginine or dehydroarginine) and Dbt is 1,4-diaminobutane (also referred to as α,δ-diamino butane). Accordingly, based on structures of different odilorhabdins as discloed herein and as known to the skilled person, odilorhabdins may be defined as peptide comprising the following sequence:
(Lys)₁-(Dab(β OH))₂-(AA3)₃-(Gly)₄-(Orn)₅-(Pro)₆-(AA7)₇(AA8)₈-(Dha)₉
wherein AA3 is Dab(β OH) or Dab, wherein Dab is 2,4-diaminobutyrate, preferably (2S)-2,4-diaminobutyrate, AA7 is an aromatic amino acid residue, preferably selected from His, Phe, Tyr, Trp or modified Phe wherein the phenyl ring is optionally substituted with one or more optional substituents independently selected from Hal (preferably F), -OH, -SH, -NH₂, -CN, C₁₋₅ alkyl, C₂₋₅ alkenyl and C₂₋₅ alkynyl, and wherein AA8 is Dhl or Lys.

### Sequences:

SEQ ID NO: 1: 
MGCVVEVTRFDQNECHRTLAQGCAIVFRGLLKDWPAIERWGADYFQAKCPQIDVIIKFFDADGVRFEPAKMYRYL
ELLNEYKKGEKGGSIPYCHDIPIFLQAQQLLDDIGDFPLMALPKLYREDWWQFVQFFMSPAGAVTPLHFDTLRTN
NLFFQIQGSKRFTFIPWEERNACSRRGWRWFEYNPEIQGDNKFVNISNCTIEVHQGDMLLMPAGMLHHVRTLTD
SISFNIDFHTRSSAIASFKSIKEKMPRENLYYNGLSLCGLLGLIKRERFFEKYRPYLNYIS
SEQ ID NO: 2: 
MLELFEDQLVEIKEKYGHDCLKRVEDMFYPLLQQRSPLQADAKYIMPGLSTTPWLETNDFPQLQPLVTALMKSAG
KIKQEMNAVMSGECEYVIDYEHYLGTQKDWKALYLFKNGQPNSAIANILPATWYIFNNELRDWHCPLLEVHFSVL
QPGTVIKPHCDLWNFTLNLHFAVDIPASNCQIIVANEARNWQEGECLLFDYSYQHEAYNRSDKHRICLLMDIWHP
NLSFAEREALVLIFTGIRKLFG
SEQ ID NO: 3: 
MMPDTQEVSDFIFELNPTELISLTQMFSESDLKYDIDNFEDFYWKCFQYSFNLPERLQKRLFNFRALENKNYLLIKG
LPVPDNLSLTPLSYEQTNSPEVASVRTLISTILSRIGYIYSFVNKKNFNFIDDIFPIEKYKDMQLGTNKKFLEWHVED
GFHDAKADWVALYCLRGDINAETYLFQTKNIHLDAETVSELQKPNFEIDADPTFMSDTDGCRRIAVLSQHKEPEIV
FDPAYMRCLTPNSEIALKKLNQCIDENREAFILSPGEMLLFDNRKVAHARSEYEPRYDGYDRWLLRALILESKFKA
RDHFYAGTLRAK
SEQ ID NO: 4: 
MIMTSFKTIILLLTILLIIGCGESNSYPIKEAAPTFEARIVRTHYGVPHIYAKSLGSLSFGNAYAQAQDNICIMADNYVR
VKGERARFFGPDKEKKGDALNLIQDFGYKSLAFVEQASEKWSQLSPDSQEIIDGFVAGYNTYLSHVKEGRYSLPK
 
ACRDQEWVVPVRREDVLAYLFSIAAFPGSERFMADYYAANPNNTGDQKQLASSQHDDHGYSIGSNAWALGHDK
IEQGNGMLLANPHFPYEGDMRFWQAHHVVDGKIDVMGASLVGFPGVINIGFNRHIAWTHTYSSAEHVVVYKMQT
ADNNPLSYVIDGVVRPVKSKPVHIEFKSDNGIQTITKDHFYTDIGPIIENEDFIWSESEFYVVKDINLYNLDMIDHWLA
LNRANSLAEVKQSYKDFNGLVFNNTLLTGREDVGVFMGDKAVPALSFEAIQLLKNDEMWSAARKEYGVTILPGHL
SSMIFTAALGYDQTPQLVKSSYVQNANDSHWATNPKHLLEGFSPLLGNERSPLSFRTRMGLQLIEDAAGDDKKFS
LMELEQALLSDRSYLAELILKDVVALCDETPNKLINMMNKVKVDITQACQLLSEWNGEFNADSRGAIVFRELAFLID
QQEHFSVPFDMAYPATTPRQLKQNEEIITLLARAVINVNNVGLDLDTPVNKFQFIESTAPDGKPAGNRMSWPGVF
EEEGGFNMIATSDWDKTLFPKHHYPVGNDVVSGEELESQLTDQGYHVRFGSSWSMLVEFNQQGPNARGLLTYS
QSSDLDSPHFADQSLFYSRYKQLLPLPYRSEEIKQSTISELIIQH
SEQ ID NO: 5:

DVYPLSSLQMGSIYHQLKDESYHIVEHYAINAEWNEQAFYQVFNAIVQRHELLRVAYNFKLGRALQYVRKTVALPI
VLNDWRNLSAQEFEHQLGYCLEQENNHFFVEDELMWRVNVLIDAGNTFVLVLSHQHAILDGWSVANLTVEFMGN
YLSVSSGGAVPLRADLPHYGEYIAEELAAIDSPEQKTYWQHIANAAVLPDWTGKESTQHYASRLELSYLHDDLQQ
LAGRLNVPLRTLLLAVHVYVLAKVNGTDSITSSVVSHGRLEVEGAEACLGLFLNTPPVNANIDQQTWASLIQQLTE
YNTASWPYRHYPVAMMQEVFKCDYSGALFNYVDFHVADELQATQDAESEQIYEHEHEHEYERVDYGYDLVCHH
SSASGFLALQLALTQSVFKAHQVSQILAYYETAIKSMLVSQHHRLAHTDYLAPGQIQHL

The invention will be illustrated by the following Examples. The Examples serve illustrative purpose and are not meant to be construed as limiting.

### Examples

Non-ribosomal peptide synthetases (NRPS) are giant multimodular enzymes found in bacteria and fungi, which produce a wide range of natural products with diverse biological activities. Only recently identified was odilorhabdin, a compound produced in entomopathogenic bacteria of the genera *Xenorhabdus* and *Photorhabdus* (1). Its antibiotic activity against Gram-negative bacteria inspired the development of the derivative NOSO-502, which holds huge potential in application as a new clinical drug (2,3). While the mechanism and target of this drug was broadly investigated, the synthesis pathway encoded by the odilorhabdin biosynthetic gene cluster (BGC) remained poorly understood. Described here are the utilization of NRPS engineering to elucidate the role of several NRPS domains as well as three additional hydroxylases and one deacylase involved in the synthesis of odilorhabdin. Therefore, individual NRPS modules of the odilorhabdin synthetase were assembled to well-studied BGCs or artificial NRPS encoding BGCs to *in vivo* investigate the bio-synthesis of respective peptides in *E. coli.* Besides obtaining mechanistic knowledge about the biosynthesis, these insights could be directly applied to generate non-proteinogenic amino acid building blocks or novel odilorhabdin analogoues via NRPS engineering in the future.

In order to understand the synthesis mechanism of the odilorhabdin synthetase (1) (OdlS), the first two modules (M) of OdlS were assembled to the last two modules of GameXPeptide Synthetase (4) (GxpS) by the recently introduced evolution inspired NRPS engineering approach, denoted as XUT approach (Fig. 2)(5). Both NRPS fragments, OdlS_M_1-2 and GxpS_M_4-5, were fused within the thiolation (T) domains in front of two strictly conserved glycine residues (Position IV) (Fig. 3A). The hybrid *NRPS-1,* cloned on a pACYC plasmid, was co-transformed with pCOLA and pCDF plasmid controls (empty multiple cloning side) into *E*. *coli* DH10B::*mtaA* strain. pCOLA and pCDF served as a control for co-expressing further genes of the odilorhabdin BGC. Induction of NRPS-1 along with pCOLA and pCDF in absence of L-2,4-Diaminobutyric acid (DABA) led to the production of compounds **9** and **10** (Fig. 7). However, supplementation with 1 mM DABA resulted in compounds **1, 2, 5, 6** and **11,** all containing DABA in their peptide chains (Fig. 3B and C). When *odIB* was additionally expressed from pCOLA plasmid, products **3, 4, 7, 8** and **12** were produced instead. The mass shifts of 16, the retention times as well as the peptide fragmentation indicated a hydroxylation of the DABA residue, which is reported for odilorhabdin at its beta position. Interestingly, all compounds featured either a C10 or C12 fatty acid at the *N*-terminus of the peptide, a modification not described for odilorhabdin. Therefore, *NRPS-1* and *odIB* were co-expressed with *odIG* cloned on a pCDF plasmid, which led to the absence of nearly all previous products except **12** and the formation of a new product **13.** This finding supports a role of OdlG in the removal of a potential *N*-terminal fatty acid introduced by the OdlS condensation starter domain. However, a decrease in growth upon co-expression of *odIG* was observed (Fig. 8A). Therefore, *odIG* was cloned to pCDF plasmid containing a vanillic acid promotor substituting the original *pBAD* promotor. Co-expression of *NRPS-1* together with *odIB* and *odIG* induced with 24-hour delay upon addition of vanililic acid showed no effects on growth while compounds **12** and **13** were produced at comparable levels (Fig. 8B). Therefore, further experiments involving odIG were performed with the modified pCDF plasmid instead.

To investigate whether *odIB* operates as a standalone hydroxylase specific for DABA or in association with OdIS, cross-feeding experiments were performed (Fig. 4A) - one strain expressed *odIB* and a second strain *NRPS-1.* Co-cultivation of both strains resulted in the same product formation of **3** and **7** with 15-fold lower intensity compared to expressing both genes in the same cell (Fig. 4B). This indicated an OdlB hydroxylase activity independent of OldS. Furthermore, a small metabolite analysis demonstrated that the expression of *odIB* in the presence of DABA **(14)** led to the formation of a more hydrophilic compound with a mass shift of 16, which we assigned as compound **15** (Fig. 4C). This finding supports a role of OdlB as standalone DABA hydroxylase.

To demonstrate a direct application of these findings for NRPS engineering, NRPS-1 was further modified by substitution of the first module of OdlS (Fig. 5A). As described for the construction of NRPS-1, the XUT approach was applied once more to assemble the Xenolindicine synthetase (6) (XldS) starter module (M1) to the second OdlS module (M2), resulting in NRPS-2. Expression of *NRPS-2* in medium supplemented with 1 mM DABA resulted in formation of **16** and **17, with** DABA incorporated into the peptide. Additional expression of *odIB* led to production of compound **17** and **19,** the DABA hydroxylated derivatives of **16** and **17.** Consequently, XUT NRPS engineering can specifically introduce the second module of OdlS to other NRPS, which incorporates DABA with the option for further modification by hydroxylation of the beta position via expression of *odIB.*

To characterize the specificity of Od!G as a deacylase, the last module of GxpS (M5) was merged via XUT NRPS engineering with either the first module (M1) of OdlS (NRPS-3), XldS (NRPS-4), or Xenoamicin Synthetase (XabABC) from *X*. *stockiae* (NRPS-5) (5,7). Co-expression of *NRPS-3* with pCDF empty vector control induced with a delay of 24 hours, resulted in product formation of **9, 10, 20, 21, 22** and **23** while for *NRPS-4* products **26** to **33** and for *NRPS-5* **34** to **37** were detected (Figure 5B and 17). However, when *NRPS-3,* -*4* and -*5* were expressed with *odIG*, it was observed that only products of NPRS-3 vanished while products of NRPS-4 and -5 were not affected by co-expression of *odIG*. Since product **27** differs only in a glutamine residue from compound **21** produced by NRPS-3, we concluded that OdlG has a specificity for lysine carrying a fatty acid at the *N*-terminus. Furthermore, co-expression of *odIG* with NRPS-3 resulted in production of compounds **24** and **25,** which corresponds to masses of either linear or cyclic di-peptides of deacylated NRPS-3 products.

Beside the non-proteinogenic amino acids at the *N*-terminus of odilorhabdin, unusual structures were also reported at the C-terminus of the peptide. Therefore, the last two modules of OdlS (M8 & M9) were fused by XUT to an artificial NRPS described in previous studies leading to NRPS-6 (Fig. 6A)(5). The construct consisted of the first module of XldS assembled via XUT to xenoamicin (7) (XabABC) T1 and C2 domain from *X*. *doucetiae,* which in turn was merged by earlier described XU engineering approach to Kolossin (8) synthetase A2 and T2. Utilization of this artificial NRPS was reasoned by previous experiments with high production titers, linear peptide structure and incorporation of an inert amino acid. The expression of *NRPS-6* without any additional genes resulted in no detectable production of any product (Fig. 6B). However, co-expression with *odIF* cloned on pCOLA plasmid led to production of compounds **38** and **39.** While the fragmentation pattern of both compounds indicated the incorporation of dehydrated arginine, the latter also contained a putrescin at its C-terminus. Compound **40** was produced upon co-expression of *odIF* and *odIE* which hydroxylated the terminal lysine, a modification described for odilorhabdin at the lysine delta position. Furthermore, compound **41** was detected, were we predict a *C*-terminal lactam ring structure formed by the lysine.

Based on current literature, we hypothesized a role of OdlF in hydroxylation of *L*-arginine at its beta position. Subsequently, the second last OdlS A domain (A 8 domain) binds and activates the modified amino acid, which gets covalently attached to the T domain. Next, the following ^{D}C_{L} domain dehydrates the amino acid, resulting in the formation of 2,3-didehydroarginine. To verify the incorporation of L-3-hydroxyarginine, NRPS-6 was further modified to NRPS-7 by assembly of GxpS thioesterase (TE) domain via XUT to the second last module (M8) of OdlS (Fig. 6C). In this experiment the incorporation of the GxpS TE domain served as an intermediate off loader of the nascent peptide chain. Expression of *NRPS-7* with a pCOLA empty vector control produced compound **46** consisting of a *C*-terminal *L*-arginine (Fig. 6C). Co-expression with *odIF* produced **47** with a mass shift of 16 and a fragmentation pattern indicating a hydroxylation at the incorporated *L*-arginine. Furthermore, a cross-feeding experiment with OdlF and NRPS-6, as demonstrated for the characterization of OdlB, as well as small metabolite analysis in LB medium were performed (Fig. 6B and 28). As for OdlB, these experiments pointed to an OdlS independent activity of OdlF in modifying *L*-arginine to *L*-3-hydroxyarginine.

The same experiments were also carried out to analyze the dependency of OdlE to OdlS as a hydroxylase of *L-*lysine. However, no OdlS independent hydroxylase activity was observed.

The final modification analyzed in this work is the *C*-terminal putrescin of odilorhabdin. NRPS-6 produced **38** and **39,** which have a carboxy and a putrescine terminus, respectively. Since OdlS TE domain consists of a cysteine at the active center where other TE domains possess a conserved serine residue (9), a role in putrescin transfer was suspected for this domain (Fig. 32C). Therefore, NRPS-6 C3457 was substituted by a serine (C3457S) or alanine (C3457A) via site directed mutagenesis (Fig. 6D). Both mutants, as well as a mutant were the entire TE domain was deleted, showed no production of compound **39,** while **38** was still produced. This indeed emphasize a role of the TE domain in putrescin transfer to the peptide. Substitution of the OdlS TE by GxpS TE utilizing the XUT engineering approach resulted in the absence of both compounds. This finding can be explained by the modification of the T domain where the terminal hybrid T domain is not capable to interact properly with the upstream domains. Therefore, no products are detected.

### Materials and Methods

### Cultivation of strains

All *E*. *coli* DH10B::*mtaA* were cultured either on liquid or solid low salt LB medium (pH 7.5, 10 g/L tryptone, 5 g/L yeast extract and 5 g/L NaCl). As selection markers either chloramphenicol (34 ug/ml), kanamycin (50 µg/ml) or spectinomycin (50 µg/ml) was added. Solid medium was prepared with 1% agar (w/v). Cells were cultivated at 37 °C.

### Cloning of biosynthetic gene clusters and NRPS modules

Genomic DNA (gDNA) was isolated via Monarch^{®} Genomic DNA Purification Kit (NEB) from the respective strains depicted in Table 1 for usage as template in PCR. Alternatively, plasmid DNA was used. Only for cloning of pPC1094 a synthetic gene fragment depicted in Table 4 and Figure 33 was ordered from Integrated DNA technologies (IDT) which was further used as a PCR template. Q5^{®} High-Fidelity DNA Polymerase (NEB) or Hot start *Phusion* DNA Polymerase (NEB/Thermo Fisher Scientific) were utilized to generate all DNA fragments in this work. Primer pair sequences and product sizes of the amplified fragments are documented in Table 3. PCR amplified fragments were purified by gel extraction from 1% (w/v) agarose gels taking the Monarch^{®} DNA Gel Extraction Kit (NEB). Gibson cloning employing NEBuilder^{®} HiFi DNA Assembly Cloning Kit (NEB) assembled the plasmid which was subsequently transformed by electroporation into electro competent *E*. *coli* DH10B. Finally, plasmids were isolated by the PureYield^{™} Plasmid Miniprep System (Promega).

### Heterologous expression of NRPS constructs and modifying genes

Plasmids were transformed via electroporation into electro competent *E*. *coli* DH10B::*mtaA*. At least three different clones were heterologous expressed for each combination of plasmids. Cells were cultivated overnight as described in section 1.2. Afterwards, 4 ml XPP medium (10) containing all necessary antibiotics and 0.02 % *L-*arabinose (w/v) were inoculated with 1% overnight grown culture. 1 mM L-Diaminobutyric acid (DABA) was supplemented to the medium in experiments involving NRPS-1 and -2. Only 0.5 mM DABA was added to cultures analyzing strains expressing *odIB* without any NRPS in a small metabolite analysis (Section 3.5). The cells were cultivated for 72 hours at 22 °C and 180 rpm.

In experiments involving pPC1094 and pPC1096, 4 ml XPP medium containing all necessary antibiotics and 0.02 % *L*-arabinose (w/v) were inoculated with 1% overnight grown culture and were initially incubated for 24 hours at 22 °C and 180 rpm. Subsequently, the vanillic acid promotor was induced upon addition of 100 µM vanillic acid. The cultures were incubated for additional 48 hours at 22 °C and 180 rpm.

For cross-feeding experiments, two strains were co-cultured in te same medium. Both strains contained the same set of base plasmids (pACYC and pCOLA) carrying different or no genes. Therefore, the 4 ml XPP medium containing all necessary antibiotics and 0.002 mg/ml *L*-arabinose was inoculated with 1% overnight grown culture of each strain.

### Culture extraction and HPLC-MS analysis

The cultures were extracted by mixing the culture in a 1:1 ratio with methanol followed by 30 min incubation at room temperature while shaking. Some set of experiments were further diluted 1:10 with methanol. The extract was centrifuged for 30 min at 22 °C and 17000 g. Cleared supernatant was further used for HPLC-MS analysis.

HPLC-UV-HRMS analysis was conducted on an UltiMate 3000 system (Thermo Fisher) coupled to an Impact II qTof mass spectrometer (Bruker) with an ACQUITY UPLC BEH C18 column (130 Å, 2.1 mm × 100 mm, 1.7-µm particle size, Waters) at a flow rate of 0.4 ml min⁻¹ (5-95% acetonitrile/water with 0.1% formic acid, vol/vol,16 min, UV detection wavelength 190-800 nm) and an electrospray ionization (ESI) source set to positive ionization mode.

HPLC-UV-MS analysis was conducted on an UltiMate 3000 system (Thermo Fisher) coupled to an AmaZonX mass spectrometer (Bruker) with an ACQUITY UPLC BEH C18 column (130 Å, 2.1 mm × 100 mm, 1.7-µm particle size, Waters) at a flow rate of 0.4 ml min-1 (5-95% acetonitrile/water with 0.1 % formic acid, vol/vol, 16 min, UV detection wavelength 190-800 nm) and an electrospray ionization (ESI) source set to positive ionization mode. The data was analysed using DataAnalysis 5.3 software (Bruker).

### Small Metabolite Analysis

The sample preparation was performed as described in section 3.4. HPLC-UV-HRMS analysis was conducted on an UltiMate 3000 system (Thermo Fisher) coupled to an Impact II qTof mass spectrometer (Bruker) with an ACQUITY UPLC BEH Amide column (130 Å, 1.7 µm, 2.1 mm × 50 mm) and an electrospray ionization (ESI) source set to positive ionization mode. Prior to each sample injection and analysis, the column was first conditioned with a flow rate of 0.4 ml min-1. Conditioning begins with an isocratic flow of 100% water with 0.1 % formic acid for 4 min, followed by a 1 minute gradient to 90:10 acetonitrile/water mixture with 0.1 % formic acid and another 6 minutes of isocratic flow with 90:10 acetonitrile/water mixture with 0.1 % formic acid. Sample analysis was performed with a flow rate of 0.4 ml min-1 and a 90-40% gradient with acetonitrile/water with 0.1% formic acid over 5 minutes, followed by 2 minutes isocratic flow with 10:90 acetonitrile/water mixture with 0.1% formic.

**Table 1. Strains used in this work.**

| Strain | Genotype/NRPS | Reference |
|---|---|---|
| *E. coli* DH10B | F_mcrA (*mrr-hsd*RMS*-mcr*BC)*,* 80*Iac*ZΔ, M15, Δ*IacX74 rec*A1 *end*A1 *ara*D 139Δ(*ara*, *leu*)7697 *gal*U *gal*K λ *rps*L (*Strr*) *nup*G / - | (11) |
| *E. coli* DH10B::*mtaA* | DH10B with *mtaA* from pCK_*mtaAΔentD* / - | (6) |
| *P. luminescens* subsp. laumondii TT01 | WT (*gxpS, koIS)* | DSMZ |
| *X. nematophila* ATCC19061 | WT (*odIS*, *xtpS, PAX)* | ATCC |
| *X. indica* DSM17382 | WT (*xIdS)* | DSMZ |
| *Bacillus subtilis* subsp. subtilis str. 168 DSM402 | WT (*srfAB*) | DSMZ |
| *X. stockiae* DSM17904 | WT (*xabABC*) | DMSZ |
| *X. doucetiae* 17909 | WT (*xabABC*) | DMSZ |

**Table 2. Plasmids and corresponding NRPS used in this work.**

| NRPS | Plasmids | Genotype | Reference |
|---|---|---|---|
| | pACYC_ara/araE_tacl | ori p15A, *cm^{R}*, *araC- P_{BAD}*, *tacl* and *araE* | (12) |
| | pCOLA_ara/tacl | ori CoIA, *kan^{R}*, *araC- P_{BAD},* and *tacl* | (13) |
| | pCDF_ara/tacl | ori CoIDF13, *spec^{R}, araC- P_{BAD},* and *tacl* | (12) |
| | pPI16 | ori p15A, *cm^{R}*, *araC-P_{BAD} xldS*_C1A1T1_{1/2}*-* | (5) |
| | | *xabA*_T1_{1/2}C1-*koIS_*A2T2C3-*gxpS*_A2T2_{1/2}- | |
| | | *xtvAB*_T2_{1/2}Red *tacl* and *araE* | |
| | pPC1094 | ori CoIDF13, *spec^{R}*, *vanp* and *tacl* | This work |
| 1 | pPC1020 | ori p15A, *cm^{R}*, *araC-P_{BAD}* | This work |
| | | *odIS*_C1A1T1C/E2A2T2_{1/2}- | |
| | | *gxpS*_T3_{1/2}C/E4A4T4CE5A5T5TE *tacI* and *araE* | |
| 2 | pPC1080 | ori p15A, *cm^{R}*, *araC-P_{BAD} xldS*_C1A1T1_{1/2} | This work |
| | | *odIS*_T1_{1/2}-C/E2A2T2_{1/2}- | |
| | | *gxpS*_T3_{1/2}C/E4A4T4CE5A5T5TE *tacI* and *araE* | |
| 3 | pPC1102 | ori p15A, *cm^{R}, araC-P_{BAD} odIS*_C1A1T1_{1/2}- | This work |
| | | *gxpS*_T4_{1/2}CE5A5T5TE *tacI* and *araE* | |
| 4 | pPC1103 | ori p15A, *cm^{R}*, *araC-P_{BAD} xldS*_C1A1T1_{1/2}- | This work |
| | | *gxpS*_T4_{1/2}CE5A5T5TE *tacI* and *araE* | |
| 5 | pPC1104 | ori p15A, *cm^{R}*, *araC-P_{BAD}* | This work |
| | | *xabABC*_C1A1T1_{1/2}-*gxpS*_T4_{1/2}CE5A5T5TE | |
| | | *tacI* and *araE* | |
| 6 | pPC1031 | ori p15A, *cm^{R}*, *araC-P_{BAD} xIdS*_C1A1T1_{1/2}- | This work |
| | | *xabA*_T1_{1/2}C1-*koIS*_A2T2_{1/2} | |
| | | *odIS*_T8_{1/2}C9A8T9C10A9T10TE *tacI* and *araE* | |
| 6 ΔTE | pPC1093 | ori p15A, *cm^{R}*, *araC-P_{BAD} xldS*_C1A1T1_{1/2}- | This work |
| | | *xabA*_T1_{1/2}C1-*koIS*_A2T2_{1/2} | |
| | | *odIS*_T8_{1/2}C9A8T9C10A9T10 *tacI* and *araE* | |
| 6 | pPC1092 | ori p15A, *cm^{R}*, *araC-P_{BAD} xIdS*_C1A1T1_{1/2}- | This work |
| C3457A | | *xabA*_T1_{1/2}C1-*koIS*_A2T2_{1/2} | |
| | | *odIS*_T8_{1/2}C9A8T9C10A9T10TE C3457A | |
| | | *tacI* and *araE* | |
| 6 | pPC1099 | ori p15A, *cm^{RI} araC-P_{BAD} xIdS*_C1A1T1_{1/2}- | This work |
| C3457S | | *xabA*_T1_{1/2}C1-*koIS*_A2T2_{1/2} | |
| | | *odIS*_T8_{1/2}C9A8T9C10A9T10TE C3457S | |
| | | *tacI* and *araE* | |
| 7 | pPC1084 | ori p15A, *cm^{R}*, *araC-P_{BAD} xIdS*_C1A1T1_{1/2}- | This work |
| | | *xabA*_T1_{1/2}C1-*koIS*_A2T2_{1/2} | |
| | | *odIS*_T8_{1/2}C9A8T9_{1/2} *gxpS*_T5_{1/2}TE *tacI* and | |
| | | *araE* | |
| 8 | pPC1095 | ori p15A, *cm^{R}*, *araC-P_{BAD} xIdS*_C1A1T1_{1/2}- | This work |
| | | *xabA*_T1_{1/2}C1-*koIS*_A2T2_{1/2} | |
| | | *odIS*_T8_{1/2}C9A8T9C10A9T10_{1/2} *gxpS*_T5_{1/2}TE *tacI* and *araE* | |
| | pPC1018 | ori CoIA, *kan^{R}*, *araC- P_{BAD} odIB* and *tacI* | This work |
| | pPC1017 | ori CoIA, *kan^{R}*, *araC- P_{BAD} odIF* and *tacI* | This work |
| | pPC1064 | ori CoIA, *kan^{R}*, *araC- P_{BAD} odIEF* and *tacI* | This work |
| | pPC1089 | ori CoIDF13, *spec^{R}*, *araC- P_{BAD} odIG* and *tacI* | This work |
| | pPC1096 | ori CoIDF13, *spec^{R}*, *vanp odlG* and *tacI* | This work |

**Table S\3. Primer and template used in this work to generate indicated plasmids. Sizes of the PCR products are depectided below the template**

| Plasmids | Oligonucleotides | Sequence (5' 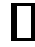 3'; overlapping ends), alternatively restriction enzymes | Template Product size in bp |
|---|---|---|---|
| pPC1094 | pc1214 (SEQ ID NO: 6) | TTTTGTTATCAATAAAAAAGGCCCC | Vanp |
| | | | 1.120 kb |
| | | | (see Table 4) |
| | pc1234 (SEQ ID NO: 7) | TATACGAGCCGATGATTAATTGTCACTAGTATTTCCCC TCTTTCTC | |
| | pc1216 (SEQ ID NO: 8) | TGACAATTAATCATCGGCTCGTATAATGTGTGGAATTG TGAG | pCDF_ara/tacI |
| | | | 2.171 kb |
| | pc1215 (SEQ ID NO: 9) | GGGGCCTTTTTTATTGATAACAAAATTGTCTAGAGCGG TTCAG | |
| pPC1020 | pc1075 (SEQ ID NO: 10) | TGGGCTAACAGGAGGAATTCCATGTTTCTAGATAAAGT CGGGCAG | gDNA |
| | | | *X. nematophila* |
| | | | 6.415 kb |
| | pc1105 (SEQ ID NO: 11) | TGACTGCCAACAGCGAGTGACCGCCGAGGTGGAAAAA GTTGTCGTTTC | |
| | pc1106 (SEQ ID NO: 12) | GGCGGTCACTCGCTGTTGGCAG | gDNA |
| | | | *P. luminescens* |
| | | | TT01 |
| | pc1102 (SEQ ID NO: 13) | GCCAGTGCCAACGAGGCATATTCG | 4.463 kb |
| | pc1103 (SEQ ID NO: | CGAATATGCCTCGTTGGCACTGGC | gDNA |
| | | | *P. luminescens* |
| | 14) | | TT01 |
| | pc1104 (SEQ ID NO: 15) | ATACGAGCCGATGATTAATTGTCACAGCGCCTCCGCTT CACAATTC | 3.019 kb |
| | pc1091 (SEQ ID NO: 16) | TGACAATTAATCATCGGCTCGTATAATGTG | pACYC_ara/araE_t |
| | | | acI |
| | | | 5.220 kb |
| | pc1090 (SEQ ID NO: 17) | GGAATTCCTCCTGTTAGCCCAAAAAAACG | |
| pPC1080 | pc1080 (SEQ ID NO: 18) | TTTTTGGGCTAACAGGAGGAATTCCATGAATATGACAC GTAACCATACATCC | gDNA *X*. *indica* |
| | | | 3.09 kb |
| | pc1197 (SEQ ID NO: 19) | CCCCCAAGCTCAAAGAAATGATCGTGGCGAC | |
| | pc1198 (SEQ ID NO: 20) | ACGATCATTTCTTTGAGCTTGGGGGTCACTCTTTATTA ATTATGTCATTG | gDNA |
| | | | *X*. *nematophila* |
| | | | 3.339 kb |
| | pc1105 | TGACTGCCAACAGCGAGTGACCGCCGAGGTGGAAAAA GTTGTCGTTTC | |
| | pc1106 | GGCGGTCACTCGCTGTTGGCAG | gDNA |
| | pc1102 | GCCAGTGCCAACGAGGCATATTCG | *P. luminescens* |
| | | | TT01 |
| | | | 4.463 kb |
| | pc1103 | CGAATATGCCTCGTTGGCACTGGC ATACGAGCCGATGATTAATTGTCACAGCGCCTCCGCTT CACAATTC | gDNA |
| | pc1104 | | *P. luminescens* |
| | | | TT01 |
| | | | 3.019 kb |
| | pc1091 | TGACAATTAATCATCGGCTCGTATAATGTG | pACYC_ara/araE_t |
| | pc1090 | GGAATTCCTCCTGTTAGCCCAAAAAAACG | acI |
| | | | 5.220 kb |
| pPC1102 | pc1075 | TGGGCTAACAGGAGGAATTCCATGTTTCTAGATAAAGT CGGGCAG | gDNA |
| | | | *X*. *nematophila* |
| | pc1246 (SEQ ID NO: | GTACCGCAAGCAACGAATGGCCCCCCAAAGCAAAAAA GTTGTCATGGCG | 3.121 kb |
| | 21) | | |
| | pc1245 (SEQ ID NO: 22) | GGGGGCCATTCGTTGCTTGCGGTAC | gDNA |
| | | | *P. luminescens* |
| | | | TT01 |
| | pc1104 | ATACGAGCCGATGATTAATTGTCACAGCGCCTCCGCTT CACAATTC | 4.230 kb |
| | pc1091 | TGACAATTAATCATCGGCTCGTATAATGTG | pACYC_ara/araE_t |
| | pc1090 | GGAATTCCTCCTGTTAGCCCAAAAAAACG | acI |
| | | | 5.220 kb |
| pPC1103 | pc1080 | TTTTTGGGCTAACAGGAGGAATTCCATGAATATGACAC GTAACCATACATCC | gDNA *X*. *indica* |
| | | | 3.110 kb |
| | pc1247 (SEQ ID NO: 23) | GTACCGCAAGCAACGAATGGCCCCCAAGCTCAAAGAA ATGATCGTGGC | |
| | pc1245 | GGGGGCCATTCGTTGCTTGCGGTAC | gDNA |
| | pc1104 | ATACGAGCCGATGATTAATTGTCACAGCGCCTCCGCTT CACAATTC | *P. luminescens* |
| | | | TT01 |
| | | | 4.230 kb |
| | pc1091 | TGACAATTAATCATCGGCTCGTATAATGTG | pACYC_ara/araE_t |
| | pc1090 | GGAATTCCTCCTGTTAGCCCAAAAAAACG | acI |
| | | | 5.220 kb |
| pPC1104 | pc1242 (SEQ ID NO: 24) | TTTTTGGGCTAACAGGAGGAATTCCATGCCTATGTCGT GCAATCGTATC | gDNA *X*. *stockiae* |
| | | | 3.185 kb |
| | pc1248 (SEQ ID NO: 25) | GTACCGCAAGCAACGAATGGCCCCCCAATTCAAAGAA ATGGTCACGGCG | |
| | pc1245 | GGGGGCCATTCGTTGCTTGCGGTAC | gDNA |
| | pc1104 | ATACGAGCCGATGATTAATTGTCACAGCGCCTCCGCTT CACAATTC | *P. luminescens* |
| | | | TT01 |
| | | | 4.230 kb |
| | pc1091 | TGACAATTAATCATCGGCTCGTATAATGTG | pACYC_ara/araE_t |
| | pc1090 | GGAATTCCTCCTGTTAGCCCAAAAAAACG | acI |
| | | | 5.220 kb |
| pPC1031 | pc1080 | TTTTTGGGCTAACAGGAGGAATTCCATGAATATGACAC GTAACCATACATCC | pPI16 |
| | | | 6.226 kb |
| | pc1078 (SEQ ID NO: 26) | GTTGTCATACCGACTGATCCGC | |
| | pc1128 (SEQ ID NO: 27) | TGAGCGGATCAGTCGGTATGACAACTTTTTCAGGCTC GGAGG | gDNA |
| | | | *X. nematophila* |
| | | | 7.779 kb |
| | pc1079 (SEQ ID NO: 28) | TACGAGCCGATGATTAATTGTCACTCATTATTATGCCT GCTTTGTTTTTC | |
| | pc1091 | TGACAATTAATCATCGGCTCGTATAATGTG | pACYC_ara/araE_t |
| | pc1090 | GGAATTCCTCCTGTTAGCCCAAAAAAACG | acI |
| | | | 5.220 kb |
| pPC1093 | pc1080 | TTTTTGGGCTAACAGGAGGAATTCCATGAATATGACAC GTAACCATACATCC | pPI16 |
| | | | 6.226 kb |
| | pc1078 | GTTGTCATACCGACTGATCCGC | |
| | pc1128 | TGAGCGGATCAGTCGGTATGACAACTTTTTCAGGCTC GGAGG | gDNA |
| | | | *X. nematophila* |
| | pc1210 (SEQ ID NO: 29) | CGCCGACAGATTACGCCAATCATTGAGC | 3.705 kb |
| | pc1211 (SEQ ID NO: 30) | GCTCAATGATTGGCGTAATCTGTCGGCG | gDNA |
| | | | *X. nematophila* |
| | | | 2.927 kb |
| | pc1231 (SEQ ID NO: 31) | TATACGAGCCGATGATTAATTGTCATTGTTGCCGTTGT TTGCAC | |
| | pc1091 | TGACAATTAATCATCGGCTCGTATAATGTG | pACYC_ara/araE_t |
| | pc1090 | GGAATTCCTCCTGTTAGCCCAAAAAAACG | acI |
| | | | 5.220 kb |
| pPC1092 | pc1080 | TTTTTGGGCTAACAGGAGGAATTCCATGAATATGACAC GTAACCATACATCC | pPI16 |
| | | | 6.226 kb |
| | pc1078 | GTTGTCATACCGACTGATCCGC | |
| | pc1128 | TGAGCGGATCAGTCGGTATGACAACTTTTTCAGGCTC GGAGG | gDNA |
| | | | *X. nematophila* |
| | | | 3.705 kb |
| | pc1210 | CGCCGACAGATTACGCCAATCATTGAGC | |
| | pc1211 | GCTCAATGATTGGCGTAATCTGTCGGCG | gDNA |
| | pc1230 (SEQ ID NO: 32) | CCACAGCATCCCCCACCAGCCGATAATG | *X. nematophila* |
| | | | 3.197 kb |
| | pc1229 (SEQ ID NO: | TATCGGCTGGTGGGGGATGCTGTGGGGGCATGTGTT GCTTTCG | gDNA |
| | | | *X. nematophila* |
| | 33) | | 905 bp |
| | pc1079 | TACGAGCCGATGATTAATTGTCACTCATTATTATGCCT GCTTTGTTTTTC | |
| | pc1091 | TGACAATTAATCATCGGCTCGTATAATGTG | pACYC_ara/araE_t |
| | pc1090 | GGAATTCCTCCTGTTAGCCCAAAAAAACG | acI |
| | | | 5.220 kb |
| pPC1099 | pc1080 | TTTTTGGGCTAACAGGAGGAATTCCATGAATATGACAC GTAACCATACATCC | pPI16 |
| | | | 6.226 kb |
| | pc1078 | GTTGTCATACCGACTGATCCGC | |
| | pc1128 | TGAGCGGATCAGTCGGTATGACAACTTTTTCAGGCTC GGAGG | gDNA |
| | | | *X. nematophila* |
| | pc1210 | CGCCGACAGATTACGCCAATCATTGAGC | 3.705 kb |
| | pc1211 | GCTCAATGATTGGCGTAATCTGTCGGCG | gDNA |
| | pc1238 (SEQ ID NO: 34) | CAGAATCCCCCACCAGCCGATAATG | *X. nematophila* |
| | | | 3.194 kb |
| | pc1239 (SEQ ID NO: 35) | TATCGGCTGGTGGGGGATTCTGTGGGGGCATGTGTTG CTTTCG | gDNA |
| | | | *X. nematophila* |
| | | | 905 bp |
| | pc1079 | TACGAGCCGATGATTAATTGTCACTCATTATTATGCCT GCTTTGTTTTTC | |
| | pc1091 | TGACAATTAATCATCGGCTCGTATAATGTG | pACYC_ara/araE_t |
| | pc1090 | GGAATTCCTCCTGTTAGCCCAAAAAAACG | acI |
| | | | 5.220 kb |
| pPC1084 | pc1080 | TTTTTGGGCTAACAGGAGGAATTCCATGAATATGACAC GTAACCATACATCC | pPI16 |
| | | | 6.226 kb |
| | pc1078 | GTTGTCATACCGACTGATCCGC | |
| | pc1128 | TGAGCGGATCAGTCGGTATGACAACTTTTTCAGGCTC | gDNA |
| | | GGAGG | *X. nematophila* |
| | pc1205 (SEQ ID NO: 36) | CATAGCGAGCAGCGAATGTCCACCTAAAGCGAAGAAA TTATCGTAACGAC | 3.249 kb |
| | pc1206 (SEQ ID NO: 37) | GGTGGACATTCGCTGCTCGCTATGC | gDNA |
| | | | *P. luminescens* |
| | | | TT01 |
| | pc1104 | ATACGAGCCGATGATTAATTGTCACAGCGCCTCCGCTT CACAATTC | 1.017 kb |
| | pc1091 | TGACAATTAATCATCGGCTCGTATAATGTG | pACYC_ara/araE_t |
| | pc1090 | GGAATTCCTCCTGTTAGCCCAAAAAAACG | acI |
| | | | 5.220 kb |
| pPC1095 | pc1080 | TTTTTGGGCTAACAGGAGGAATTCCATGAATATGACAC GTAACCATACATCC | pPI16 |
| | | | 6.226 kb |
| | pc1078 | GTTGTCATACCGACTGATCCGC | |
| | pc1128 | TGAGCGGATCAGTCGGTATGACAACTTTTTCAGGCTC GGAGG | gDNA |
| | | | *X. nematophila* |
| | pc1210 | CGCCGACAGATTACGCCAATCATTGAGC | 3.730 kb |
| | pc1211 | GCTCAATGATTGGCGTAATCTGTCGGCG | gDNA |
| | pc1235 (SEQ ID NO: 38) | GCATAGCGAGCAGCGAATGTCCACCCATTTCCATAAA AGATGCGGTTAAG | *X. nematophila* |
| | | | 2.786 kb |
| | pc1206 | GGTGGACATTCGCTGCTCGCTATGC | gDNA |
| | pc1104 | ATACGAGCCGATGATTAATTGTCACAGCGCCTCCGCTT CACAATTC | *P. luminescens* |
| | | | TT01 |
| | | | 1.017 kb |
| | pc1091 | TGACAATTAATCATCGGCTCGTATAATGTG | pACYC_ara/araE_t |
| | pc1090 | GGAATTCCTCCTGTTAGCCCAAAAAAACG | acI |
| | | | 5.220 kb |
| pPC1018 | pc1098 (SEQ ID NO: 39) | TTTTTGGGCTAACAGGAGGAATTCCATGGGCTGTGTA GTAGAAGTG | gDNA |
| | | | *X. nematophila* |
| | | | 905 bp |
| | pc1099 (SEQ ID NO: 40) | TATACGAGCCGATGATTAATTGTCAAGAAATATAATTAA GGTACGGACGGTAC | |
| | pc1091 | TGACAATTAATCATCGGCTCGTATAATGTG | pCOLA_ara/tacl |
| | pc1090 | GGAATTCCTCCTGTTAGCCCAAAAAAACG | 3.309 kb |
| pPC1017 | pc1096 (SEQ ID NO: 41) | TTTTTTGGGCTAACAGGAGGAATTCCATGATGCCTGAT ACTCAGGAAG | gDNA |
| | | | *X. nematophila* |
| | | | 1.008 kb |
| | pc1097 (SEQ ID NO: 42) | TATACGAGCCGATGATTAATTGTCATTTCGCCCTTAAA GTACCTG | |
| | pc1091 | TGACAATTAATCATCGGCTCGTATAATGTG | pCOLA_ara/tacl |
| | pc1090 | GGAATTCCTCCTGTTAGCCCAAAAAAACG | 3.309 kb |
| pPC1064 | pc1088 (SEQ ID NO: 43) | GTTTTTTTGGGCTAACAGGAGGAATTCCATGTTGGAAC TATTTGAAGATCAATTG | gDNA |
| | | | *X. nematophila* |
| | | | 1.835 kb |
| | pc1097 | TATACGAGCCGATGATTAATTGTCATTTCGCCCTTAAA GTACCTG | |
| | pc1091 | TGACAATTAATCATCGGCTCGTATAATGTG | pCOLA_ara/tacl |
| | pc1090 | GGAATTCCTCCTGTTAGCCCAAAAAAACG | 3.309 kb |
| pPC1089 | pc1222 (SEQ ID NO: 44) | TTTTTGGGCTAACAGGAGGAATTCCATGATAATGACAT CATTTAAGAC | gDNA *X*. *nematophila* |
| | | | 2.453 kb |
| | pc1223 (SEQ ID NO: 45) | TATACGAGCCGATGATTAATTGTCAATGTTGAATAATTA ACTCTGAAATC | |
| | pc1091 | TGACAATTAATCATCGGCTCGTATAATGTG | pCOLA_ara/tacl |
| | pc1090 | GGAATTCCTCCTGTTAGCCCAAAAAAACG | 3.368 kb |
| pPC1096 | pc1236 (SEQ ID NO: 46) | ACTAGAGAAAGAGGGGAAATACTAGATGATAATGACAT CATTTAAGAC | gDNA |
| | | | *X. nematophila* |
| | | | 2.453 kb |
| | pc1223 | TATACGAGCCGATGATTAATTGTCAATGTTGAATAATTA ACTCTGAAATC | |
| | pc1216 | TGACAATTAATCATCGGCTCGTATAATGTGTGGAATTG TGAG | pPC1094 |
| | | | 3.241 kb |
| | pc1213 (SEQ ID NO: 47) | CTAGTATTTCCCCTCTTTCTC | |

**Table 4. Vanp promotor sequence. Rearranged vanillic promotor derived from sequences described in (14).**

| Promotor | Sequence |
|---|---|
| Vanp (SEQ ID NO: 48) | TTTTGTTATCAATAAAAAAGGCCCCCCGATTTGGGAGGCCTTTTTAGTTAGAGGATCCTTATCA ATCTGCACGAATTGACCATGCTGCACCCAGCGGTGCGCCTGCGCTTGCTGCTGCTTCAAAAAC TTTTGCATTACGAATTGCTGCCAGTGCATGATCACGCATAATACGTTCTGCACCTTCGGCATCA CCACAGCTAACTGCATCCAGAACTGCCTGATGCTGACGATGTGCTGCCAGCAGATGTTCATAT TCGGCAGACAGGTCCATCAGATCCAGGGCCAGTGCACCGGCTGCTGCAAACGGTTCAAAACC ATTACGTGCCAGTGCGCTTTCAACTGCACCATTACCTGCTGCGCTAACCAGGGTATCATGAAAT GCCTGATTATATGCGGCATAACGATCCAGATCTTCACCATTCAGGCGACCGGCTGCAAACAGT GCTTCACCTTCTGCAATCAGTACAACAAAACGTGCATGGGTTTCTGCGGTCATACCACGTTCTG CCAGACGACGTGCTGCAAAACCTTCCAGAACACCACGAACTTCAATTGCATCACGAATCTGAT CGCTGCTAACACCACGGGCTGCATAACCACGTGCACCCAGACGAACAACCAGACCTTCTTGTT CCAGTGAACGCAGTGCGATACGAACCGGCATACGGCTAACACCCAGTGCTGCTGCGGTCGGA ATTTCTGCAATACGTTCACCACTTTTGATTTCACCGCTTGCAATCATTTTACGCAGTGCCATCAT AACACGCTGACCCGGTTTAATACGAGGCATGTCCATTATTCACCCCCCTGAATTGACTCTCTTC CGCTAGCACTGTACCTAGGACTGAGCTAGCCGTCAACATTGGACCAAAACGAAAAAAGGCCCC CCTTTCGGGAGGCCTCTTTTCTGGAATTTGGTACCGAGGCTTAACGATCGTTGGCTGATTGGA TCCAATTGACAGCTAGCTCAGTCCTAGGTACCATTGGATCCAATAGCTGTCACCGGATGTGCTT TCCGGTCTGATGAGTCCGTGAGGACGAAACAGCCTCTACAAATAATTTTGTTTAATACTAGAGA AAGAGGGGAAATACTAG |

**Table 5. Detected compounds in the present study.**

| Compound | MS detected [M+H]⁺ | MS calculated [M+H]⁺ | Molecular **ion** formula | 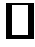 ppm |
|---|---|---|---|---|
| 1 | 314.2439 2+ | 314.2438 | C₃₂H₆₄N₆O₆ | -0.2 |
| 1' | 314.2442 2+ | 314.2438 | C₃₂H₆₄N₆O₆ | -1.1 |
| 2 | 328.2597 2+ | 328.2595 | C₃₄H₆₆N₆O₆ | -0.8 |
| 2' | 328.2590 2+ | 328.2595 | C₃₄H₆₆N₆O₆ | 1.4 |
| 3 | 322.2411 2+ | 322.2413 | C₃₂H₆₄N₆O₇ | 0.4 |
| 4 | 336.2570 2+ | 336.2569 | C₃₄H₆₆N₆O₇ | -0.3 |
| 5 | 383.3013 | 383.3017 | C₂₀H₃₉N₄O₃ | 1.1 |
| 6 | 411.3328 | 411.3330 | C₂₂H₄₃N₄O₃ | 0.4 |
| 7 | 399.2963 | 399.2966 | C₂₀H₃₉N₄O₄ | 0.6 |
| 8 | 427.3274 | 427.3279 | C₂₂H₄₃N₄O₄ | 1.1 |
| 9 | 301.2485 | 301.2486 | C₁₆H₃₃N₂O₃ | 0.2 |
| 10 | 329.2795 | 329.2799 | C₁₈H₃₇N₂O₃ | 1.1 |
| 11 | 345.2491 | 345.2496 | C₁₆H₃₃N₄O₄ | 1.5 |
| 12 | 361.2453 | 361.2445 | C₁₆H₃₃N₄O₅ | -2.1 |
| 13 | 245.1728 | 245.1734 | C₂₂H₄₆N₆O₆ | 2.5 |
| 14 | 119.0815 | 119.0815 | C₄H₁₁N₂O₂ | 0.1 |
| 15 | 135.0763 | 135.0764 | C₄H₁₁N₂O₃ | 0.9 |
| 16 | 683.5046 | 683.5066 | C₃₅H₆₇N₆O₇ | 2.8 |
| 17 | 439.3272 | 439.3279 | C₂₃H₄₃N₄O₄ | 1.5 |
| 18 | 699.4999 | 699.5015 | C₃₅H₆₇N₆O₈ | 2.3 |
| 19 | 455.3224 | 455.3228 | C₂₃H₄₃N₄O₅ | 0.9 |
| 20 | 414.3321 | 414.3326 | C₂₂H₄₄N₃O₄ | 1.3 |
| 20' | 414.3323 | 414.3326 | C₂₂H₄₄N₃O₄ | 0.7 |
| 21 | 442.3632 | 442.3639 | C₂₄H₄₆N₃O₄ | 1.6 |
| 21' | 442.3635 | 442.3639 | C₂₄H₄₆N₃O₄ | 1.0 |
| 22 | 440.3478 | 440.3483 | C₂₄H₄₆N₃O₄ | 1.1 |
| 22' | 440.3477 | 440.3483 | C₂₄H₄₆N₃O₄ | 1.2 |
| 23 | 396.3217 | 396.3221 | C₂₂H₄₂N₃O₃ | 0.9 |
| 24 | 242.1862 | 242.1863 | C₁₂H₂₄N₃O₂ | 0.3 |
| 24' | 242.1864 | 242.1863 | C₁₂H₂₄N₃O₂ | -0.2 |
| 25 | 260.1972 | 260.1969 | C₁₂H₂₆N₃O₃ | -1.2 |
| 26 | 470.3585 | 470.3588 | C₂₅H₄₆N₃O₅ | 0.7 |
| 26' | 470.3582 | 470.3588 | C₂₅H₄₆N₃O₅ | 1.3 |
| 27 | 442.3273 | 442.3275 | C₂₃H₄₄N₃O₅ | 0.5 |
| 27' | 442.3271 | 442.3275 | C₂₃H₄₄N₃O₅ | 1.0 |
| 28 | 468.3428 | 468.3432 | C₂₅H₄₆N₃O₅ | 0.9 |
| 28' | 468.3426 | 468.3432 | C₂₅H₄₆N₃O₅ | 1.3 |
| 29 | 498.3897 | 498.3901 | C₂₇H₅₂N₃O₅ | 0.8 |
| 29' | 498.3895 | 498.3901 | C₂₇H₅₂N₃O₅ | 1.2 |
| 30 | 484.3738 | 484.3745 | C₂₆H₅₀N₃O₅ | 1.4 |
| 30' | 484.3741 | 484.3745 | C₂₆H₅₀N₃O₅ | 0.8 |
| 31 | 496.3740 | 496.3745 | C₂₇H₅₀N₃O₅ | 1.0 |
| 31' | 496.3739 | 496.3745 | C₂₇H₅₀N₃O₅ | 1.2 |
| 32 | 488.3148 | 488.3152 | C₂₄H₄₆N₃O₅S | -0.8 |
| 32' | 488.3148 | 488.3152 | C₂₄H₄₆N₃O₅S | -0.8 |
| 33 | 460.2837 | 460.2839 | C₂₂H₄₂N₃O₅S | -0.4 |
| 34 | 331.2229 | 331.2227 | C₁₆H₃₁N₂O₅ | -0.6 |
| 34' | 331.2229 | 331.2227 | C₁₆H₃₁N₂O₅ | -0.4 |
| 35 | 303.1916 | 303.1914 | C₁₄H₂₇N₂O₅ | -0.4 |
| 36 | 349.1796 | 349.1791 | C₁₅H₂₈N₂O₅S | 1.4 |
| 37 | 345.2388 | 345.2384 | C₁₇H₃₃N₂O₅ | -1.0 |
| 38 | 355.7495 2+ | 355.7498 | C₃₄H₆₅N₉O₇ | 0.7 |
| 39 | 390.7944 2+ | 390.7945 | C₃₈H₇₅N₁₁O₆ | 0.3 |
| 40 | 363.7469 2+ | 363.7473 | C₃₄H₆₅N₉O₈ | 1.1 |
| 41 | 708.4748 | 708.4767 | C₃₄H₆₂N₉O₇ | 2.6 |
| 42 | 175.1190 | 175.1190 | C₆H₁₅N₄O₂ | -0.2 |
| 43 | 191.1139 | 191.1139 | C₆H₁₅N₄O₃ | -0.0 |
| 44 | 116.0818 | 116.0818 | C₄H₁₀N₃O | -0.0 |
| 45 | 311.0923 | 311.0925 | C₁₁H₁₁F₅N₄O | -0.6 |
| 46 | 584.4121 | 584.4130 | C₂₈H₅₄N₇O₆ | 1.5 |
| 47 | 600.4069 | 600.4079 | C₂₈H₅₄N₇O₇ | 1.7 |

**Table 6. Peptide sequences for compounds 1-4 as in Table 5.**

| Compound | MS calculated [M+H]⁺ | Peptide Sequence |
|---|---|---|
| 1 | 314.2438 | Decanoic acid-k-(2S-Diaminobutyric acid)-/-*L* |
| 2 | 328.2595 | Doecanoic acid-k-(2S-Diaminobutyric acid)-/-*L* |
| 3 | 322.2411 | Decanoic acid-k-(2S,3R-3-hydroxy diaminobutyric acid)-/-*L* |
| 4 | 336.2570 | Dodecanoic acid-k-(2S,3R-3-hydroxy diaminobutyric acid)-/-*L* |

### References

1 Pantel, L.; Florin, T.; Dobosz-Bartoszek, M.; Racine, E.; Sarciaux, M.; Serri, M.; Houard, J.; Campagne, J. M.; de Figueiredo, R. M.; Midrier, C.; Gaudriault, S.; Givaudan, A.; Lanois, A.; Forst, S.; Aumelas, A.; Cotteaux-Lautard, C.; Bolla, J. M.; Vingsbo Lundberg, C.; Huseby, D. L.; Hughes, D.; Villain-Guillot, P.; Mankin, A. S.; Polikanov, Y. S.; Gualtieri, M. (2018) Odilorhabdins, Antibacterial Agents That Cause Miscoding by Binding at a New Ribosomal Site. Mol. Cell 70, 83-94.e7.
2 Pantel, L.; Juarez, P.; Serri, M.; Boucinha, L.; Lessoud, E.; Lanois, A.; Givaudan, A.; Racine, E.; Gualtieri, M. (2021) Missense Mutations in the Crrb Protein Mediate Odilorhabdin Derivative Resistance in Klebsiella Pneumoniae. Antimicrob. Agents Chemother. 65**,** .
3 Zhao, M.; Lepak, A. J.; Marchillo, K.; Vanhecker, J.; Andes, R. (2018) In Vivo Pharmacodynamic Characterization of a Novel Odilorhabdin Antibiotic, NOSO-502, against Escherichia Coli and Klebsiella Pneumoniae in a Murine Thigh Infection Model. Antimicrob. Agents Chemother. 62, 1-9**.**
4 Bode, H. B.; Reimer, D.; Fuchs, S. W.; Kirchner, F.; Dauth, C.; Kegler, C.; Lorenzen, W.; Brachmann, A. O.; Grün, P. (2012) Determination of the Absolute Configuration of Peptide Natural Products by Using Stable Isotope Labeling and Mass Spectrometry. Chem. - A Eur. J. 18, 2342-2348.
5 Bozhüyük, K. A. J.; Präve, L.; Kegler, C.; Kaiser, S.; Shi, Y.-N.; Kuttenlochner, W.; Schenk, L.; Mohiuddin, T. M.; Groll, M.; Hochberg, G. K. A.; Bode, H. B. (2022) Evolution Inspired Engineering of Megasynthetases. bioRxiv 2022.12.02.518901.
6 Schimming, O.; Fleischhacker, F.; Nollmann, F. I.; Bode, H. B. (2014) Yeast Homologous Recombination Cloning Leading to the Novel Peptides Ambactin and Xenolindicin. ChemBioChem 15,1290-1294**.**
7 Zhou, Q.; Grundmann, F.; Kaiser, M.; Schiell, M.; Gaudriault, S.; Batrer, A.; Kurz, M.; Bode, H. B. (2013) Structure and Biosynthesis of Xenoamicins from Entomopathogenic Xenorhabdus. Chem. - A Eur. J. 19, 16772-16779.
8 Bode, H. B.; Brachmann, A. O.; Jadhav, K. B.; Seyfarth, L.; Dauth, C.; Fuchs, S. W.; Kaiser, M.; Waterfield, N. R.; Sack, H.; Heinemann, S. H.; Arndt, H. D. (2015) Structure Elucidation and Activity of KolossinA, the D -/ L -Pentadecapeptide Product of a Giant Nonribosomal Peptide Synthetase. Angew. Chemie - Int. Ed. 54, 10352-10355.
9 Galea, C. A.; Roberts, K. D.; Zhu, Y.; Thompson, P. E.; Li, J.; Velkov, T. (2017) Functional Characterization of the Unique Terminal Thioesterase Domain from Polymyxin Synthetase. Biochemistry 56, 657-668.
10 Bode, E.; Heinrich, A. K.; Hirschmann, M.; Abebew, D.; Shi, Y. N.; Vo, T. D.; Wesche, F.; Shi, Y. M.; Grün, P.; Simonyi, S.; Keller, N.; Engel, Y.; Wenski, S.; Bennet, R.; Beyer, S.; Bischoff, I.; Buaya, A.; Brandt, S.; Cakmak, I.; Çimen, H.; Eckstein, S.; Frank, D.; Fürst, R.; Gand, M.; Geisslinger, G.; Hazir, S.; Henke, M.; Heermann, R.; Lecaudey, V.; Schäfer, W.; Schiffmann, S.; Schüffler, A.; Schwenk, R.; Skaljac, M.; Thines, E.; Thines, M.; Ulshöfer, T.; Vilcinskas, A.; Wichelhaus, T. A.; Bode, H. B. (2019) Promoter Activation in Δhfq Mutants as an Efficient Tool for Specialized Metabolite Production Enabling Direct Bioactivity Testing. Angew. Chemie - Int. Ed. 58, 18957-18963.
11 Durfee, T.; Nelson, R.; Baldwin, S.; Plunkett 3rd, G.; Burland, V.; Mau, B.; Petrosino, J. F.; Qin, X.; Muzny, D. M.; Ayele, M.; Gibbs, R. A.; Csörgo, B.; Pósfai, G.; Weinstock, G. M.; Blattner, F. R. (2008) The Complete Genome Sequence of Escherichia Coli DH10B: Insights into the Biology of a Laboratory Workhorse. J Bacteriol 190, 2597-2606.
12 Bozhueyuek, K. A. J.; Watzel, J.; Abbood, N.; Bode, H. B. (2021) Synthetic Zippers as an Enabling Tool for Engineering of Non-Ribosomal Peptide Synthetases**. Angew. Chemie - Int. Ed. 60, 17531-17538**.**
13 Lorenzen, W.; Ahrendt, T.; Bozhüyük, K. A. J.; Bode, H. B. (2014) A Multifunctional Enzyme Is Involved in Bacterial Ether Lipid Biosynthesis. Nat. Chem. Biol. 10, 425-427**.**
14 Meyer, A. J.; Segall-Shapiro, T. H.; Glassey, E.; Zhang, J.; Voigt, C. A. (2019) Escherichia Coli "Marionette" Strains with 12 Highly Optimized Small-Molecule Sensors. Nat. Chem. Biol. 15, 196-204.

Further examples and/or embodiments of the present invention are disclosed in the following numbered items.
1. A method of preparing a non-standard amino acid or a peptide/protein comprising a non-standard amino acid residue, wherein the non-standard amino acid is 2,4-diamino-3-hydroxybutyrate, the method comprising the step of contacting 2,4-diaminobutyrate with an enzyme having 2,4-diaminobutyrate 3-hydroxylase activity.
2. The method of item 1, wherein the enzyme having 2,4-diaminobutyrate 3-hydroxylase activity is a polypeptide comprising an amino acid sequence with at least 80% sequence identity to SEQ ID NO: 1.
3. A method of preparing a non-standard amino acid or a peptide/protein comprising a non-standard amino acid residue, wherein the non-standard amino acid is L-5-hydroxylysine, the method comprising the step of contacting L-lysine or a peptide/protein comprising an L-lysine residue with an enzyme having L-lysine 5-hydroxylase activity.
4. The method of item 3, wherein the enzyme having L-lysine 5-hydroxylase activity is a polypeptide comprising an amino acid sequence with at least 80% sequence identity to SEQ ID NO: 2.
5. A method of preparing L-3-hydroxyarginine or a peptide/protein comprising a non-standard amino acid residue, wherein the non-standard amino acid is L-3-hydroxyarginine, the method comprising the step of contacting L-arginine with an enzyme having L-arginine 3-hydroxylase activity.
6. The method of item 5, wherein the enzyme having L-arginine 3-hydroxylase activity is a polypeptide comprising an amino acid sequence with at least 80% sequence identity to SEQ ID NO: 3.
7. A method of preparing a peptide/protein comprising a non-standard amino acid residue, wherein the non-standard amino acid is 2,3-didehydroarginine, the method comprising the step of contacting a peptide/protein comprising an L-3-hydroxyarginine residue with an enzyme having L-3-hydroxyarginine dehydratase activity, wherein a peptide/protein comprising a 2,3-didehydroarginine residue is formed.
8. The method of item 7, wherein the enzyme having L-3-hydroxyarginine dehydratase activity is a condensation domain comprised within a non-ribosomal peptide synthetase, preferably wherein said condensation domain is a polypeptide comprising an amino acid sequence with at least 80% sequence identity to SEQ ID NO.: 5.
9. A method of preparing a peptide/protein comprising one or more non-standard amino acids selected from 2,4-diamino-3-hydroxybutyrate, L-5-hydroxylysine, L-3-hydroxyarginine and 2,3-didehydroarginine, the method comprising the step(s) as defined in any one of items 1 to 8.
10. The method of item 9, wherein the peptide/protein is an odilorhabdin, which is preferably selected from NOSO-95179, NOSO-95A, NOSO-95B, NOSO-95C, and NOSO-502.
11. The method of item 9 or 10, wherein the method is performed using a cell-based or a cell-free system comprising a biosynthetic gene cluster which comprises a gene encoding a polypeptide having 2,4-diaminobutyrate 3-hydroxylase activity, a gene encoding a polypeptide having L-lysine 5-hydroxylase activity, a gene encoding a polypeptide having L-arginine 3-hydroxylase activity, and/or a gene encoding a polypeptide having L-3-hydroxyarginine dehydratase activity, wherein the biosynthetic gene cluster preferably further comprises a gene encoding a polypeptide with deacylase activity which is capable of enzymatic removal of fatty acyl from the N-terminus of the peptide/protein.
12. The method of item 11, wherein the polypeptide with deacylase activity is a polypeptide comprising an amino acid sequence with at least 80% sequence identity to SEQ ID NO: 4.
13. Use of a polypeptide comprising an amino acid sequence with at least 80% sequence identity to SEQ ID NO: 1 for catalyzing a hydroxylation at position 3 of 2,4-diaminobutyrate.
14. Use of a polypeptide comprising an amino acid sequence with at least 80% sequence identity to SEQ ID NO: 2 for catalyzing a hydroxylation at position 5 of L-lysine.
15. Use of a polypeptide comprising an amino acid sequence with at least 80% sequence identity to SEQ ID NO: 3 for catalyzing a hydroxylation at position 3 of L-arginine.
16. Use of a polypeptide comprising an amino acid sequence with at least 80% sequence identity to SEQ ID NO: 4 for catalyzing a deacylation, preferably for removing a fatty acyl from the N-terminus of an odilorhabdin.
17. A polypeptide comprising an amino acid sequence with at least 80% sequence identity to SEQ ID NO: 1 and having 2,4-diaminobutyrate-3-hydroxylase activity, wherein said polypeptide is not a polypeptide consisting of the amino acid sequence according to SEQ ID NO: 1.
18. A polypeptide comprising an amino acid sequence with at least 80% sequence identity to SEQ ID NO: 2 and having L-lysine 5-hydroxylase activity, wherein said polypeptide is not a polypeptide consisting of the amino acid sequence according to SEQ ID NO: 2.
19. A polypeptide comprising an amino acid sequence with at least 80% sequence identity to SEQ ID NO: 3 and having L-arginine 3-hydroxylase activity, wherein said polypeptide is not a polypeptide consisting of the amino acid sequence according to SEQ ID NO: 3.
20. A polypeptide comprising an amino acid sequence with at least 80% sequence identity to SEQ ID NO: 4 and having a deacylase activity, which is preferably capable of removing fatty acyl from the N-terminus of an odilorhabdin, wherein said polypeptide is not a polypeptide consisting of the amino acid sequence according to SEQ ID NO: 4.

## Claims

1. A method of preparing a non-standard amino acid or a peptide/protein comprising a non-standard amino acid residue, wherein the non-standard amino acid is 2,4-diamino-3-hydroxybutyrate, the method comprising the step of contacting 2,4-diaminobutyrate with an enzyme having 2,4-diaminobutyrate 3-hydroxylase activity;
preferably wherein the enzyme having 2,4-diaminobutyrate 3-hydroxylase activity is a polypeptide comprising an amino acid sequence with at least 80% sequence identity to SEQ ID NO: 1.

2. A method of preparing a non-standard amino acid or a peptide/protein comprising a non-standard amino acid residue, wherein the non-standard amino acid is L-5-hydroxylysine, the method comprising the step of contacting L-lysine or a peptide/protein comprising an L-lysine residue with an enzyme having L-lysine 5-hydroxylase activity;
preferably wherein the enzyme having L-lysine 5-hydroxylase activity is a polypeptide comprising an amino acid sequence with at least 80% sequence identity to SEQ ID NO: 2.

3. A method of preparing L-3-hydroxyarginine or a peptide/protein comprising a non-standard amino acid residue, wherein the non-standard amino acid is L-3-hydroxyarginine, the method comprising the step of contacting L-arginine with an enzyme having L-arginine 3-hydroxylase activity.

4. The method of claim 3, wherein the enzyme having L-arginine 3-hydroxylase activity is a polypeptide comprising an amino acid sequence with at least 80% sequence identity to SEQ ID NO: 3.

5. A method of preparing a peptide/protein comprising a non-standard amino acid residue, wherein the non-standard amino acid is 2,3-didehydroarginine, the method comprising the step of contacting a peptide/protein comprising an L-3-hydroxyarginine residue with an enzyme having L-3-hydroxyarginine dehydratase activity, wherein a peptide/protein comprising a 2,3-didehydroarginine residue is formed; preferably wherein the enzyme having L-3-hydroxyarginine dehydratase activity is a condensation domain comprised within a non-ribosomal peptide synthetase, more preferably wherein said condensation domain is a polypeptide comprising an amino acid sequence with at least 80% sequence identity to SEQ ID NO.: 5.

6. A method of preparing a peptide/protein comprising one or more non-standard amino acids selected from 2,4-diamino-3-hydroxybutyrate, L-5-hydroxylysine, L-3-hydroxyarginine and 2,3-didehydroarginine, the method comprising the step(s) as defined in any one of claims 1 to 5.

7. The method of claim 6, wherein:
- the peptide/protein is an odilorhabdin, which is preferably selected from NOSO-95179, NOSO-95A, NOSO-95B, NOSO-95C, and NOSO-502; and/or
- the method is performed using a cell-based or a cell-free system comprising a biosynthetic gene cluster which comprises a gene encoding a polypeptide having 2,4-diaminobutyrate 3-hydroxylase activity, a gene encoding a polypeptide having L-lysine 5-hydroxylase activity, a gene encoding a polypeptide having L-arginine 3-hydroxylase activity, and/or a gene encoding a polypeptide having L-3-hydroxyarginine dehydratase activity, wherein the biosynthetic gene cluster preferably further comprises a gene encoding a polypeptide with deacylase activity which is capable of enzymatic removal of fatty acyl from the N-terminus of the peptide/protein,
preferably wherein the polypeptide with deacylase activity is a polypeptide comprising an amino acid sequence with at least 80% sequence identity to SEQ ID NO: 4.

8. Use of a polypeptide comprising an amino acid sequence with at least 80% sequence identity to SEQ ID NO: 1 for catalyzing a hydroxylation at position 3 of 2,4-diaminobutyrate.

9. Use of a polypeptide comprising an amino acid sequence with at least 80% sequence identity to SEQ ID NO: 2 for catalyzing a hydroxylation at position 5 of L-lysine.

10. Use of a polypeptide comprising an amino acid sequence with at least 80% sequence identity to SEQ ID NO: 3 for catalyzing a hydroxylation at position 3 of L-arginine.

11. Use of a polypeptide comprising an amino acid sequence with at least 80% sequence identity to SEQ ID NO: 4 for catalyzing a deacylation, preferably for removing a fatty acyl from the N-terminus of an odilorhabdin.

12. A polypeptide comprising an amino acid sequence with at least 80% sequence identity to SEQ ID NO: 1 and having 2,4-diaminobutyrate-3-hydroxylase activity, wherein said polypeptide is not a polypeptide consisting of the amino acid sequence according to SEQ ID NO: 1.

13. A polypeptide comprising an amino acid sequence with at least 80% sequence identity to SEQ ID NO: 2 and having L-lysine 5-hydroxylase activity, wherein said polypeptide is not a polypeptide consisting of the amino acid sequence according to SEQ ID NO: 2.

14. A polypeptide comprising an amino acid sequence with at least 80% sequence identity to SEQ ID NO: 3 and having L-arginine 3-hydroxylase activity, wherein said polypeptide is not a polypeptide consisting of the amino acid sequence according to SEQ ID NO: 3.

15. A polypeptide comprising an amino acid sequence with at least 80% sequence identity to SEQ ID NO: 4 and having a deacylase activity, which is preferably capable of removing fatty acyl from the N-terminus of an odilorhabdin, wherein said polypeptide is not a polypeptide consisting of the amino acid sequence according to SEQ ID NO: 4.
